(19) Europäisches Patentamt
European Patent Office
Office européen des brevets

(11) Veröffentlichungsnummer: **0 313 997 B1**

(12) # EUROPÄISCHE PATENTSCHRIFT

(45) Veröffentlichungstag der Patentschrift: **09.03.94**

(51) Int. Cl.[5]: **C07F 9/547**, A61K 31/675

(21) Anmeldenummer: **88117519.4**

(22) Anmeldetag: **21.10.88**

(54) **Arzneimittel, darin enthaltene phosphorhaltige 2-Isoxazoline und Isoxazole, sowie Herstellungsverfahren für diese heterocyclischen Verbindungen.**

(30) Priorität: **26.10.87 DE 3736113**

(43) Veröffentlichungstag der Anmeldung:
**03.05.89 Patentblatt 89/18**

(45) Bekanntmachung des Hinweises auf die Patenterteilung:
**09.03.94 Patentblatt 94/10**

(84) Benannte Vertragsstaaten:
**AT BE CH DE ES FR GB GR IT LI LU NL SE**

(56) Entgegenhaltungen:
**EP-A- 0 087 953**
**FR-A- 2 330 685**

**JOURNAL OF GENERAL CHEMISTRY OF THE USSR, Band 45, Nr. 12, Teil 2, Dezember 1975, Seite 2708; "A translation of Zhurnal Obshchei Khimii"**

(73) Patentinhaber: **HOECHST AKTIENGESELLSCHAFT**

**D-65926 Frankfurt(DE)**

(72) Erfinder: **Schwab, Wilfried, Dr.**
**Auf den Erlen 1D**
**D-6200 Wiesbaden(DE)**
Erfinder: **Bartlett, Robert Ryder, Dr.**
**Sandbergstrasse 20**
**D-6100 Darmstadt(DE)**
Erfinder: **Gebert, Ulrich, Dr.**
**Albert-Lortzing-Strasse 2**
**D-6233 Kelkheim (Taunus)(DE)**
Erfinder: **Schorlemmer, Hans Ulrich, Dr.**
**Am Kirschenwald 2**
**D-3550 Marburg(DE)**
Erfinder: **Dickneite, Gerhard, Dr.**
**Zum Neuen Hieb 31**
**D-3550 Marburg(DE)**
Erfinder: **Sedlacek, Hans Harald, Dr.**
**Sonnenhang 3**
**D-3550 Marburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Arzneimittel, die sich insbesondere zur Prophylaxe und/oder Behandlung von Erkrankungen des Immunsystems bei Mensch und Tier eignen, die in ihnen enthaltenen pharmakologisch aktiven 2-Isoxazoline und Isoxazole, sowie Verfahren zur Herstellung dieser als Wirkstoffe dienenden heterocyclischen Verbindungen.

Es ist bekannter, daß der lebende Organismus über humorale und zelluläre immunologische Abwehrmechanismen verfügt. Sie dienen dazu, Fremdkörper, die pathogenetische Veränderungen hervorrufen können, zu neutralisieren und zu eliminieren, vornehmlich Mikroorganismen und neoplastische Zellen. Immunologische Untersuchungen haben ergeben, daß Zusammenhänge zwischen der natürlichen oder durch äußere Faktoren provozierten Abnahme der immunologischen Aktivität und der Zunahme der Infektions- und Tumorkrankheiten bestehen. Eine Reihe anderer Krankheiten, wie die Autoimmun- oder durch Immunkomplexe hervorgerufenen Erkrankungen, Intoxikationen und Septikämien, entstehen durch Entgleisung einzelner Funktionen des komplexen Immunsystems.

Man sucht deshalb seit langem nach potenten und gut verträglichen Immunmodulatoren, die einen breiten therapeutischen Einsatz zur Unterstützung oder Normalisierung der natürlichen Abwehrkräfte bei Tier und Mensch gestatten.

Versuche zur Stimulation der Immunität mit BCG (Bacillus Calmette Guérin) und Corynebacterium parvum sowie Extrakten aus Mycobacterium tuberculosis und Brucellen verliefen unbefriedigend, da diese Substanzen in den erforderlichen Konzentrationen gravierende Nebenwirkungen, beispielsweise lokale Granulome, hervorrufen. Darüber hinaus erschwert die Unkenntnis von Zusammensetzung der heterogenen Stoffgemische und Struktur der Einzelkomponenten eine systematische klinische Untersuchung mit gut reproduzierbaren Ergebnissen. Es besteht somit ein dringendes Bedürfnis nach neuen, gut verträglichen Immunmodulatoren, die chemisch definierte Substanzen darstellen.

Überraschenderweise wurde nun gefunden, daß durch Einführung bestimmter phosphorhaltiger Reste, wie einer Phosphinyl-, Phosphonyl- oder Phosphonogruppe, in die 5-Position von in 3-Stellung substituierten 2-Isoxazolinen und Isoxazolen Verbindungen erhalten werden, die aufgrund ihrer pharmakologischen Eigenschaften die zuvor dargestellten Anforderungen erfüllen und sich demnach hervorragend zur Prophylaxe und/oder Behandlung von Erkrankungen eignen, die mit pathologischen Veränderungen des Immunsystems einhergehen.

Die Verbindungen besitzen bei äußerst guter Verträglichkeit eine starke immunmodulierende Wirkung bei warmblütigen Säugern, wie sich beispielsweise durch Stimulation der DTH(delayed-type hypersensitivity)-Reaktion auf Schaferythrozyten, Aktivierung von mononukleären Phagozyten, Hemmung bestimmter Aminopeptidasen, tumorhemmende Wirksamkeit, etwa gegen das B 16 Melanom an der Maus, und Erhöhung der immunologischen Widerstandskraft gegen Infektionen oder Autoimmunerkrankungen, beispielsweise in verschiedenen experimentellen Infektionsmodellen bzw. dem Modell der aktiven Arthus-Reaktion an der Ratte und dem chronischen Graft-versus-Host(cGvH)-Modell an der Maus, demonstrieren läßt. Sie stellen somit wertvolle Wirkstoffe dar, die das pathologisch veränderte Immunsystem bei Mensch und Tier wiederherzustellen vermögen.

Zwar ist in der Literatur die Synthese einiger 2-Isoxazolin-5-yl- und 2-Isoxazolin-5-yl-methyl-phosphonate (Zh. Obshch. Khim. 38 (1968), 1248 - 1254; Zh. Obshch. Khim. 45 (1975), 2746 - 2747), Isoxazol-5-yl-methyl-phosphonate(Synthesis 1979), 712 - 714) und 5-(Methylmethoxyphosphinyl)-isoxazole (J. Org. Chem. 45 (1980), 529 - 531) bereits beschrieben worden, doch über pharmakologische Eigenschaften dieser Verbindungen, die ihre Verwendung als Wirkstoffe in Arzneimitteln nahelegen würden, ist bisher nichts bekannt. Bei dem in der Patentschrift EP 174 685 erwähnten 3-(2-Nitro-5-(2-chlor-4-trifluormethyl-phenoxy)-phenyl)-2-isoxazolin-5-yl-phosphonsäurediethylester mit angeblich herbiziden Effekten handelt es sich allenfalls um ein potentielles Pflanzenschutzmittel.

Die vorliegende Erfindung beschreibt demgegenüber größtenteils neue 3-substituierte 2-Isoxazoline und Isoxazole mit einem 5-ständigen phosphorhaltigen Rest, die sich aufgrund ihrer zuvor erwähnten immunmodulierenden Eigenschaften als Wirkstoffe in Arzneimitteln zur Prophylaxe und/oder behandlung von Tumoren, Infektionen und Autoimmunerkrankungen eignen.

Gegenstand der Erfindung sind somit Arzneimittel, die als Wirkstoffe phosphorhaltige 2-Isoxazoline oder Isoxazole der allgemeinen Formel I und/oder gegebenenfalls deren physiologisch verträgliche Salze enthalten, wobei

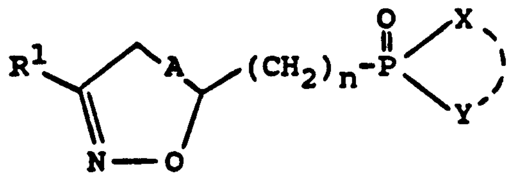

(I)

$R^1$ für

a) eine geradkettige oder verzweigte Alkyl- oder Alkenylgruppe mit 1 bis 6 C-Atomen, deren Kohlenstoffkette mit Halogen, beispielsweise Fluor, Chlor oder Brom, Hydroxy, $(C_1-C_4)$Alkoxy, $(C_1-C_4)$Acyloxy oder gegebenenfalls mit $(C_1-C_4)$Alkoxy-oder Halogen-substituiertem Aryl substituiert sein kann, oder

b) eine ein- oder zweikernige aromatische oder heteroaromatische Gruppe mit 1 bis 2 Stickstoffatomen und/oder einem Schwefel- oder Sauerstoffatom im Ringsystem, wobei diese Gruppe ein- oder mehrfach und gleich oder verschieden mit geradkettigem oder verzweigtem $(C_1-C_4)$Alkyl, $(C_3-C_6)$Cycloalkyl, Hydroxy, $(C_1-C_3)$Alkoxy, Aryloxy, $(C_1-C_4)$Acyloxy oder Benzoyloxy, Halogen, Trifluormethyl, Nitro, gegebenenfalls mono- oder disubstituiertem Amino, $(C_1-C_4)$Alkoxycarbonyl, Carboxy, Carbamoyl, $(C_1-C_4)$-Alkylcarbonyl, deren Carbonylgruppe auch in ketalisierter Form vorliegen kann, oder gegebenenfalls mit $(C_1-C_4)$Alkyl, Halogen, oder $(C_1-C_3)$Alkoxy ringsubstituiertem Phenyl oder Benzyl substituiert sein kann, oder

c) Carboxy oder Alkoxycarbonyl mit 1 bis 4 C-Atomen im Alkylteil oder

d) Arylcarbonyl, das gegebenenfalls im Arylteil mit $(C_1-C_4)$Alkyl, Halogen oder $(C_1-C_3)$Alkoxy substituiert ist, oder

e) Halogen, vorzugsweise Chlor oder Brom, steht,

A eine C,C-Einfach- oder eine C,C-Doppelbindung,

n eine ganze Zahl von 0 bis 2 und

X und Y, die gleich oder verschieden sein können, jeweils unabhängig voneinander eine geradkettige oder verzweigte $(C_1-C_4)$Alkylgruppe, den Rest $-OR^2$ oder die Gruppe $-NR^2R^3$ bedeuten, wobei $R^2$ und $R^3$ für Wasserstoff oder gegebenenfalls substituierte $(C_1-C_6)$Alkylreste stehen, die in der Gruppe $-NR^2R^3$ zusammen mit dem Stickstoffatom auch eine fünf- bis siebengliedrigen Ring oder im Strukturelement $-P(O)(OR^2)_2$ zusammen mit dem Phosphoratom einen gegebenenfalls noch mit $(C_1-C_3)$Alkyl, $(C_1-C_4)$-Alkoxycarbonyl oder Carboxy substituierten Heterocyclus der Formel

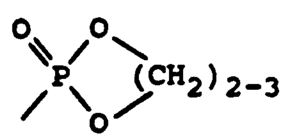

bilden können,

und wobei die Verbindungen der Formel I gegebenenfalls als reine Stereoisomere oder als deren Gemische vorliegen können.

Bevorzugt sind dabei solche Arzneimittel, die Verbindungen der Formel I und/oder gegebenenfalls deren Salze enthalten, bei denen

$R^1$ für

a) gegebenenfalls verzweigtes $(C_1-C_4)$Alkyl oder $(C_1-C_4)$Hydroxyalkyl, wie Methyl, Ethyl, Propyl, Isopropyl, Butyl, tert.Butyl, Hydroxymethyl, oder 1-Hydroxy-1-methyl-ethyl, oder Phenyl$(C_1-C_2)$alkyl oder Phenyl$(C_1-C_3)$alkenyl, wie Benzyl oder Styryl,

b) unsubstituiertes oder ein- oder mehrfach mit $(C_1-C_4)$Alkyl, wie Methyl, Ethyl oder tert.Butyl, Hydroxy, $(C_1-C_2)$Alkoxy, Phenoxy, Halogen, wie Chlor oder Fluor, Trifluormethyl, Nitro, Di$(C_1-C_2)$alkylamino, wie Dimethyl- oder Diethylamino, $(C_1-C_2)$Alkoxycarbonyl, wie Meth- oder Ethoxycarbonyl, Carboxy oder Phenyl substituiertes Phenyl, Naphthyl, Pyridyl oder Thienyl,

c) Carboxy, Meth- oder Ethoxycarbonyl,

d) Benzoyl,

e) Chlor oder Brom steht,

A eine C,C-Einfach- oder eine C,C-Doppelbindung bedeutet,

n für 0 oder 1 steht und

X und Y, die gleich oder verschieden sein können, unabhängig voneinander eine Methyl- oder

Ethylgruppe oder die Reste $-OR^2$ oder $-NR^2R^3$ darstellen, wobei $R^2$ für Wasserstoff, Methyl oder Ethyl und $R^3$ ebenfalls für Wasserstoff, Methyl oder Ethyl oder aber für das Kohlenstoffgerüst einer gegebenenfalls carboxygeschützten Aminosäure stehen, die Reste $R^2$ und $R^3$ in der Gruppe $-NR^2R^3$ zusammen mit dem Stickstoffatom auch einen Pyrrolidin-, Piperidin- oder Morpholinring und die Reste $-OR^2$ im Strukturelement $-P(O)(OR^2)_2$ zusammen mit dem Phosphoratom einen gegebenenfalls mit $(C_1-C_2)$Alkyl substituierten 2-Oxo-1,3,2-dioxaphospholan-oder 2-Oxo-1,3,2-dioxaphosphiran-Ring bilden können, und wobei diese Verbindungen gegebenenfalls als reine Stereoisomere oder als deren Gemische vorliegen können.

Unter diesen Arzneimitteln sind wiederum solche bevorzugt, die Verbindungen der Formel I und/oder gegebenenfalls deren Salze enthalten, bei denen entweder

$R^1$ für tert.Butyl, Benzyl, Phenyl, Naphthyl, Pyridyl, Thienyl oder mit Methyl, Hydroxy, Methoxy, Phenoxy, Chlor, Fluor, Trifluormethyl, Nitro, Dimethylamino, Methoxycarbonyl oder Carboxy substituiertes Phenyl steht oder

X und Y unabhängig voneinander Hydroxy, Methoxy oder Ethoxy bzw.

X Methyl und Y Hydroxy, Methoxy oder Ethoxy bedeuten, und wobei diese Verbindungen gegebenenfalls als reine Stereoisomere oder als deren Gemische vorliegen können.

Hierunter sind weiterhin jene Arzneimittel hervorzuheben, die Verbindungen der Formel I und/oder gegebenenfalls deren Salze enthalten, bei denen $R^1$, X und Y gleichzeitig die vorgenannten Bedeutungen haben, insbesondere wenn darüber hinaus A für eine C,C-Einfachbindung steht und n den Wert 0 hat, wobei diese Verbindungen gegebenenfalls als reine Stereoisomere oder als deren Gemische vorliegen können.

Eine besonders bevorzugte Gruppe von Arzneimitteln stellen schließlich jene dar, die Verbindungen der Formel I und/oder deren Salze enthalten, bei denen $R^1$ für tert.Butyl oder Phenyl steht, X und Y jeweils Hydroxy oder X Methyl und Y Hydroxy bedeuten, A eine C,C-Einfachbindung darstellt und n den Wert 0 hat, beispielsweise 3-Phenyl-2-isoxazolin-5-yl-phosphonsäure, 3-Phenyl(oder 3-tert.Butyl)-2-isoxazolin-5-yl-(P-methyl)-phosphinsäure, wobei diese Verbindungen als reine Stereoisomere oder als deren Gemische vorliegen können.

Gegenstand der Erfindung ist auch die Verwendung der erfindungsgemäßen Arzneimittel zur Prophylaxe und/oder Behandlung von Erkrankungen des Immunsystems bei Mensch und Tier, insbesondere von Tumoren, Infektionen und/oder Autoimmunerkrankungen.

Ein weiterer Gegenstand der Erfindung sind neue phosphorhaltige 2-Isoxazoline und Isoxazole der allgemeinen Formel I, ihre gegebenenfalls stereoisomeren Formen und gegebenenfalls ihre physiologisch verträglichen Salze, wobei

$R^1$ für

a) eine geradkettige oder verzweigte Alkyl- oder Alkenylgruppe mit 1 bis 6 C-Atomen, deren Kohlenstoffkette mit Halogen, beispielsweise Fluor, Chlor oder Brom, Hydroxy, $(C_1-C_4)$Alkoxy, $(C_1-C_4)$Acyloxy oder gegebenenfalls mit $(C_1-C_4)$Alkoxy oder Halogen substituiertem Aryl substituiert sein kann, oder

b) eine ein- oder zweikernige aromatische oder heteroaromatische Gruppe mit 1 bis 2 Stickstoffatomen und/oder einem Schwefel- oder Sauerstoffatom im Ringsystem, wobei diese Gruppe ein- oder mehrfach und gleich oder verschieden mit geradkettigem oder verzweigtem $(C_1-C_4)$Alkyl, $(C_3-C_6)$Cycloalkyl, Hydroxy, $(C_1-C_3)$Alkoxy, Aryloxy, $(C_1-C_4)$Acyloxy oder Benzoyloxy, Halogen, Trifluormethyl, Nitro, gegebenenfalls mono- oder disubstituiertem Amino, $(C_1-C_4)$Alkoxycarbonyl, Carboxy, Carbamoyl, $(C_1-C_4)$-Alkylcarbonyl, deren Carbonylgruppe auch in ketalisierter Form vorliegen kann, oder gegebenenfalls mit $(C_1-C_4)$Alkyl, Halogen, oder $(C_1-C_3)$Alkoxy ringsubstituiertem Phenyl oder Benzyl substituiert sein kann, oder

c) Carboxy oder Alkoxycarbonyl mit 1 bis 4 C-Atomen im Alkylteil oder

d) Arylcarbonyl, das gegebenenfalls im Arylteil mit $(C_1-C_4)$Alkyl, Halogen oder $(C_1-C_3)$Alkoxy substituiert ist, oder

e) Halogen, vorzugsweise Chlor oder Brom, steht,

A eine C,C-Einfach- oder eine C,C-Doppelbindung,

n eine ganze Zahl von 0 bis 2 und

X und Y, die gleich oder verschieden sein können, jeweils unabhängig voneinander eine geradkettige oder verzweigte $(C_1-C_4)$Alkylgruppe, den Rest $-OR^2$ oder die Gruppe $-NR^2R^3$ bedeuten, wobei $R^2$ und $R^3$ für Wasserstoff oder gegebenenfalls substituierte $(C_1-C_6)$Alkylreste stehen, die in der Gruppe $-NR^2R^3$ zusammen mit dem Stickstoffatom auch einen fünf- bis siebengliedrigen Ring oder im Strukturelement $-P(O)(OR^2)_2$ zusammen mit dem Phosphoratom einen gegebenenfalls noch mit $(C_1-C_3)$Alkyl, $(C_1-C_4)$-Alkoxycarbonyl oder Carboxy substituierten Heterocyclus der Formel

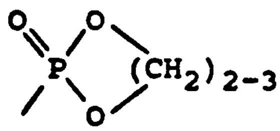

bilden können,

mit Ausnahme der Verbindungen 3-Phenyl-2-isoxazolin-5-yl-phosphonsäure, 3-Methyl( und Phenyl)-2-isoxazolin-5-yl-phosphonsäuredimethylester, 3-(3-Nitrophenyl)-2-isoxazolin-5-yl-phosphonsäuredipropylester, 3-(2-Nitro-5-(2-chlor-4-trifluormethyl-phenoxy)-phenyl)-2-isoxazolin-5-yl-phosphonsäurediethylester, 3-Methyl(und Phenyl)-2-isoxazolin-5-yl-phosphonsäuretetramethyldiamid,3-Phenyl-2-isoxazolin-5-yl-methyl-phosphonsäure und deren Diethylester, 3-Methyl(Ethyl, Isopropyl, tert.Butyl, Methoxymethyl, Phenyl und Ethoxycarbonyl)-isoxazol-5-yl-methylphosphonsäurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegebenenfalls racemischen Formen handelt.

Bevorzugt sind dabei solche Verbindungen der Formel I, einschließlich ihrer gegebenenfalls stereoisomeren Formen und gegebenenfalls ihrer Salze, bei denen

$R^1$ für

a) gegebenenfalls verzweigtes $(C_1\text{-}C_4)$Alkyl oder $(C_1\text{-}C_4)$Hydroxyalkyl, wie Methyl, Ethyl, Propyl, Isopropyl, Butyl, tert.Butyl, Hydroxymethyl, oder 1-Hydroxy-1-methyl-ethyl, oder Phenyl$(C_1\text{-}C_2)$alkyl oder Phenyl$(C_1\text{-}C_3)$alkenyl, wie Benzyl oder Styryl,

b) unsubstituiertes oder ein- oder mehrfach mit $(C_1\text{-}C_4)$Alkyl, wie Methyl, Ethyl oder tert.Butyl, Hydroxy, $(C_1\text{-}C_2)$Alkoxy, Phenoxy, Halogen, wie Chlor oder Fluor, Trifluormethyl, Nitro, Di$(C_1\text{-}C_2)$alkylamino, wie Dimethyl-oder Diethylamino, $(C_1\text{-}C_2)$Alkoxycarbonyl, wie Meth- oder Ethoxycarbonyl, Carboxy oder Phenyl substituiertes Phenyl, Naphthyl, Pyridyl oder Thienyl,

c) Carboxy, Meth- oder Ethoxycarbonyl,

d) Benzoyl,

e) Chlor oder Brom steht,

A eine C,C-Einfach- oder eine C,C-Doppelbindung bedeutet,

n für 0 oder 1 steht und

X und Y, die gleich oder verschieden sein können, unabhängig voneinander eine Methyl- oder Ethylgruppe oder die Reste $-OR^2$ oder $-NR^2R^3$ darstellen, wobei $R^2$ für Wasserstoff, Methyl oder Ethyl und $R^3$ ebenfalls für Wasserstoff, Methyl oder Ethyl oder aber für das Kohlenstoffgerüst einer gegebenenfalls carboxygeschützten Aminosäure stehen, die Reste $R^2$ und $R^3$ in der Gruppe $-NR^2R^3$ zusammen mit dem Stickstoffatom auch einen Pyrrolidin-, Piperidin- oder Morpholinring und die Reste $-OR^2$ im Strukturelement $-P(O)\,(OR^2)_2$ zusammen mit dem Phosphoratom einen gegebenenfalls mit $(C_1\text{-}C_2)$Alkyl substituierten 2-Oxo-1,3,2-dioxaphospholan- oder 2-Oxo-1,3,2-dioxaphosphiran-Ring bilden können,

mit Ausnahme der Verbindungen 3-Phenyl-2-isoxazolin-5-yl-phosphonsäure, 3-Methyl( und Phenyl)-2-isoxazolin-5-yl-phosphonsäuredimethylester, 3-Methyl( und Phenyl)-2-isoxazolin-5-yl-phosphonsäuretetramethyldiamid, 3-Phenyl-2-isoxazolin-5-yl-methyl-phosphonsäure und deren Diethylester, 3-Methyl(Ethyl, Isopropyl, tert.Butyl, Phenyl und Ethoxycarbonyl)-isoxazol-5-yl-methyl-phosphonsäurediethylester, 3-(4-Fluor- und 4-Chlor-phenyl)-isoxazol-5-yl-(P-methyl)-phosphinsäure und 3-Phenyl-isoxazol-5-yl-(P-methyl)-phosphinsäuremethylester, sofern es sich um deren gegebenenfalls racemischen Formen handelt.

Unter diesen Verbindungen sind wiederum solche, einschließlich ihrer gegebenenfalls stereoisomeren Formen und gegebenenfalls ihrer Salze, bevorzugt, bei denen entweder $R^1$ für tert.Butyl, Benzyl, Phenyl, Naphthyl, Pyridyl, Thienyl oder mit Methyl, Hydroxy, Methoxy, Phenoxy, Chlor, Fluor, Trifluormethyl, Nitro, Dimethylamino, Methoxycarbonyl oder Carboxy substituiertes Phenyl steht oder X und Y unabhängig voneinander Hydroxy, Methoxy oder Ethoxy bzw.

X Methyl und Y Hydroxy, Methoxy oder Ethoxy bedeuten, mit Ausnahme der Verbindungen 3-Phenyl-2-isoxazolin-5-yl-phosphonsäure, 3-Methyl( und Phenyl)-2-isoxazolin-5-yl-phosphonsäuredimethylester, 3-Phenyl-2-isoxazolin-5-yl-phosphonsäuretetramethyldiamid, 3-Phenyl-2-isoxazolin-5-yl-methyl-phosphonsäure und deren Diethylester, 3-Methyl(Ethyl, Isopropyl, tert.Butyl, Phenyl und Ethoxycarbonyl)-isoxazol-5-yl-methyl-phosphonsäurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegebenenfalls racemischen Formen handelt.

Hierunter sind weiterhin jene Verbindungen, einschließlich ihrer gegebenenfalls stereoisomeren Formen und gegebenenfalls ihrer Salze, hervorzuheben, bei denen $R^1$, X und Y gleichzeitig die vorgenannten Bedeutungen haben, insbesondere wenn darüber hinaus A für eine C,C-Einfachbindung steht und n den

Wert 0 hat,
mit Ausnahme der Verbindungen 3-Phenyl-2-isoxazolin-5-yl-phosphonsäure und deren Dimethylester, 3-Phenyl-2-isoxazolin-5-yl-methyl-phosphonsäure und deren Diethylester, 3-tert.Butyl(und Phenyl)-isoxazol-5-yl-methyl-phosphonsäurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegenenfalls racemischen Formen handelt.

Eine besonders bevorzugte Gruppe von Verbindungen, einschließlich ihrer stereoisomeren Formen und ihrer Salze, stellen schließlich jene dar, bei denen $R^1$ für tert.Butyl oder Phenyl steht, X und Y jeweils Hydroxy oder X Methyl und Y Hydroxy bedeuten, A eine C,C-Einfachbindung darstellt und n den Wert 0 hat, beispielsweise 3-Phenyl-2-isoxazolin-5-yl-phosphonsäure oder 3-Phenyl(oder 3-tert.Butyl)-2-isoxazolin-5-yl-(P-methyl)-phosphinsäure, mit Ausnahme von racemischer 3-Phenyl-2-isoxazolin-5-yl-phosphonsäure.

Gegenstand der Erfindung ist weiterhin ein Verfahren zur Herstellung der phosphorhaltigen 2-Isoxazoline und Isoxazole der allgemeinen Formel I, ihrer gegebenenfalls stereoisomeren Formen sowie gegebenenfalls ihrer physiologisch verträglichen Salze, das darin besteht, daß man ein Nitriloxid der Formel II

$R^1$-C≡N→O (II)

a) für den Fall, daß A in Formel I eine C,C-Einfachbindung bedeutet,
mit einer olefinischen Phosphorverbindung der Formel III

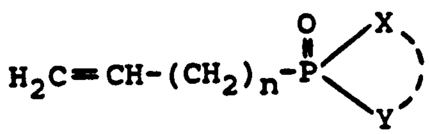

(III)

umsetzt oder
b) für den Fall, daß A in Formel I eine C,C-Doppelbindung bedeutet,
mit einer olefinischen Phosphorverbindung der Formel IV

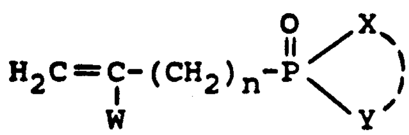

(IV)

zu einem 2-Isoxazolin der Formel V

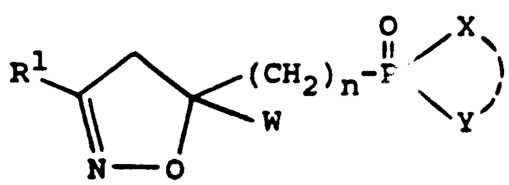

(V)

umsetzt und aus diesem Zwischenprodukt unter basischen Bedingungen oder thermischer Belastung HW eliminiert, wobei in den Formeln II bis V $R^1$, n, X und Y die vorgenannten Bedeutungen haben und W für eine Abgangsgruppe, wie Halogen, vorzugsweise Brom oder Chlor, eine ($C_1$-$C_6$)Alkoxy- oder Sulfonsäureester-Gruppierung steht, und gegebenenfalls
c) einen nach a) oder b) erhaltenen Phosphon- oder Phosphinsäureester der Formel I zum Phosphonsäurehalbester oder zur Phosphon- bzw. Phosphinsäure der Formel I spaltet oder
d) einen nach a) oder b) gewonnenen Phosphonsäuredialkylester der Formel I mit einem Amin der Formel $HNR^2R^3$ (VI) unter Austausch einer der beiden phosphorständigen Alkoxygruppen gegen den Rest $-NR^2R^3$ zu einem Halbesterhalbamid der Formel I umsetzt, wobei $R^2$ und $R^3$ die vorgenannten Bedeutungen haben und Verbindungen, in denen $R^1$ Halogen bedeutet, ausgenommen sind, oder

e) eine nach a), b) oder c) hergestellte Phosphonsäure der Formel I zunächst in ein am Phosphoratom aktiviertes Säurederivat überführt und dieses anschließend mit Alkoholen der Formel $R^2OH$ (VII), oder einem Diol der Formel $HO-(CH_2)_{2-3}-OH$ (VIII) und/oder Aminen der Formel VI wahlweise zu einem Mono- oder gegebenenfalls gemischten Diester, einem cyclischen Ester, einem Halbesterhalbamid oder einem Mono- oder gegebenenfalls gemischten Diamid der Formel I umsetzt oder

einen nach a), b) oder c) erhaltenen Phosphonsäurehalbester der Formel I nach Aktivierung am Phosphoratom mit einem Alkohol VII oder einem Amin VI zu einem gegebenenfalls gemischten Diester bzw. einem Halbesterhalbamid der Formel I umsetzt oder

eine nach a), b) oder c) hergestellte Phosphinsäure der Formel I nach Aktivierung am Phosphoratom mit einem Alkohol VII oder einem Amin VI zu einem Ester bzw. Amid der Formel I umsetzt,

wobei $R^2$ und $R^3$ in Formel VI die vorgenannten Bedeutungen haben, $R^2$ in Formel VII für gegebenenfalls substituiertes $(C_1-C_6)$Alkyl steht und die Alkylenkette $-(CH_2)_{2-3}-$ in Formel VIII noch mit $(C_1-C_3)$Alkyl, $(C_1-C_4)$Alkoxycarbonyl oder Carboxy substituiert sein kann, oder

f) einen nach a) hergestellten 3-Chlor(oder Brom)-2-isoxazolin-phosphonsäuredi- oder monoester oder -phosphinsäureester der Formel I, in der n eine ganze Zahl von 0 bis 2 bedeutet, mit Tri$(C_1-C_4)$-alkylhalogensilanen unter Esterspaltung und gleichzeitigem Austausch von Chlor bzw. Brom gegen das Halogenatom des jeweils eingesetzten Silans zu den entsprechenden 3-Halogen-2-isoxazolinphosphon- oder -phosphinsäuren der Formel I umsetzt oder

g) eine nach a) bis f) erhaltene Verbindung der Formel I, die aufgrund ihrer chemischen Struktur in diastereomeren oder enantiomeren Formen auftritt, in an sich bekannter Weise in die reinen Stereoisomeren spaltet,

wobei man die nach a) bis g) hergestellten Verbindungen der Formel I entweder in freier Form isoliert oder gegebenenfalls in physiologisch verträgliche Salze umwandelt.

Die Herstellung physiologisch verträglicher Salze aus zur Salzbildung befähigten Verbindungen der Formel I, einschließlich deren gegebenenfalls stereoisomeren Formen, erfolgt in an sich bekannter Weise. So bilden die Phosphon- und Phosphinsäuren sowie die Phosphonsäurehalbester mit basischen Reagenzien, wie Hydroxiden, Alkoholaten, Carbonaten, Hydrogencarbonaten, Ammoniak oder organischen Basen, beispielsweise Trimethyl- oder Triethylamin, Ethanolamin oder auch basischen Aminosäuren, etwa Lysin, Ornithin oder Arginin, stabile Alkali-, beispielsweise Natrium- oder Kalium-, Erdalkali-, wie Calcium- oder Magnesium-, bzw. gegebenenfalls substituierte Ammoniumsalze, wobei im Falle der Phosphonsäuren durch Umwandlung nur einer der beiden sauren OH-Gruppen in die Salzform auch stabile Hydrogenphosphonate erhältlich sind.

Sofern die Verbindungen der Formel I eine basische Gruppe im Rest $R^1$ aufweisen, lassen sich mit starken Säuren auch stabile nichttoxische Säureadditionssalze herstellen. Hierfür kommen sowohl anorganische als auch organische Säuren,wie Chlorwasserstoff-, Bromwasserstoff-, Schwefel-, Phosphor-, Methansulfon-, Benzolsulfon-, p-Toluolsulfon-, 4-Brombenzolsulfon-, Cyclohexylamidosulfon- oder Trifluormethylsulfonsäure in Frage.

Die bei der 1,3-dipolaren Cycloaddition an die Olefine III und IV nach den Verfahrensvarianten a) bzw. b) als Ausgangsstoffe verwendeten Nitriloxide der Formel II sind größtenteils bekannt oder lassen sich nach literaturbekannten Methoden herstellen. So können sie beispielsweise durch Dehydratisierung von aliphatischen oder araliphatischen Nitroverbindungen, vorteilhaft mit Isocyanaten, wie Phenylisocyanat oder 1,4-Diisocyanatobenzol, nach der Methode von Mukaiyama (J. Am. Chem. Soc. 82 (1960), 5339 - 5342) oder, ausgehend von Hydroxamoylhalogeniden, die ihrerseits nach ebenfalls literaturbekannten Methoden, etwa durch Halogenierung von Aldoximen (K. C. Liu et al., J. Org. Chem. 45 (1980), 3916 - 3918; C. J. Peake et al., Synth. Commun. 16 (1986), 763 - 765; D. M. Vyas et al., Tetrahedron Lett. 25 (1984), 487 - 490), zugänglich sind, durch basenkatalysierte Dehydrohalogenierung, vorzugsweise nach dem von Huisgen entwickelten "In situ"-Verfahren (Chem. Ber. 106 (1973), 3258 - 3274), hergestellt werden.

Die weiterhin als Reaktionspartner dienenden olefinischen Phosphorverbindungen der Formeln III und IV sind ebenfalls zumeist literaturbekannt oder sogar käuflich, wie etwa der Vinylphosphonsäurediethylester, oder aber nach in der Literatur beschriebenen Verfahren (H. J. Kleiner et al., Angew. Chem. 94 (1982), 561 - 562; T. Ya. Medved et al., Zh. Akad. Nauk. SSSR, Ser. Khim. 1956, 684; Deutsche Offenlegungsschrift 2601467) leicht herstellbar. Geeignete Verbindungen der Formel IV sind vornehmlich solche, in denen die Abgangsgruppe W Methoxy oder Ethoxy, Alkylsulfonyloxy, beispielsweise Methyl- oder Trifluormethylsulfonyloxy, oder Arylsulfonyloxy, wie etwa Benzol-, p-Toluol-oder 4-Brombenzolsulfonyloxy, vorzugsweise jedoch Halogen, insbesondere Brom oder Chlor, bedeutet.

In der Regel werden die Erzeugung der Nitriloxide II entweder aus den Nitroverbindungen nach Mukaiyama oder aber aus den Hydroxamoylhalogeniden nach Huisgen und deren 1,3-dipolare Cycloaddition an die olefinische Phosphorverbindungen III und IV, die man im Falle der Phosphon- und Phosphinsäuren vorteilhaft in Form der Ester, beispielsweise der Methyl- oder Ethylester einsetzt, in einer sogenannten Eintopfreaktion ohne Isolierung der jeweiligen Zwischenprodukte durchgeführt, wobei es sich empfiehlt, in einem gegenüber den Reaktionspartnern inerten, aprotischen Lösungs- oder Verteilungsmittel zu arbeiten. Hierfür kommen z. B. Ethylacetat, Dimethylformamid, Dimethylacetamid, Dimethylsulfoxid, Ether, wie Diisopropylether, Diethylether, tert.Butylmethylether und Tetrahydrofuran, halogenierte Kohlenwasserstoffe, wie Dichlormethan oder Chloroform, Kohlenwasserstoffe, wie Hexan, Cyclohexan, Benzol, Toluol und andere substituierte aromatische Kohlenwasserstoffe sowie Mischungen der genannten Lösungsmittel, vorzugweise jedoch die aliphatischen Ether oder aromatischen Kohlenwasserstoffe in Frage. Bei der Hydroxamoylhalogenid-Methode nach Huisgen kann die Cycloaddition beim Einsatz von anorganischen Basen zur Nitriloxiderzeugung zusätzlich auch in zweiphasigen Lösungsmittelgemischen, beispielsweise Ethylacetat/Wasser oder Dichlormethan/Wasser durchgeführt werden. Bei Verwendung organischer Basen zur Dehydrohalogenierung arbeitet man hingegen bevorzugt in den vorgenannten chlorierten Kohlenwasserstoffen oder aliphatischen Ethern. Die Herstellung der Nitriloxide und die Cycloaddition erfolgen in der Regel bei Temperaturen zwischen - 20° C und + 50° C, vorzugsweise jedoch zwischen 0° und + 40° C.

Für die basenkatalysierte Umwandlung der Isoxazoline V in die Isoxazole der Formel I unter Eliminierung von HW müssen die Zwischenprodukte V ebenfalls nicht isoliert werden, sondern können vorteilhaft durch Anwendung der zur Nitriloxidsynthese benutzten Base bis zu einem 5-fachen, bevorzugt jedoch in einem zweifachen, Überschuß direkt in die Isoxazole überführt werden. Als Basen eignen sich hierfür beispielsweise Natrium- oder Kaliumhydroxid oder -carbonat sowie organische Amine, wie Mono-, Di- oder Trialkylamine, vorzugsweise jedoch Trialkylamine, wie zum Beispiel Trimethyl- oder Triethylamin. Aber auch durch thermische Eliminierung von HW bei Temperaturen oberhalb 50° C können die Isoxazoline V im allgemeinen in die Isoxazole der Formel I umgewandelt werden.

Die Esterspaltung der Phosphon- und Phosphinsäureester der Formel I zu den entsprechenden Phosphonsäurehalbestern oder Phosphon- bzw. Phosphinsäuren der Formel I nach Verfahrensweise c) erfolgt mit Hilfe dem Fachmann bekannter Standardverfahren unter Verwendung saurer oder alkalischer-Reagenzien. So kann die Umsetzung sowohl mit anorganischen als auch organischen Säuren oder Basen in wäßriger Lösung oder protischen organischen Solventien durchgeführt werden. Auch die Verwendung von Trialkylsilylhalogeniden in aprotischen Lösungsmitteln ist möglich.

Die Umwandlung der Phosphonsäurediester in die entsprechenden Phosphonsäuren gelingt vorteilhaft unter sauren Bedingungen. Besonders geeignet erweist sich das Arbeiten in wasserfreiem Medium mit Halogenwasserstoffen, wie Chlor-, Brom- oder Jodwasserstoff, in organischen Carbonsäuren, beispielsweise Ameisen- oder Essigsäure, wobei das System aus Bromwasserstoff und Eisessig wiederum bevorzugt ist. Die Reaktion wird mit einer 0,5- bis 4-normalen, vorzugsweise mit einer 2- bis 4-normalen, HBr/Eisessig-Lösung bei Temperaturen zwischen 0° und 100° C, bevorzugt jedoch zwischen 20° und 50° C, durchgeführt.

Zur Herstellung der Phosphonsäurehalbester werden die Phosphonsäurediester in der Regel einer alkalischen Hydrolyse, vorzugsweise in wäßrigem Milieu, unterworfen. Dabei wird zum Auflösen des Diesters vorteilhaft ein mit Wasser mischbares, organisches Lösungsmittel, zum Beispiel ein niederer Alkohol, wie Methanol oder Ethanol, verwendet, und danach eine 0,1- bis 5-normale, vorzugsweise jedoch 0,5- bis 2-normale, wäßrige Base, beispielsweise Natrium-oder Kaliumhydroxid, zugegeben. Die Base kann in stöchiometrischen Mengen oder in bis zu 10-fachem, vorzugsweise in 2- bis 4-fachem, Überschuß eingesetzt werden. Die Reaktion wird bei Temperaturen zwischen 0° C und dem Siedepunkt des verwendeten Reaktionsmediums durchgeführt. Bevorzugt ist ein Temperaturbereich von 0° bis 50° C, insbesondere von 20° bis 40° C.

Beide voranstehend beschriebenen Verfahrensweisen zur Esterspaltung eignen sich gleichermaßen für die Darstellung der Phosphinsäuren aus den entsprechenden Phosphinsäureestern.

Die Phosphonsäuren, Phosphonsäurehalbester und Phosphinsäuren sind in der Regel als kristalline Produkte isolierbar, die zum Teil beim Umkristallisieren aus Wasser oder wasserhaltigen Lösungsmittelgemischen stabile Hydrate bilden können.

Die Aminolyse von Phosphonsäurediestern der Formel I mit den primären oder sekundären Aminen der Formel VI zu den entsprechenden Phosphonsäurehalbesterhalbamiden der Formel I nach Verfahrensweise d) kann entweder in Substanz oder in protischen und aprotischen Lösungsmitteln bei in der Regel erhöhten Temperaturen durchgeführt werden. Als Amine VI kommen bevorzugt niedere Mono- oder insbesondere Dialkylamine, beispielsweise Methyl- und Ethylamin bzw. Dimethyl- und Diethylamin, sowie cyclische Amine, wie Pyrrolidin, Piperidin und Morpholin, in Frage. Geeignete Lösungsmittel sind unter anderem halogenierte Kohlenwasserstoffe, Ether, aromatische Kohlenwasserstoffe und Alkohole, vorzugsweise niedere Alkohole, wie Methanol und Ethanol, sowie cyclische Ether, etwa Dioxan. Das jeweilige Amin VI wird in 2- bis 100-fachem, vorteilhaft in 5- bis 10-fachem, Überschuß eingesetzt. Die Reaktionstemperatur wird

gewöhnlich im Bereich zwischen 40° und 100° C, vorzugsweise 60° und 80° C, gewählt.

Für die Ester- und/oder Amidbildung aus den Phosphonsäuren, Phosphonsäurehalbestern und Phosphinsäuren der Formel I nach Verfahrensweise e) bedient man sich vorteilhaft der am Phosphoratom aktivierten Säurederivate. Zur Aktivierung der (P-OH)-Gruppen können die aus der Nucleotidchemie bekannten Reagenzien, beispielsweise 1-Mesitylsulfonyl-3-nitro-1H-1,2,4-triazol und Mesitylsulfonylchlorid zusammen mit Tetrazol, oder - in einfacherer Variante - Phosphorhalogenide, wie Phosphortrihalogenide, hier vorzugsweise Phosphortrichlorid, sowie Phosphoroxychlorid und Phosphorpentahalogenide, hier vorzugsweise Phosphorpentachlorid, verwendet werden.

Die auf diese Weise aus den Phoshonsäuren der Formel I erhaltenen Säurederivate mit zwei reaktiven Gruppen lassen sich nach dem Fachmann hinlänglich bekannten Standardverfahren

mit einem Äquivalent Alkohol der Formel VII, vorzugsweise in Form eines Alkali- oder Erdalkali-Alkoholats, zu Monoestern der Formel I,

mit mindestens zwei Äquivalenten Alkohol VII zu Diestern der Formel I,

nacheinander mit je einem Äquivalent zweier verschiedener Alkohole VII zu gemischten Diestern der Formel I,

mit einem Äquivalent Diol VIII zu cyclischen Estern der Formel I,

nacheinander mit je einem Äquivalent Alkohol VII und Amin VI zu Halbesterhalbamiden der Formel I,

mit einem Äquivalent Amin VI zu Monoamiden der Formel I,

mit mindestens zwei Äquivalenten Amin VI zu Diamiden der Formel I oder

nacheinander mit je einem Äquivalent zweier verschiedener Amine VI zu gemischten Diamiden der Formel I umsetzen.

Analog werden aus den Phosphonsäurehalbestern der Formel I nach Aktivierung der freien (P-OH)-Gruppe durch Umsetzung mit einem Äquivalent Alkohol VII gegebenenfalls gemischte Diester der Formel I oder mit einem Äquivalent Amin VI Halbesterhalbamide der Formel I erhalten.

Ebenso reagieren die Phosphinsäuren nach Aktivierung der (P-OH)-Gruppe mit einem Äquivalent Alkohol VII oder Amin VI zu den entsprechenden Phosphinsäureestern bzw. -amiden der Formel I.

Auf diesem Wege lassen sich besonders vorteilhaft auch die im allgemeinen als labil bekannten P-Alkoxy- und P-Alkylphosphapeptide herstellen, wenn als Aminkomponente VI Aminosäuren, vornehmlich neutrale Aminosäuren, wie Glycin, Alanin, Valin, Leucin oder Isoleucin, zweckmäßig in carboxygeschützter Form, beispielsweise als Benzyl- oder tert.Butylester, eingesetzt werden.

Erfolgt die Aktivierung der (P-OH)-Gruppen mit Phosphorhalogenverbindungen, so wird die Reaktion zur Knüpfung der Amidbindung vorteilhaft mit einer mindestens zweifach stöchiometrischen Menge des jeweiligen Amins VI oder aber in Gegenwart einer in mindestens stöchiometrischer Menge eingesetzten zweiten Base, vorzugsweise eines tertiären Amins, wie Triethylamin, Morpholin oder auch Pyridin, durchgeführt, um den freiwerdenden Halogenwasserstoff zu binden. Bei Verwendung der Arylsulfonylverbindungen als Aktivierungsreagenzien dienen vornehmlich dipolare aprotische Lösungsmittel, wie Dioxan, Pyridin oder Dimethylformamid, als Reaktionsmedium. Wird die Aktivierung hingegen mit Phosphorhalogeniden vorgenommen, so bewähren sich außerdem auch aromatische und halogenierte Kohlenwasserstoffe. Die Reaktionstemperaturen liegen dabei in der Regel zwischen - 20° und + 40° C, vorzugsweise jedoch zwischen 0° und 20° C.

Der Halogenaustausch in den 3-Chlor(oder Brom)-2-isoxazolin-phosphon- oder -phosphinsäureestern der Formel I mit Trialkylhalogensilanen unter gleichzeitiger Esterspaltung nach Verfahrensweise f) wird zweckmäßig in einem dipolaren aprotischen Lösungsmittel bei Temperaturen zwischen etwa 0° C und dem Siedepunkt des jeweils verwendeten Reaktionsmediums durchgeführt. Besonders bewährt sich das Arbeiten in halogenierten Kohlenwasserstoffen, wie etwa Dichlormethan. Geeignete Trialkyhalogensilane stellen beispielsweise Trimethylchlor- und Trimethylbromsilan dar.

Die Trennung jener Verbindungen der Formel I, die aufgrund ihrer chemischen Struktur in diasteromeren oder enantiomeren Formen auftreten und bei der Synthese als deren Gemische anfallen, in die reinen Stereoisomeren nach Verfahrensweise g) erfolgt entweder durch Chromatographie an einem gegebenenfalls chiralen Trägermaterial oder, sofern die racemischen Verbindungen der Formel I zur Salzbildung befähigt sind, durch fraktionierte Kristallisation der mit einer optisch aktiven Base oder Säure als Hilfsstoff gebildeten diastereomeren Salze.

Aber auch der stereoeinheitliche Aufbau von Verbindungen der Formel I ist prinzipiell möglich, wenn man bei den Verfahrensvarianten b) bis f) die betreffenden Ausgangsstoffe in stereoisomerenreiner Form zu Synthese einsetzt.

Dies trifft in ganz besonderem Maße für die Herstellung der Phosphapeptide durch Umsetzung der aktivierten Säurederivate von Phosphonsäure, Phosphonsäurehalbestern und Phosphinsäuren der Formel I mit Aminosäuren nach Verfahrensweise e) zu.

Als chirale Stationärphasen für die dünnschicht- oder säulenchromatographische Trennung von Racematen, insbesondere auch der nach Verfahrensvariante a) in der Regel racemisch anfallenden 2-Isoxazoline mit asymmetrischen C-Atom in Ringposition 5, eignen sich beispielsweise modifizierte Kieselgelträger (sogenannte Pirkle-Phasen) sowie hochmolekulare Kohlenhydrate, wie Cellulose, Tribenzoylcellulose und - insbesondere bei Trennungen im größeren präparativen Maßstab - Triacetylcellulose. Diese optisch aktiven Trägermaterialien sind kommerziell erhältlich. Als mobile Phasen werden solche Lösungsmittel bzw. Lösungsmittelgemische verwendet, die mit den funktionellen Gruppen der aufzutrennenden stereoisomeren Verbindungen keine Reaktion eingehen können.

Zur Enantiomerentrennung etwa der racemischen Phosphonsäuren, deren sauren Halbester und Phosphinsäuren der Formel I durch fraktionierte Kristallisation werden nach dem Fachmann bekannter Verfahrensweise mit Hilfe einer optisch aktiven Base die beiden unterschiedlich löslichen diastereomeren Salze gebildet, die schwerer lösliche Komponente als Feststoff abgeschieden, das leichter lösliche Diastereomer aus der Mutterlauge isoliert und die so gewonnenen reinen Diastereomeren in die erwünschten Enantiomeren zerlegt. Als leicht zugängliche optisch aktive Basen seien bevorzugt Aminbasen, beispielsweise (-)-Nicotin, (-)-Brucin, (+)- und (-)-1-Phenylethylamin, (-)-Norephedrin, (+)-Norpseudoephedrin, (+)-3-Aminomethylpinan, (-)-Chinin, (+)-Chinidin, (-)-Cinchonidin, (+)-Cinchonin, L-Lysin, und L- oder D-Arginin, genannt.

Die Phosphon- und Phosphinsäuren sowie die sauren Phosphonsäurehalbester der Formel I bilden auch mit quartären organischen Basen, beispielsweise mit käuflichen hochmolekularen Anionenaustauschern in der Hydroxidform, wie etwa Amberlite® IRA 402 oder dem flüssigen Ionenaustauscher Amberlite® LA-2, stabile Salze. Von dieser Eigenschaft läßt sich bei der Reinigung der gewonnenen Rohsäuren vorteilhaft Gebrauch machen, indem man sie aus wäßriger Lösung mit in einem organischen, mit Wasser wenig mischbaren Lösungsmittel, wie Toluol, Cyclohexan oder Ethylacetat, gelöstem Amberlite® LA-2 extrahiert. Die Ablösung der Säuren vom Austauscherharz erfolgt zweckmäßigerweise mit starken wäßrigen Basen im Überschuß, vorrangig mit 1 bis 25%-iger, vorzugsweise jedoch 5 bis 15%-iger wäßriger Ammoniaklösung. Die dabei anfallenden reinen Ammoniumsalze können als solche kristallisiert oder aber in alkoholischer, bevorzugt methanolischer, oder wäßriger Lösung mit Säuren oder sauren Ionenaustauschern, z.B. Amberlyst® 15, in die freien Säuren überführt werden. Dieses einfach durchführbare Reinigungsverfahren bietet deutliche Vorteile gegenüber den konventionellen Methoden zur Reindarstellung durch Kristallisation oder Chromatographie.

Die erfindungsgemäßen Arzneimitel, die als Wirkstoffe die Verbindungen der Formel I, gegebenenfalls in stereoisomerenreiner Form und/oder als physiologisch verträgliche Salze, entweder für sich allein, beispielsweise in Mikrokapseln, in Mischungen untereinander oder vorzugsweise in Kombination mit geeigneten pharmazeutischen Trägerstoffen, Verdünnungsmitteln und/oder anderen Hilfsstoffen enthalten, können parenteral, rektal oder oral verabreicht werden.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoffe-Freigabe, bei deren Herstellung üblicherweise Hilfsmittel, wie Trägerstoffe, Spreng-, Binde-, Überzugs-, Quellungs-, Gleit-oder Schmiermittel, Geschmacksstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, wie Lebertran, Sonnenblumen-, Erdnuß- oder Sesamöl, Polyethylenglykole und Lösungsmittel, wie etwa steriles Wasser, physiologische Kochsalzlösung und ein- oder mehrwertige Alkohole, z. B. Glycerin, genannt. Bei der Herstellung von wäßrigen Lösungen der stark sauer reagierenden Phosphon- und Phosphinsäuren sowie Phosphonsäurehalbester gemäß Formel I wird der Wirkstoff zweckmäßig so formuliert, daß er in Salzform mit physiologisch verträglichem pH-Wert vorliegt.

Vorzugsweise werden die pharmazeutischen Präparate in Dosierungseinheiten hergestellt und verabreicht, wobei jede Einheit als aktiven Bestandteil eine bestimmte Dosis mindetens einer Verbindung gemäß Formel I, gegebenenfalls in stereoisomerenreiner und/oder Salzform, enthält. Bei festen Dosierungseinheiten, wie Tabletten, Kapseln, Dragees oder Suppositorien, kann diese Dosis bis zu etwa 1000 mg, bevorzugt jedoch etwa 50 bis 300 mg, und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindungen gemäß Formel I, gegebenenfalls in stereoisomerenreiner und/oder Salzform, bei Mensch und Tier - Tagesdosen von etwa 50 bis 3000 mg Wirkstoff, vorzugsweise etwa 150 bis 1000 mg, bei oraler Verabreichung und von etwa 50 bis 1000 mg, bevorzugt etwa 100 bis 300 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit

oder aber mehrerer kleinerer Dosierungseinheiten als auch durch Mehrfachgabe unterteilter Dosen in bestimmten Intervallen erfolgen.

Schließlich können die Verbindungen der Formel I, ihre gegebenenfalls stereoisomeren Formen und/oder gegebenenfalls ihre physiologisch verträglichen Salze bei der Herstellung der vorgenannten galenischen Zubereitungsformen auch zusammen mit anderen geeigneten Wirkstoffen, beispielsweise antibakteriellen, antimykotischen, antiviralen oder auch anderen tumorhemmenden Mitteln und/oder klassischen Antiphlogistika oder Antirheumatika, formuliert werden. Darüber hinaus eignen sie sich aufgrund ihrer immunmodulierenden Eigenschaften in hervorragender Weise für die Adjuvierung von Impfstoffen.

Beispiele

Die folgenden Beispiele erläutern die Erfindung, ohne sie jedoch in ihrem Umfang einzuschränken.
Die Strukturen der nachstehend beschriebenen Verbindungen wurden durch Elementaranalysen, IR- und $^{1}H$-NMR-Spektren, in Einzelfällen auch über $^{13}C$- und $^{31}P$-NMR-Spektren gesichert.

Beispiel 1: 3-Phenyl-2-isoxazolin-5-yl-phosphonsäurediethylester (nach Verfahrensweise a))

a) Benzhydroxamoylchlorid

58.7 g (0.44 mol) N-Chlorsuccinimid werden in 300 ml Dichlormethan unter Zusatz von 2 ml Pyridin suspendiert und 48.5 g (0.4 mol) Benzaldoxim, gelöst in 150 ml Dichlormethan, unter Rühren und exotherm verlaufender Reaktion zugetropft. Das Reaktionsgemisch wird anschließend noch 30 Minuten unter Rückfluß erhitzt, dann abgekühlt, und direkt zur Cycloaddition eingesetzt.

b) Cycloaddition mit Vinylphosphonsäurediethylester

72.2 g (0.44 mol) Vinylphosphonsäurediethylester, gelöst in 100 ml Dichlormethan, werden zu der unter a) hergestellten Lösung gegeben und dann unter Rühren bei Raumtemperatur tropfenweise mit 61.2 ml (0.44 mol) Triethylamin, gelöst in 200 ml Dichlormethan, innerhalb von 6 Stunden versetzt. Man läßt über Nacht bei Raumtemperatur stehen, filtriert und schüttelt nacheinander mit wäßriger Natriumhydrogencarbonat-Lösung, 1 N Citronensäure und mehrmals mit Wasser aus. Nach dem Trocknen und Einengen verbleiben 122 g eines gelben Öles, das durch Säulenchromatographie an Kieselgel (Laufmittel: Ethylacetat/Petrolether 1 : 1; Vol.) oder durch Destillation unter vermindertem Druck analysenrein erhalten werden kann.
$^{1}H$-NMR ($CDCl_3$):
$\delta$ = 1.2 - 1.6 (2t, J = 7 Hz, 6H, $P(OEt)_2$), 3.4 - 4.6 (m, 6H, 4-H und $P(OEt)_2$), 4.75 - 5.25 (mc, 1H, 5-H), 7.45 - 8.1 ppm (m, 5H, Phenyl-H).

| $C_{13}H_{18}NO_4P$ (283.3) | | | | |
|---|---|---|---|---|
| Analyse: | Ber.:<br>Gef.: | C 55.12<br>C 55.50 | H 6.41<br>H 6.70 | N 4.95<br>N 5.25 |

Beispiel 2: 3-Phenyl-2-isoxazolin-5-yl-methylphosphonsäurediethylester

Analog Beispiel 1 werden ausgehend von 30.3 g (0.25 mol) Benzaldoxim, 36.7 (0.275 mol) N-Chlorsuccinimid, 49 g (0.275 mol) Allylphosphonsäurediethylester und 38.2 ml (0.275 mol) Triethylamin, 76.7 g eines öligen Rohproduktes erhalten, das, wie in Beispiel 1 beschrieben, gereinigt werden kann.
$^{1}H$-NMR ($CDCl_3$):
$\delta$ = 1.1 - 1.45 (2t, J = 7 Hz, 6H, $P(OEt)_2$), 1.8 - 2.8 (m, 2H, $CH_2$-P), 3.15 - 3.49 (m, 2H, 4-H), 3.75 - 4.3 (m, 4H, $P(OEt)_2$), 4.65 - 5.1 (mc, 1H, 5-H), 7.05 - 7.6 ppm (m, 5H, Phenyl-H).

Beispiel 3: 3-(4-Chlorphenyl)-2-isoxazolin-5-yl-phosphonsäurediethylester

Die Verbindung wird analog Beispiel 1 aus 23.34 g (0.15 mol) 4-Chlorbenzaldoxim, 22 g N-Chlorsuccinimid, 27.1 g Vinylphosphonsäurediethylester und 22.9 ml Triethylamin hergestellt, wobei man 49 g öliges Produkt erhält.

$^1$H-NMR ($CDCl_3$):
= 1.19 - 1.5 (2t, J = 7 Hz, 6H, $P(OEt)_2$), 3.2 - 4.45 (m, 6H, 4-H und $P(OEt)_2$), 4.55 - 5.0 (mc, 1H, 5-H), 7.2 und 7.45 ppm (AA'BB', 4H, Aryl-H).

Beispiel 4: 3-(4-Chlorphenyl)-2-isoxazolin-5-yl-methyl-phosphonsäurediethylester

Die Herstellung erfolgt wie bei Beispiel 1 aus 23.34 g 4-Chlorbenzaldoxim, 22 g N-Chlorsuccinimid, 29.5 g Allylphosphonsäurediethylester und 22.9 ml Triethylamin in Dichlormethan und liefert 52 g öliges Produkt.
$^1$H-NMR ($CDCl_3$):
= 1.15 - 1.5 (2t, J = 7 Hz, 6H, $P(OEt)_2$), 1.8 - 2.8 (m, 2H, $CH_2$-P), 3.15 - 3.45 (m, 2H, 4-H), 3.7 - 4.35 (m, 4H, $P(OEt)_2$), 4.7 - 5.1 (mc, 1H, 5-H), 7.22 und 7.5 ppm (AA'BB', 4H, Aryl-H).

Beispiel 5: 3-Phenyl-2-isoxazolin-5-yl-phosphonsäuredimethylester

Gemäß Beispiel 1 werden 58.2 g (0.374 mol) Benzhydroxamoylchlorid (hergestellt wie in Beispiel 1a) aus Benzaldoxim und N-Chlorsuccinimid in Dimethylformamid, dann nach Zusatz von Eiswasser mit Diethylether extrahiert und als Öl isoliert), 50.9 g (0.375 mol) Vinylphosphonsäuredimethylester und 52 ml (0.375 mol) Triethylamin in 1.1 l Diethylether umgesetzt. Das nach üblicher Aufarbeitung in einer Rohausbeute von 68.5 g erhaltene Öl kann durch Kristllisation aus Methanol/Diethylether oder Dichlormethan/Diethylether gereinigt werden, wobei man 60.6 g Reinprodukt mit einem Schmelzpunkt von 75° bis 77° C erhält.
$^1$H-NMR ($CDCl_3$):
$\delta$ = 3.25 - 3.9 (m, 8H, 4-H sowie $P(OMe)_2$ als d, J = 10 Hz bei 3.77), 4.55 - 5.05 (mc, 1, 5-H), 7.1 - 7.65 ppm (m, 5H, Phenyl-H).

| $C_{11}H_{14}NO_4P$ (255.3) | | | | |
|---|---|---|---|---|
| Analyse: | Ber.:<br>Gef.: | C 51.77<br>C 51.79 | H 5.53<br>H 5.62 | N 5.49<br>N 5.45 |

Beispiel 6: 3-(2-Pyridyl)-2-isoxazolin-5-yl-phosphonsäurediethylester

a) 2-Pyridylhydroxamoylchlorid-hydrochlorid

Die Herstellung erfolgt durch Chlorierung von Pyridin-2-carbaldoxim in Dichlormethan in Anlehnung an eine Literaturvorschrift (Bull. Soc. Chim. France 1962, 2215), Kristallisiert wird direkt aus der Reaktionslösung durch Zugabe von Diethylether nach Entfernen von überschüssigem Chlor durch kurzzeitiges Evakuieren. Die Ausbeute beträgt 95.5%. Der Schmelzpunkt liegt bei 173° bis 178° C (Zers.).

b) Cycloaddition mit Vinylphosphonsäurediethylester (nach Verfahrensweise a))

77.8 g (0.4 mol) des unter a) hergestellten Hydroxamoylchlorides werden in 1 l Tetrahydrofuran suspendiert, 72.2 g (0.44 mol) Vinylphosphonsäurediethylester zugegeben, und die Hälfte einer Lösung von 111.2 ml (0.8 mol) Triethylamin in 200 ml Tetrahydrofuran während einer Stunde, der Rest während weiterer 5 Stunden unter intensivem Rühren zugetropft. Man rührt über Nacht, filtriert, engt ein, versetzt mit Wasser und extrahiert das Produkt mit Ethylacetat. Es werden 111 g öliges Isoxazolin erhalten.
$^1$H-NMR ($CDCl_3$):
$\delta$ = 1.3 (tb, J = 7 Hz, 6H, $P(OEt)_2$), 3.4 - 4.45 (m, 6H, 4-H und $P(OEt)_2$), 4.6 - 5.05 (mc, 1H, 5-H), 7.0 - 8.0 (m, 3H, Pyridyl-H), 8.35 - 8.55 ppm (m, 1H, Pyridyl-6-H).

Beispiel 7: 3-(4-Pyridyl)-2-isoxazolin-5-yl-phosphonsäurediethylester

Die Herstellung erfolgt analog Beispiel 6 aus 17 g (0.088 mol) 4-Pyridyl-hydroxamoylchlorid-hydrochlorid, 15.8 g (0.096 mol) Vinylphosphonsäurediethylester und 26.6 ml (0.192 mol) Triethylamin. Man erhält 17.8 g öliges Produkt.
$^1$H-NMR (DMSO-$d_6$):
$\delta$ = 1.23 (tb, J = 7 Hz, 6H, $P(OEt)_2$), 3.2 - 4.3 (m, 6H, 4-H und $P(OEt)_2$), 4.75 - 5.24 (mc, 1H, 5-H), 7.5 und

8.55 ppm (AA'BB', 4H, Pyridyl-H).

Beispiel 8: 3-(4-Methoxyphenyl)-2-isoxazolin-5-yl-phosphonsäurediethylester

Die Umsetzung von 45.35 g (0.3 mol) 4-Methoxybenzaldoxim, 40 g (0.3 mol) N-Chlorsuccinimid, 54.1 g (0.33 mol) Vinylphosphonsäurediethylesterund 46 ml (0.33 mol) Triethylamin analog Beispiel 1 ergibt 90.5 g öliges Produkt.
$^1$H-NMR ($CDCl_3$):
$\delta$ = 1.3 (tb, J = 7 Hz, 6H, $P(OEt)_2$), 3.15 - 4.4 (m, 9H, 4-H, OMe und $P(OEt)_2$), 4.5 - 4.95 (mc, 1H, 5-H), 6.75 und 7.43 ppm (AA'BB', 4H, Aryl-H).

Beispiel 9: 3-(3-Phenoxyphenyl)-2-isoxazolin-5-yl-phosphonsäuredimethylester

Analog Beispiel 1 werden 42.65 g (0.2 mol) 3-Phenoxybenzaldoxim, 29.4 g (0.22 mol) N-Chlorsuccinimid, 29.2 ml (0.22 mol) Vinylphosphonsäuredimethylester und 30.6 ml (0.22 mol) Triethylamin umgesetzt und 66.4 g öliges Produkt erhalten.
$^1$H-NMR ($CDCl_3$):
= 3.15 - 3.95 (m, 8H, 4-H und $P(OMe)_2$), 4.5 - 5.0 (mc, 1H, 5-H), 6.7 - 7.4 ppm (m, 9H, Aromaten-H).

Beispiel 10: 3-(1-Naphthyl)-2-isoxazolin-5-yl-phosphonsäuredimethylester

Die Herstellung erfolgt analog Beispiel 1 aus 31.2 g (0.2 mol) 1-Naphthaldoxim, 29.4 g (0.22 mol) N-Chlorsuccinimid, 29.2 ml (0.22 mol) Vinylphosphonsäuredimethylester und 30.6 ml (0.22 mol) Triethylamin, wobei 56 g ölige Verbindung erhalten werden.
$^1$H-NMR ($CDCl_3$):
= 3.4 - 4.1 (m, 8H, 4-H und $P(OMe)_2$), 4.6 - 5.1 (mc, 1H, 5-H), 7.1 - 7.9 (m, 6H, Aromaten-H), 8.6 - 8.9 ppm (m, 1H, Aromaten-H).

Beispiel 11: 3-Styryl-2-isoxazolin-5-yl-phosphonsäurediethylester

Die Verbindung wird analog Beispiel 1 aus 73.6 g (0.5 mol) Styrylaldoxim, 73.4 g (0.55 mol) N-Chlorsuccinimid, 90.2 g (0.55 mol) Vinylphosphonsäurediethylester und 76.4 ml (0.55 mol) Triethylamin hergestellt. Man gewinnt 153 g öliges Produkt.
$^1$H-NMR (Methanol-$d_4$):
$\delta$ = 1.3 (tb, J = 7 Hz, 6H, $P(OEt)_2$), 3.2 - 4.35 (m, 6H, 4-H und $P(OEt)_2$), 4.5 - 5.0 (mc, 1H, 5-H), 6.8 (sb, 2H, Ph-C<u>H</u>=C<u>H</u>-), 6.95 - 7.6 ppm (m, 5H, Aryl-H).

Beispiel 12: 3-Benzoyl-2-isoxazolin-5-yl-phosphonsäurediethylester

Die Synthese des Benzoylhydroxamoylchlorides ist literaturbekannt (z. B.: J. Heterocyclic Chem. 21 - (1984), 1029). 45 g (0.245 mol) dieses Hydroxamsäurechlorides werden gemäß Beispiel 1 oder 6 mit 44.3 g (0.270 mol) Vinylphosphonsäurediethylester und 37.5 ml (0.270 mol) Triethylamin in insgesamt 600 ml tert.Butylmethylether umgesetzt. Die übliche Aufarbeitung liefert 73.9 g rotbraunes Öl.
$^1$H-NMR ($CDCl_3$):
$\delta$ = 1.3 (tb, J = 7 Hz, 6H, $P(OEt)_2$), 3.1 - 4.4 (m, 6H, 4-H und $P(OEt)_2$), 4.55 - 5.0 (mc, 1H, 5-H), 7.1 - 7.55 und 7.85 - 9.2 ppm (m, 5H, Aryl-H).

Beispiel 13: 3-Phenyl-2-isoxazolin-5-yl-(P-methyl)-phosphinsäuremethylester (nach Verfahrensweise a))

Die Herstellung erfolgt analog Beispiel 5 in Diethylether unter Einsatz von 11 g (0.07 mol) Benzhydroxamoylchlorid, 9.2 g (0.077 mol) Vinyl-(P-methyl)-phosphinsäuremethylester und 10.7 ml (0.077 mol) Triethylamin. Nach üblicher Aufarbeitung werden die wäßrigen Waschphasen vereinigt, bei pH 2 mit Dichlormethan extrahiert und dieser Extrakt zusammen mit der etherischen Phase getrocknet und eingeengt. Kristallisation des Rückstandes aus Diethylether liefert 12.2 g Produkt vom Schmelzpunkt 72° bis 74° C.
$^1$H-NMR ($CDCl_3$):
$\delta$ = 1.6 (d, J = 15 Hz, 3H, P-Me), 3.45 - 4.15 (m, 5H, 4-H und P-OMe), 4.75 - 5.25 (mc, 1H, 5-H), 7.45 - 8.05 ppm (m, 5H, Aryl-H).

| $C_{11}H_{14}NO_3P$ (239.2) | | | | |
|---|---|---|---|---|
| Analyse: | Ber.:<br>Gef.: | C 55.23<br>C 55.25 | H 5.90<br>H 6.00 | N 5.86<br>N 5.91 |

Beispiel 14: 3-(4-Methoxyphenyl)-2-isoxazolin-5-yl-(P-methyl)-phosphinsäuremethylester

Aus 45.35 g (0.3 mol) 4-Methoxybenzaldoxim, 40 g (0.3 mol) N-Chlorsuccinimid, 39.6 g (0.33 mol) vinyl-(P-methyl)-phosphinsäuremethylester und 46 ml (0.33 mol) Triethylamin werden analog Beispiel 1 72.3 g öliges Produkt gewonnen.
$^1$H-NMR ($CDCl_3$):
$\delta$ = 1.5 (d, J = 15 Hz, 3H, P-Me), 3.2 - 3.9 (m, 8H, 4-H, C-OMe und P-OMe), 4.5 - 4.95 (mc, 1H, 5-H), 6.75 und 7.43 ppm (AA'BB', 4H, Aryl-H).

Beispiel 15: 3-(4-Methylphenyl)-2-isoxazolin-5-yl-(P-methyl)-phosphinsäuremethylester

Analog Beispiel 1 erhält man aus 33.8 g (0.25 mol) 4-Toluyladoxim, 36.7 g (0.275 mol) N-Chlorsuccinimid, 33 g (0.275 mol) Vinyl-(P-methyl)-phosphinsäuremethylester und 38.2 ml (0.275 mol) Triethylamin nach Umkristallisation aus tert.Butylmethylether 36.5 g analysenreines Produkt vom Schmelzpunkt 96° - 100°C.
$^1$H-NMR ($CDCl_3$):
$\delta$ = 1.5 (d, J = 14 Hz, 3H, P-Me), 2.33 (s, 3H, Aryl-$CH_3$), 3.25 - 3.9 (m, 5H, 4-H und P-OMe), 4.5 - 4.95 (mc, 1H, 5-H), 7.05 und 7.4 ppm (AA'BB', 4H, Aryl-H).

| $C_{12}H_{16}NO_3P$ (253.2) | | | | |
|---|---|---|---|---|
| Analyse: | Ber.:<br>Gef.: | C 56.92<br>C 57.21 | H 6.37<br>H 6.44 | N 5.53<br>N 5.67 |

Beispiel 16: 3-(1-Naphthyl)-2-isoxazolin-5-yl-(P-methyl)-phosphinsäuremethylester

Gemäß Beispiel 1 werden 39. 1 g (0.25 mol) Naphthaldoxim, 36.7 g (0.275 mol) N-Chlorsuccinimid, 33 g (0.275 mol) Vinyl-(P-methyl)-phosphinsäuremethylester und 38.2 ml (0.275 mol) Triethylamin zu 70 g öligem Produkt umgesetzt.
$^1$H-NMR ($CDCl_3$):
$\delta$ = 1.55 (d, J = 14 Hz, 3H, P-Me), 3.4 - 4.1 (m, 5H, 4-H und P-OMe), 4.5 - 4.95 (mc, 1H, 5-H), 7.05 - 8.0 (m, 6H, Aryl-H) und 8.75 ppm (mc, 1H, Aryl-4-H).

Beispiel 17: 3-Phenyl-2-isoxazolin-5-yl-dimethylphosphinoxid (nach Verfahrensweise a))

Die Herstellung erfolgt analog Beispiel 1, indem man von 60.6 g (0.5 mol) Benzaldoxim, 73.45 g (0.55 mol) N-Chlorsuccinimid, 57.3 g (0.55 mol) Vinyldimethylphosphinoxid und 76.5 ml (0.55 mol) Triethylamin ausgeht. Nach üblicher Aufarbeitung wird aus tert.Butylmethylether umkristallisiert. Man erhält 64.4 g reines Produkt vom Schmelzpunkt 153°C.
$^1$H-NMR ($CDCl_3$):
$\delta$ = 1.4 und 1.6 (2d, J = 12 Hz, 6H, $PMe_2$), 3.25 - 3.85 (AB von ABX, 2H, 4-H), 4.4 - 4.95 (mc, 1H, 5-H), 6.95 - 7.55 ppm (m, 5H, Aryl-H).

| $C_{11}H_{14}NO_2P$ (223.2) | | | | |
|---|---|---|---|---|
| Analyse: | Ber.:<br>Gef.: | C 59.19<br>C 59.20 | H 6.32<br>H 6.43 | N 6.28<br>N 6.30 |

Beispiel 18: 3-Phenyl-isoxazol-5-yl-phosphonsäurediethylester (nach Verfahrensweise b))

Zu einer Lösung von 0.25 mol Benzhydroxamoylchlorid in Dichlormethan - hergestellt wie in Beispiel 1a) beschrieben - werden 66.8 g (0.275 mol) $\alpha$-Bromvinylphosphonsäurediethylester, gelöst in Dichlormethan, und anschließend innerhalb von 5.5 Stunden eine Lösung von 76. 4 ml (0.55 mol) Triethylamin in 250 ml Dichlormethan hinzugetropft. Man rührt über Nacht nach und arbeitet wie in Beispiel 1b) auf, wobei 76 g öliges Produkt anfallen.
$^1$H-NMR ($CDCl_3$):
$\delta$ = 1.38 (tb, 6H, $P(OEt)_2$), 4.2 (dq, 4H, $P(OEt)_2$), 7.2 (d, J = 1.5 Hz, 1H, 4-H), 7.3 - 7.9 ppm (m, 5H, Aryl-H).

Beispiel 19: 3-(4-Methoxyphenyl)-isoxazol-5-yl-phosphonsäurediethylester

Die Herstellung erfolgt gemäß Beispiel 18 aus 30.2 g (0.2 mol) 4-Methoxybenzaldoxim, 26.7 g (0.2 mol) N-Chlorsuccinimid, 1 ml Pyridin, 48.6 g (0.2 mol) $\alpha$-Bromvinylphosphonsäurediethylester und 61.2 ml (0.44 mol) Triethylamin in Dichlormethan. Nach üblicher Aufarbeitung werden 65 g eines braunen Öles erhalten.
$^1$H-NMR ($CDCl_3$):
$\delta$ = 1.4 (tb, 6H, $P(OEt)_2$), 3.95 (s, 3H, OMe), 3.95 - 4.6 (m, 4H, $P(OEt)_2$), 7.0 - 7.4 (m, 3H, 4-H und Ar-H), 7.85 - 8.1 ppm (m, 2H, Ar-H).

Beispiel 20: 3-(2-Pyridyl)-isoxazol-5-yl-phosphonsäurediethylester

Entsprechend den Beispielen 6 und 18 liefert die Umsetzung von 38.6 g (0.2 mol) 2 Pyridylhydroxamoylchloridhydrochlorid (vergl. Beispiel 6a)) mit 48.6 g (0.2 mol) $\alpha$-Bromvinylphosphonsäurediethylester unter tropfenweisem Zusatz von 83.4 ml (0.6 mol) Triethylamin in 1 1 Tetrahydrofuran 50.9 g öliges Produkt.
$^1$H-NMR ($CDCl_3$):
$\delta$ = 1.35 (tb, 6H, $P(OEt)_2$), 4.1 (dq, 4H, $P(OEt)_2$), 7.05 - 8.0 (m, 4H, Pyridyl-H und Isoxazol-4-H bei 7.35 ppm), 8.35 - 8.6 ppm (m, 1H, Pyridyl-6-H).

Beispiel 21 3-(4-Pyridyl)-isoxazol-5-yl-phosphonsäurediethylester

4-Pyridylhydroxamoylchlorid-hydrochlorid wird analog Beispiel 6 bzw. 7 hergestellt. Die Cyclisierung mit $\alpha$-Bromvinylphosphonsäurediethylester und Triethylamin erfolgt gemäß Beispiel 20 und liefert das erwünschte Isoxazol als öliges Produkt.
$^1$H-NMR ($CDCl_3$):
$\delta$ = 1.35 (tb, 6H, $P(OEt)_2$), 4.1 (dq, 4H, $P(OEt)_2$), 7.1 (d, J = 1.8 Hz, 1H, 4-H), 7.55 und 8.55 ppm (AA'BB', 4H, Pyridyl-H).

Beispiel 22: 3-Propyl-2-isoxazolin-5-yl-phosphonsäurediethylester (nach Verfahrensweise a))

In 180 ml tert.Butylmethylether werden 42.7 g (0.26 mol) Vinylphosphonsäurediethylester und 1.1 ml Triethylamin gelöst, 56.4 g (0.473 mol) Phenylisocyanat zugegeben und eine Lösung von 24.4 g (0.237 mol) Nitrobutan in 120 ml tert.Butylmethylether innerhalb von 7 Stunden zugetropft. Es wird 3 Tage lang nachgerührt, nach Zugabe von 30 ml 2 N ethanolischer Ammoniaklösung weitere 30 Minuten gerührt, filtriert mit Ethylacetat verdünnt und nacheinander mit wäßriger Ammoniaklösung, Wasser, 2 N HCl und erneut mit Wasser gewaschen. Nach dem Trocknen und Einengen verbleiben 27.3 g öliges Endprodukt.
$^1$H-NMR ($CDCl_3$):
$\delta$ = 0.8 - 1.8 (m, 11H, $P(OEt)_2$ und $CH_3$-$CH_2$-), 2.15 - 2.5 (mc, 2H, -$CH_2$-), 2.8 - 3.5 (m, 2H, 4-H), 3.8 - 4.8 ppm (m, 5H, $P(OEt)_2$ und 5-H).

Beispiel 23: 3-Benzyl-2-isoxazolin-5-yl-phosphonsäuredimethylester (nach Verfahrensweise a))

13.5 ml (0.124 mol) Phenylisocyanat, 9.5 g (0.07 mol) Vinylphosphonsäuredimethylester und 1 ml Triethylamin werden in 120 ml Toluol vorgelegt. Man tropft eine Lösung von 9.3 g (0.062 mol) 2-Phenylnitroethan und 1 ml Triethylamin, gelöst in 150 ml Toluol, während 5 Stunden hinzu, rührt über Nacht und danach noch 30 Minuten nach Zusatz von 30 ml konz. wäßrigem Ammoniak, filtriert vom ausgefallenen Diphenylharnstoff ab, wäscht mit Ethylacetat nach und schüttelt die vereinigten organischen

Phasen nacheinander mit Wasser, 2 N Salzsäure und Wasser aus, trocknet und engt ein. Es verbleiben 13.9 g rötlich braunes Öl.
$^1$H-NMR ($CDCl_3$):
δ = 2.7 - 3.85 (m, 10H, 4-H, Benzyl-H und $P(OMe)_2$), 4.3 - 4.8 (mc, 1H, 5-H), 6.8 - 7.25 ppm (m, 5H, Aryl-H).

Beispiel 24: 3-Benzyl-2-isoxazolin-5-yl-(P-methyl)-phosphinsäuremethylester

Die Herstellung erfolgt analog Beispiel 23 aus 37.1 ml (0.34 mol) Phenylisocyanat, 22.5 g (0.187 mol) Vinyl-P-methyl)-phosphinsäuremethylester, 4 ml Triethylamin und 25.7 g (0.17 mol) 2-Phenylnitroethan in Toluol, wobei man 34 g braunes Öl erhält.
$^1$H-NMR ($CDCl_3$):
δ = 1.35 (d, J = 14 Hz, 3H, P-Me), 2.7 - 3.75 (m, 7H, 4-H, Benzyl-H und P-OMe), 4.4 - 4.95 (mc, 1H, 5-H), 6.8 - 7.5 ppm (m, 5H, Aryl-H).

Beispiel 25: 3-tert.Butyl-2-isoxazolin-5-yl-(P-methyl)-phosphinsäuremethylester

40.4 g (0.4 mol) Pivalaldoxim werden analog Beispiel 1a) mit 58.7 g (0.44 mol) N-Chlorsuccinimid und 2 ml Pyridin in Dichlormethan in das Hydroxamoylchlorid überführt, das man anschließend mit 52.8 g (0.44 mol) Vinyl-(P-methyl)-phosphinsäuremethylester und 61.2 ml (0.44 mol) Triethylamin - wie in Beispiel 1b) beschrieben - umsetzt und aufarbeitet, wobei das ölige Produkt mit einer Ausbeute von ca. 50 % erhalten wird.
$^1$H-NMR ($CDCl_3$):
δ = 1.0 - 1.65 (m, 12H, tBu und P-Me), 2.8 - 3.75 (m, 5H, 4-H und P-OMe als d bei 3.6 ppm), 4.25 - 4.7 ppm (mc, 1H, 5-H).

Beispiel 26: 3-tert.Butyl-2-isoxazolin-5-yl-phosphonsäurediethylester

Die Durchführung erfolgt analog Beispiel 25 unter Einsatz von 15.2 g (0.15 mol) Pivalaldoxim, 22.0 g (0.165 mol) N-Chlorsuccinimid, 0.5 ml Pyridin, 27.1 g (0.165 mol) vinylphosphonsäurediethylester und 22.9 ml (0.165 mol) Triethylamin in Dichlormethan. Das Produkt wird nach üblicher Aufarbeitung als Öl erhalten.
$^1$H-NMR ($CDCl_3$):
δ = 1.1 - 1.5 (15H, tBu bei 1.2 ppm und $P(Oet)_2$), 2.95 - 3.5 (m, 2H, 4-H), 3.85 - 4.8 ppm (m, 5H, 5-H und $P(OEt)_2$).

Beispiel 27: 3-Phenyl-2-isoxazolin-5-yl-phosphonsäuremonoethylester (nach Verfahrensweise c))

10 g (35.3 mmol) des gemäß Beispiel 1 hergestellten Diethylesters werden in 140 ml Ethanol gelöst und nach Zusatz von 140 ml 1 N Natronlauge 30 Stunden bei Raumtemperatur gerührt. Man entfernt das Ethanol unter vermindertem Druck, ethert aus, säuert die Wasserphase mit Salzsäure bis pH 1 an und extrahiert mehrmals mit Dichlormethan. Nach dem Waschen mit halbkonzentrierter NaCl-Lösung und Einengen verbleibt ein zähes Öl, das aus Diethylether kristallisiert werden kann. Man erhält 7.2 g Reinprodukt vom Schmelzpunkt 84° bis 91° C.
$^1$H-NMR ($CDCl_3$):
δ = 1.35 (t, J = 7 Hz, 3 H, P-OEt), 3.35 - 4.6 (m, 4H, 4-H und P-OEt), 4.7 - 5.2 (mc, 1H, 5-H), 7.4 - 8.1 (m, 5H, Phenyl-H), 12.1 ppm (sb, 1H, P-OH).

| $C_{11}H_{14}NO_4P$ (255.2) | | | | |
|---|---|---|---|---|
| Analyse: | Ber.:<br>Gef.: | C 51.77<br>C 51.97 | H 5.53<br>H 5.54 | N 5.49<br>N 5.26 |

Beispiel 28: 3-(4-Methoxyphenyl)-2-isoxazolin-5-yl-phosphonsäuremonoethylester-Ammoniumsalz

Der Phosphonsäurediethylester des Beispiels 8 wird gemäß Beispiel 27 mit 1.1 Äquivalenten Natronlauge verseift, der gebildete Halbester in der Säureform als öliges Produkt isoliert (Ausbeute: 70 %) und - wie in Beispiel 35 ausführlich beschrieben - in das kristalline Ammoniumsalz mit einem Schmelzbereich von

141° bis 154° C umgewandelt (Ausbeute: 53 %).
$^1$H-NMR ($D_2O$):
δ = 1.25 (tb, J = 7 Hz, 3H, P-OEt), 3.1 - 4.25 (m, 7H, 4-H, P-OEt und OMe bei 3.72 ppm), 4.4 - 4.9 (m, ca. 5H, 5-H und $NH_4^+$), 6.75 und 7.4 ppm (AA'BB', 4H, Aryl-H).

| $C_{12}H_{19}N_2O_5P$ (302.3) | | | | |
|---|---|---|---|---|
| Analyse: | Ber.:<br>Gef.: | C 47.68<br>C 47.67 | H 6.34<br>H 6.14 | N 9.27<br>N 8.60 |

Die in Tabelle 1 auf Seite 39 als Beispiele 29 bis 32 zu sammengefaßten Isoxazolinphosphinsäuren der allgemeinen Formel

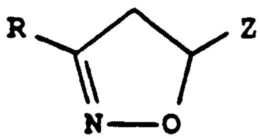

wurden ebenfalls nach Verfahrensweise c) aus den entsprechenden Methylestern der Beispiele 14, 15, 24 bzw. 16 analog Beispiel 27 durch Hydrolyse mit überschüssiger Natronlauge erhalten und durch Kristallisation als freie Säuren oder Überführung in die kristallinen Ammoniumsalze (in Ethanol mit ethanolischer Ammoniaklösung) gereinigt. Die Phosphinsäure des Beispiels 31 wurde darüber hinaus auch durch Esterspaltung mit Bromwasserstoff (HBr) in Eisessig analog Beispiel 34 gewonnen.

Beispiel 33: 3-tert.Butyl-2-isoxazolin-5-yl-phosphonsäure (nach Verfahrensweise c))

39.6 g (0.15 mol) der Verbindung aus Beispiel 26 werden in einer Mischung aus 100 ml 33 %iger HBr/Eisessig-Lösung und 50 ml Eisessig gelöst. Nach dreitägigem Stehen bei Raumtemperatur engt man das Reaktionsgemisch ein, destilliert mehrmals Methanol über und rührt den verbleibenden Rückstand mit tert.Butylmethylether aus, wobei 20.8 g analysenreines Produkt vom Schmelzpunkt 195° bis 197° C erhalten werden.
$^1$H-NMR (DMSO-$d_6$):
δ = 1.1 (s, 9H, tBu), 2.7 - 3.4 (m, 2H, 4-H), 4.1 - 4.6

Tabelle 1

| Beispiel | R | Z | Fp.: (°C) | $^1$H-NMR (DMSO-$d_6$), δ (ppm) = | Analyse |
|---|---|---|---|---|---|
| 29 | 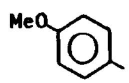 | 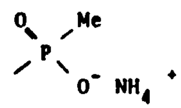 | 143-147 | (in Methanol-$d_4$)<br>1.25 (d, J = 14 Hz, 3H, P-Me)<br>3.1-3.9 (m, 5H, 4-H und OMe bei 3.7)<br>4.2-5.1 (m, ~ 5H, 5-H und $NH_4^{\oplus}$)<br>6.75 und 7.45 (AA', BB', 4H, Aryl-H) | $C_{11}H_{17}N_2O_4P$ (272.2)<br>Ber.: C 48.53 H 6.30 N 10.29<br>Gef.: C 48.31 H 5.88 N 9.80 |
| 30 | 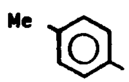 | 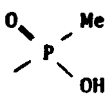 | 172-176 | 1.35 (d, J = 14 Hz, 3H, P-Me)<br>2.3 (s, 3H, Aryl-$CH_3$)<br>3.2-3.85 (m, 2H, 4-H)<br>4.35-4.9 (mc, 1H, 5-H),<br>7.1 und 7.45 (AA', BB', 4H, Aryl-H),<br>9.4 (sb, 1H, P-OH) | $C_{11}H_{14}NO_3P$ (239.2)<br>Ber.: C 55.23 H 5.90 N 5.86<br>Gef.: C 55.16 H 5.57 N 5.89 |
| 31 | Ph-$CH_2$- | 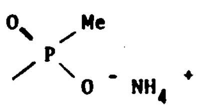 | 139 | in $D_2O$:<br>1.15 (d, J = 13 Hz, 3H, P-Me),<br>2.7-3.35 (m, 2H, 4-H),<br>3.6 (sb, 2H, Benzyl),<br>4.1-4.8 (mc, ~ 5H, 5-H und $NH_4^+$),<br>7.0-7.4 (m, 5H, Aryl-H). | $C_{11}H_{17}N_2O_3P$ (256.2)<br>Ber.: C 51.56 H 6.69 N 10.93<br>Gef.: C 51.55 H 6.81 N 10.89 |
| 32 | 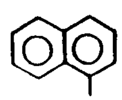 | 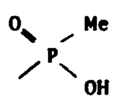 | 139-140 | 1.45 (d, J = 14 Hz, 3H, P-Me)<br>3.4-4.15 (m, 2H, 4-H)<br>4.5-5.05 (mc, 1H, 5-H)<br>7.3-8.15 (m, 6H, Aryl-H)<br>8.4-9.0 (m, 2H, Aryl-4-H und P-OH) | $C_{14}H_{14}NO_3P$ (275.25)<br>Ber.: C 61.09 H 5.13 N 5.09<br>Gef.: C 60.61 H 5.04 N 5.06 |

(mc, 1H, 5-H), 10.5 ppm (sb, 2H, P-OH).

| $C_7H_{14}NO_4P$ (207.2) | | | | |
|---|---|---|---|---|
| Analyse: | Ber.:<br>Gef.: | C 40.59<br>C 40.28 | H 6.81<br>H 6.95 | N 6.76<br>N 6.83 |

Beispiel 34: 3-Phenyl-2-isoxazolin-5-yl-phosphonsäure (nach Verfahrensweise c))

122 g des gemäß Beispiel 1 hergestellten rohen Diethylesters werden in 400 ml einer 2 N Lösung von HBr in Eisessig gelöst, 2 Tage bei Raumtemperatur belassen, dann 2 Stunden auf 40° C erwärmt, das Lösungsmittel unter vermindertem Druck entfernt und mehrmals Methanol überdestilliert. Umkristallisation des Rückstandes aus Dichlormethan liefert 60 g der analysenreinen Phosphonsäure vom Schmelzpunkt 183° bis 184° C.
$^1$H-NMR (DMSO-$d_6$):
δ = 3.1 - 3.9 (m, 2H, 4-H), 4.4 - 4.95 (mc, 1H, 5-H), 7.2 - 7.8 (m, 5H, Phenyl-H), 10.6 ppm (sb, 2H, P-OH).

| $C_9H_{10}NO_4P$ (227.2) | | | | |
|---|---|---|---|---|
| Analyse: | Ber.:<br>Gef.: | C 47.59<br>C 47.66 | H 4.44<br>H 4.44 | N 6.17<br>N 6.09 |

Die Beispiele 35 bis 38 betreffen verschiedene Salze der 3-Phenyl-2-isoxazolin-5-yl-phosphonsäure gemäß Beispiel 34.

Beispiel 35: Diammoniumsalz des Beispiels 34

10 g der Phosphonsäure aus Beispiel 34 werden in 150 ml Methanol gelöst, mit alkoholischer Ammoniaklösung alkalisch gestellt, das gebildete Salz durch Kühlen und gegebenenfalls Animpfen kristallin abgeschieden und die Fällung durch Zugabe von tert.Butylmethylether vervollständigt. Man erhält 10 g Diammoniumsalz mit einem Schmelzpunkt von 197° bis 202° C.

| $C_9H_{16}N_3O_4P$ (261.2) | | | | |
|---|---|---|---|---|
| Analyse: | Ber.:<br>Gef.: | C 41.38<br>C 41.70 | H 6.17<br>H 6.16 | N 16.09<br>N 15.65 |

Das Salz läßt sich auch bequem aus der Rohsäure des Beispiels 34 herstellen. Dazu wird das aus 0.15 mol Diethylester des Beispiels 1 gewonnene Hydrolysat in Wasser gelöst, zweimal mit einer Lösung von 80 ml Amberlite® LA-2 ($OH^-$-Form) in 240 ml Ethylacetat extrahiert, die organische Phase mit Wasser gewaschen, und die Säure durch dreimaliges Ausschütteln mit je 100 ml halbkonzentrierter wäßriger Ammoniaklösung als Diammoniumsalz reextrahiert. Man wäscht die wäßrige Phase mehrmals mit Ethylacetat, engt ein und gewinnt das reine Salz durch Ausrühren in Aceton oder Ethanol in kristalliner Form.
Falls erwünscht, kann die freie Phosphonsäure nach Suspendieren des Salzes in Methanol durch Zugabe von überschüssigem Amberlyst® 15 in der $H^+$-Form und Einengen der alkoholischen Lösung zurückgewonnen werden.

Die im allgemeinen gut kristallisierbaren Ammoniumsalze neigen bei thermischer Belastung zu merklicher Abspaltung von Ammoniak. Daher ist bei ihrer Trocknung, insbesondere unter vermindertem Druck, die Anwendung erhöhter Temperaturen zu vermeiden.

Beispiel 36: Dinatriumsalz des Beispiels 34

Man löst 4.54 g (20 mmol) der Phosphonsäure des Beispiels 34 in 40 ml Methanol und versetzt mit 1.6 g (40 mmol) Natriumhydroxid in ca. 40 ml Methanol, wobei sich ein gallertartiger Niederschlag bildet. Es wird kurz aufgekocht, drei Tage bei Raumtemperatur stehen gelassen, abgesaugt und getrocknet. Man erhält 3.85 g des Dinatriumsalzes mit einem Schmelzpunkt von >300° C. Aus der Mutterlauge läßt sich weiteres Produkt isolieren.

| $C_9H_8NNa_2O_4P$ (271.1) | | | | | |
|---|---|---|---|---|---|
| Analyse: | Ber.:<br>Gef.: | C 39.87<br>C 39.77 | H 2.97<br>H 2.89 | N 5.17<br>N 5.12 | Na 16.96<br>Na 17.20 |

Beispiel 37: Monokaliumsalz des Beispiels 34

4.54 g (20 mmol) 3-Phenyl-2-isoxazolin-5-yl-phosphonsäure werden in 120 ml Methanol gelöst und 2.76 g (20 mmol) Kaliumcarbonat zugesetzt. Nach kurzzeitigem Aufkochen wird die Lösung abgekühlt, durch Einbringen von Trockeneis mit $CO_2$ gesättigt, über Nacht im Kühlschrank belassen und der gebildete Niederschlag abgesaugt und getrocknet. Man erhält 4.2 g Monokaliumsalz vom Schmelzpunkt >300° C.

| $C_9H_9KNO_4P$ (265.3) | | | | | |
|---|---|---|---|---|---|
| Analyse: | Ber.:<br>Gef.: | C 40.75<br>C 40.51 | H 3.42<br>H 3.30 | N 5.28<br>N 5.48 | K 14.74<br>K 15.20 |

Beispiel 38: Di-Lysiniumsalz des Beispiels 34

Zu einer Lösung von 5.7 g (25 mmol) der Phosphonsäure aus Beispiel 34 in 150 ml Methanol werden 7.3 g (50 mmol) L-Lysin, gelöst in 100 ml Methanol, in der Hitze zugegeben. Es wird kurz aufgekocht, der Niederschlag nach zweistündigem Stehen bei Raumtemperatur abgesaugt, nacheinander mit Methanol und tert.Butylmethylether gewaschen und getrocknet, wobei man 11.1 g Salz vom Schmelzpunkt 217° bis 218° C erhält.
$^1$H-NMR ($D_2O$):
$\delta$ = 1.2 - 2.2 (m, 12H, Lysin-$CH_2$), 2.75 - 3.85 (m, 8H, 4-H, Lysin-CHN und -$CH_2N$), 4.3 - 4.95 (ca. 13H, 5-H und HDO), 7.3 - 7.85 ppm (m, 5H, Aryl-H).

| $C_{21}H_{38}N_5O_8P$ (519.5) | | | | |
|---|---|---|---|---|
| Analyse: | Ber.:<br>Gef.: | C 48.55<br>C 47.92 | H 7.37<br>H 7.24 | N 13.48<br>N 12.94 |

Die in Tabelle 2 auf den Seiten $4^4$ bis $4^6$ als Beispiele 39 bis 54 zusammengefaßten Isoxazolin-bzw. Isoxazolphosphonsäuren der allgemeinen Formel

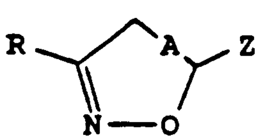

wurden ebenfalls nach Verfahrensweise c) aus den entsprechenden Methyl- oder Ethylestern durch Umsetzung mit HBr in Eisessig gemäß Beispiel 34 hergestellt und durch Chromatographie, Kristallisation als Säuren oder Überführung in die Diammoniumsalze analog Beispiel 35 gereinigt. Das Hydrobromid des Beispiels 42 wurde aus Methanol/tert.Butylmethylether umkristallisiert.

Beispiel 55: 3-Phenyl-2-isoxazolin-5-yl-(P-ethoxy)-phosphosäure-pyrrolidid (nach Verfahrensweise d))

8.5 g (0.03 mol) des Diethylesters aus Beispiel 1 werden in 150 ml Ethanol gelöst, 24.5 ml Pyrrolidin zugesetzt und 10 Stunden unter Rückfluß erhitzt. Man engt ein, reinigt durch Chromatographie an Kieselgel (Laufmittel: Dichlormethan/Methanol 5 : 1) und erhält 8.5 g des Halbesterhalbamids in öliger Form.

Tabelle 2

| Beispiel | R | Z | A | Fp.: (°C) | $^1$H-NMR (DMSO-$d_6$), δ (ppm)= | Analyse |
|---|---|---|---|---|---|---|
| 39 | $C_6H_5$ | $-CH_2-\overset{O}{\overset{\|}{P}}(OH)_2$ | $CH_2-CH$ | 209-218 | 1.7-2.35 (m, 2H, $CH_2$-P), 3.0-3.5 (m, 2H, 4-H), 4.45-5.05 (mc, 1H, 5-H), 7.1-7.6 (m, 5H, Phenyl-H), 8.1 (sb, 2H, $P(OH)_2$). | $C_{10}H_{12}NO_4P$ x 1/20 $H_2O$ (242.1)<br>Ber.: C 49.63 H 5.04 N 5.79<br>Gef.: $H_2O$ 0.36 C 48.93 H 4.96 N 5.82 |
| 40 | 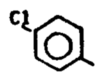 | $-\overset{O}{\overset{\|}{P}}(OH)_2$ | $CH_2-CH$ | 207 | 3.0-3.9 (m, 2H, 4-H), 4.4-4.85 (mc, 1H, 5-H), 7.35 und 7.6 (AA' BB', 4H Aryl-H), 10.2 (sb, 2H, $P(OH)_2$). | $C_9H_9NO_4ClP$ (261.6)<br>Ber.: C 41.32 H 3.47 N 5.36<br>Gef.: C 40.64 H 3.30 N 4.92 |
| 41 | 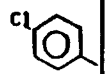 | $-CH_2-\overset{O}{\overset{\|}{P}}(OH)_2$ | $CH_2-CH$ | 203 | 1.75-2.3 (m, 2H, $CH_2$-P), 2.9-3.6 (m, 2H, 4-H), 4.5-5.1 (mc, 1H, 5-H) 7.36 und 7.6 (AA' BB', 4H, Aryl-H), 9,4 (sb, 2H, $P(OH)_2$). | $C_{10}H_{11}NO_4ClP$ (275.6)<br>Ber.: C 43.58 H 4.02 N 5.08<br>Gef.: C 43.91 H 3.87 N 5.12 |
| 42 | 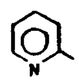<br>x HBr | $-\overset{O}{\overset{\|}{P}}(OH)_2$ | $CH_2-CH$ | 210-225 (Zers.) | 3.15-3.95 (m, 2H, 4-H), 4.5-5.0 (mc, 1H, 5-H) 7.35-8.2 und 8.4-8.7 (m, 4H, Pyridyl-H), 10.8 (sb, 3H, $P(OH)_2$ und $\overset{\oplus}{NH}$) | $C_8H_{10}N_2O_4BrP$ (309.6)<br>Ber.: C 31.09 H 3.26 N 9.06<br>Gef.: C 30.75 H 3.16 N 9.01 |
| 43 | 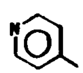 | $-\overset{O}{\overset{\|}{P}}(ONH_4)_2$ | $CH_2-CH$ | 192-194 | In $D_2O$: 3.1-3.9 (m, 2H, 4-H), 4.5-5.1 (m, ~9H, 2 $NH_4^+$ u. 5-H) 7.47 und 8.45 (AA' BB', 4H, Pyridyl-H) | $C_8H_{15}N_4O_4P$ (262.2)<br>Ber.: C 36.65 H 5.77 N 21.37<br>Gef.: C 37.05 H 5.54 N 20.92 |
| 44 | 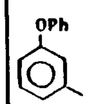 | $-\overset{O}{\overset{\|}{P}}(OH)_2$ | $CH_2-CH$ | 186-193 | 3.0-3.85 (m, 2H, 4-H) 4.35-4.85 (mc, 1H, 5-H), 6.7-7.5 (m, 9H, Aryl-H), 8.8 (sb, 2H, $P(OH)_2$). | $C_{15}H_{14}NO_5P$ (319.3)<br>Ber.: C 56.43 H 4.42 N 4.39<br>Gef.: C 55.94 H 4.31 N 4.48 |

Tabelle 2 (Fortsetzung)

| Beispiel | R | Z | A | Fp.: ($^0C$) | $^1H$-NMR (DMSO-$d_6$), δ (ppm) = | Analyse |
|---|---|---|---|---|---|---|
| 45 | 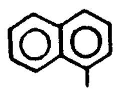 | $-\overset{\overset{O}{\|}}{P}(OH)_2$ | $CH_2$-CH | 170-209 (Zers.) | 3.3-4.1 (m, 2H, 4-H)<br>4.45-4.95 (mc, 1H, 5-H)<br>7.25-8.05 (m, 6H, Aromaten-H),<br>8.5-8.9 (m, 1H, Aromaten-H),<br>10.3 (sb, 2H, $P(OH)_2$). | $C_{13}H_{12}NO_4P$ (277.2)<br>Ber.: C 56.33 H 4.36 N 5.05<br>Gef.: C 55.55 H 4.35 N 5.64 |
| 46 | $C_6H_5$ | $-\overset{\overset{O}{\|}}{P}(OH)_2$ | CH=C | 199-206 | 7.2-8.05 (m, 6H, Aryl-H und 4-H als d, J = 1,8 Hz bei 7.3),<br>9.8 (sb, 2H, $P(OH)_2$). | $C_9H_8NO_4P$ (225.1)<br>Ber.: C 48.01 H 3.58 N 6.22<br>Gef.: C 47.77 H 3.50 N 6.29 |
| 47 | $CH_3-CH_2-CH_2$ | $-\overset{\overset{O}{\|}}{P}(ONH_4)_2$ | $CH_2$-CH | 140-143 | in $D_2O$:<br>0.9 (t, J = 6 Hz, $CH_3$)<br>1.2-1.8 (m, 2H, $CH_2$),<br>2.1-2.5 (m, 2H, $CH_2$),<br>2.75-3.4 (m 2H, 4-H),<br>4.0-4.5 (mc, 1H, 5-H),<br>4.7 (2 $NH_4^+$) | $C_6H_{18}N_3O_4P$ (227.2)<br>Ber.: C 31.72 H 7.98 N 18.49<br>Gef.: C 31.73 H 7.90 N 17.82 |
| 48 | 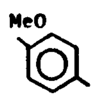 | $-\overset{\overset{O}{\|}}{P}(OH)_2$ | $CH_2$-CH | 204-206 | 3.0-3.85 (m, 5H, 4-H und OMe bei 3.68),<br>4.25-4.75 (mc, 1H, 5-H),<br>6.77 und 7.4 (AA' BB', 4H, Aryl-H)<br>9.6 (sb, 2H, $P(OH)_2$). | $C_{10}H_{12}NO_5P$ (257.2)<br>Ber.: C 46.70 H 4.70 N 5.45<br>Gef.: C 46.28 H 4.49 N 5.44 |
| 49 | $C_6H_5-CH_2-$ | $-\overset{\overset{O}{\|}}{P}(OH)_2$ | $CH_2$-CH | 155-159 | 2.35-3.35 (m, 2H, 4-H),<br>3.57 (sb, 2H, Benzyl-H),<br>4.1-4.6 (mc, 1H, 5-H),<br>6.9-7.35 (m, 5H, Aryl-H),<br>9.8 (sb, 2H, $P(OH)_2$). | $C_{10}H_{12}NO_4P$ (241.2)<br>Ber.: C 49.80 H 5.02 N 5.81<br>Gef.: C 49.81 H 4.88 N 5.80 |

Tabelle 2 (Fortsetzung)

| Beispiel | R | Z | A | Fp.: (°C) | $^1$H-NMR (DMSO-$d_6$), $\delta$(ppm) = | Analyse |
|---|---|---|---|---|---|---|
| 50 | 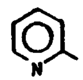 | $-\overset{O}{\overset{\|}{P}}(ONH_4)_2$ | CH=C | 222-224 | in $D_2O$:<br>4.6 (sb, ~8H, 2 $NH_4^+$),<br>6.88 (d, J = 1.5 Hz, 1H, 4-H)<br>7.1-7.85 (m, 3H, Pyridyl-H),<br>8.2-8.5 (mc, 1H, Pyridyl-6-H). | $C_8H_{13}N_4O_4P$ (260.2)<br>Ber.: C 36.92 H 5.04 N 21.52<br>Gef.: C 36.76 H 5.01 N 21.00 |
| 51 | 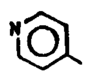 | $-\overset{O}{\overset{\|}{P}}(ONH_4)_2$ | CH=C | 238-240 | in $CF_3CO_2H$:<br>7.65 (d, J = 2 Hz, 1H, 4-H),<br>8.55 und 8.95 (AA' BB',<br>4H, Pyridyl-H). | $C_8H_{13}N_4O_4P$ (260.2)<br>Ber.: C 36.92 H 5.04 N 21.52<br>Gef.: C 36.47 H 5.19 N 20.80 |
| 52 | 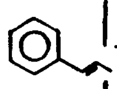 | $-\overset{O}{\overset{\|}{P}}(OH)_2$ | $CH_2$-CH | 191-193 | 2.95-3.7 (m, 2H, 4-H),<br>4.3-4.8 (mc, 1H, 5-H),<br>6.9 (sb, 2H, Ph-CH=CH),<br>7.0-7.6 (m, 5H, Aryl-H),<br>9.2 (sb, 2H, $P(OH)_2$). | $C_{11}H_{12}NO_4P$ (253.2)<br>Ber.: C 52.18 H 4.78 N 5.53<br>Gef.: C 51.96 H 4.62 N 5.54 |
| 53 | 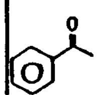 | $-\overset{O}{\overset{\|}{P}}(ONH_4)_2$ | $CH_2$-CH | 138-168 (Zers.) | in $D_2O$:<br>3.1-3.95 (m, 2H, 4-H)<br>4.45-5.0 (m, ~9H, 5-H und<br>2$NH_4^+$)<br>7.25-8.1 (m, 5H, Aryl-H). | $C_{10}H_{16}N_3O_5P$ (289.2)<br>Ber.: C 41.53 H 5.58 N 14.53<br>Gef.: C 41.05 H 5.37 N 13.63 |
| 54 | 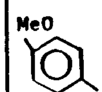 | $-\overset{O}{\overset{\|}{P}}(ONH_4)_2$ | CH=C | 187-190 | in $D_2O$:<br>3.7 (s, 3H, OMe)<br>6.6-6.95 (m, 3H, 4-H und<br>2 Ar-H)<br>7.55 (2H, Ar-H) | $C_{10}H_{16}N_3O_5P$ (289.2)<br>Ber.: C 41.53 H 5.58 N 14.53<br>Gef.: C 40.78 H 5.70 N 14.29 |

$^1$H-NMR ($CDCl_3$):
$\delta$ = 1.25 (tb, J = 7 Hz, 3H, P-OEt), 1.7 - 2.2 (m, 4H, Pyrrolidin-$CH_2$), 3.05 - 4.35 (m, 8H, Pyrrolidin-N-$CH_2$,4-H und P-OEt), 4.55 - 5.05 (mc, 1H, 5-H), 7.4 - 7.95 ppm (m, 5H, Aryl-H).

Beispiel 56: N-[(3-Phenyl-2-isoxazolin-5-yl)-(P-ethoxy)-phosphono]-glycinbenzylester (nach Verfahrensweise e))

14.6 g (0.057 mol) des Halbesters aus Beispiel 27 werden in 150 ml trockenem Toluol suspendiert mit 11.7 g (0.057 mol) Phosphorpentachlorid versetzt und 1 Stunde unter Rückfluß erhitzt. Nach Einengen unter vermindertem Druck verbleibt ein viskoses Öl, das, in 150 ml Tetrahydrofuran gelöst, unter Eiskühlung mit 23.5 ml (0.168 mol) Triethylamin und 18.9 g (0.056 mol) Glycinbenzylester als Toluolsulfonsäuresalz versetzt und 14 Stunden bei Raumtemperatur gerührt wird. Man verdünnt mit Ethylacetat und wäscht nacheinander mit wäßriger Natriumhydrogencarbonat-, Kaliumhydrogensulfat-Lösung und mehrmals mit Wasser. Das nach dem Trocknen und Einengen anfallende Öl wird aus Diethylester kristallisiert und liefert 7.8 g reines Produkt vom Schmelzpunkt 91° bis 103° C.
$^1$H-NMR ($CDCl_3$):
$\delta$ = 1.05 - 1.5 (2t, 3H, P-OEt), 3.2 - 4.25 (m, 7H, P-OEt, 4-H, Gly-$CH_2$ und NH), 4.55 - 5.1 (m, 3H, 5-H und Benzyl-$CH_2$), 7.05 - 7.6 ppm (m, 10H, Aryl-H).

| $C_{20}H_{23}N_2O_5P$ (402.4) | | | | |
|---|---|---|---|---|
| Analyse: | Ber.:<br>Gef.: | C 59.70<br>C 59.85 | H 5.76<br>H 5.79 | N 6.96<br>N 6.93 |

Beispiel 57/58: N-[(3-Phenyl-2-isoxazolin-5-yl)-(P-methyl)-phosphonyl]-glycinbenzylester (nach Verfahrensweise e))

1.1 g (5 mmol) 3-Phenyl-2-isoxazolin-5-yl-(P-methyl)-phosphinsäuren werden in 20 ml Pyridin gelöst, unter Eiskühlung mit 1.5 g (7 mmol) Mesitylsulfonylchlorid und 0.5 g (7 mmol) Tetrazol versetzt und 30 Minuten bei Raumtemperatur nachgerührt. Man fügt 1.7 g (5 mmol) Glycinbenzylester-Toluolsulfonsäuresalz hinzu und rührt weiterhin bei Raumtemperatur, wobei der Reaktionsverlauf dünnschichtchromatographisch verfolgt wird. Nach Beendigung der Reaktion wird mit Wasser und halbkonzentrierter Kaliumhydrogensulfat-Lösung bis pH 2 versetzt, dreimal mit Dichlormethan extrahiert, die organische Phase mit Wasser gewaschen, getrocknet und eingeengt. Durch Chromatographie an Kieselgel mit Dichlormethan/Methanol-Gemischen als Fließmittel erhält man das reine Produkt mit einem Schmelzpunkt von 132° bis 135°C.
$^1$H-NMR ($CDCl_3$):
$\delta$ = 1.45 - 1.63 (jew. d, J = 15 Hz, 3H, P-Me), 3.2 - 3.95 (m, 5H, 4-H, Gly-$CH_2$ und NH), 4.5 - 5.1 (m, 3H, 5-H bei 4.8 ppm und Bzl-$CH_2$ bei 5.0 ppm), 7.05 - 7.65 ppm (m, 10H, Aryl-H).

| $C_{19}H_{21}N_2O_4P$ (372.4) | | | | |
|---|---|---|---|---|
| Analyse: | Ber.:<br>Gef.: | C 61.29<br>C 60.64 | H 5.69<br>H 5.81 | N 7.52<br>N 7.16 |

Dieselbe Verbindung wurde auf alternativem Wege als Beispiel 58 aus 7.5 g (33 mmol) 3-Phenyl-2-isoxazolin-5-yl-(P-methyl)-phosphinsäure, 6.9 g (33 mmol) Phosphorpentachlorid, 11.1 g (33 mmol) Glycinbenzylester-Toluolsulfonsäuresalz und 13.9 ml (0.1 mol) Triethylamin analog Beispiel 56 hergestellt und durch Umkristallisation aus tert.Butylmethylether gereinigt. Ihre Identität mit dem vorbeschriebenen Produkt ließ sich analytisch bestätigen.

Beispiel 59: 3-Phenyl-2-isoxazolin-5-yl-phosphonsäuretetramethyldiamid (nach Verfahrensweise a))

Die Herstellung erfolgt analog Beispiel 1 aus 12.1 g (0.1 mol) Benzaldoxim, 14.7 g (0.11 mol N-Chlorsuccinimid, 0.5 ml Pyridin, 17.8 g (0.11 mol) Vinylphosphonsäuretetramethyldiamid und 13.9 ml (0.1 mol) Triethylamin. Man erhält 24.3 g öliges Rohprodukt, das durch Destillation unter vermindertem Druck gereinigt wird (Siedepunkt: 145° bis 150° C bei 0.133 mbar).
$^1$H-NMR ($CDCl_3$):
$\delta$ = 2.7 (2d, J = 9 Hz, 12H, $NMe_2$), 3.2 - 3.85 (m, 2H, 4-H), 4.75 - 5.25 (mc, 1H, 5-H), 7.1 - 7.65 ppm (m, 5H, Aryl-H).

| $C_{13}H_{20}N_3O_2P$ (281.3) | | | | |
|---|---|---|---|---|
| Analyse: | Ber.:<br>Gef.: | C 55.51<br>C 55.99 | H 7.17<br>H 7.29 | N 14.94<br>N 14.64 |

Beispiel 60: (+)- und (-)-3-Phenyl-2-isoxazolin-5-yl-phosphonsäuredimethylester (nach Verfahrensweise g))

1.5 g des racemischen Dimethylesters aus Beispiel 5 werden durch Chromatographie an einer 95 cm langen Triacetylcellulose-Säule mit einem Durchmesser von 5 cm mittels Ethanol/Hexan-Gemischen als Fließmittel in die beiden Enantiomeren aufgetrennt, wobei anfallende Mischfraktionen der Rechromatographie unterworfen werden.

a) (+)-Isomer: Öl, $[\alpha]_D^{20}$ = +214.2° (c = 2.0 in Methanol), Enantiomerenreinheit: >99 % (lt. HPLC-Analyse)

b) (-)-Isomer: Öl, $[\alpha]_D^{20}$ = -198.5° (c = 2.0 in Methanol), Enantiomerenreinheit: >95 % (lt. HPLC-Analyse)

Durch saure Esterpaltung der beiden Antipoden analog Beispiel 34 und Umkristallisation aus Aceton werden die entsprechenden enantiomerenreinen Phosphonsäuren erhalten, die in allen Eigenschaften mit den in den folgenden Beispielen 61 und 62 beschriebenen Verbindungen übereinstimmen.

Beispiel 61: (+)=3-Phenyl-2-isoxzolin-5-yl-phosphonsäure (nach Verfahrensweise g))

22.7 g (0.1 mol) der racemischen Phosphonsäure aus Beispiel 34 werden in 150 ml heißem Methanol gelöst und zu einer 50° C warmen Lösung von 59 g (0.2 mol) (-)-Cinchonidin in 500 ml Isopropanol und 50 ml Methanol gegeben. Bei langsamem Abkühlen bis auf 0° C kristallisieren 21 g Salz aus. Durch Zugabe von Aceton zur Mutterlauge werden weitere 3 g des Salzes gewonnen. Man kristallisiert den Feststoff aus Methanol/Aceton um und setzt aus dem gewonnenen Salz mit einer Isomerenreinheit von >98 % die Phosphonsäure mit Hilfe eines sauren Ionenaustauschers, z. B. Amberlyst® 15, frei, die nach weiterer Kristallisation aus Aceton laut HPLC-Analyse eine Enantiomerenreinheit von >99% aufweist.
Schmelzpunkt: 220° C (Zers.)
$[\alpha]_D^{20}$ = +204.3° (c = 2.0 in Methanol)

Beispiel 62: (-)-3-Phenyl-2-isoxazolin-5-yl-phosphonsäure (nach Verfahrensweise g))

45.3 g (0.3 mol) (-)-Norephedrin werden in 500 ml Methanol in der Hitze gelöst und bei 50° C eine Lösung von 34.1 g (0.15 mol) racemischer Phosphonsäure aus Beispiel 34 in 100 ml Methanol zugesetzt. Man läßt unter langsamem Abkühlen bis auf 0° C kristallisieren. Die so erhaltenen 22 g des Norephedriniumsalzes werden aus Methanol umkristallisiert. Die Phosphonsäure wird, wie in Beispiel 61 beschrieben, mit einem sauren Ionenaustauscher freigesetzt und kristallisiert. Die Enantiomerenreinheit beträgt gemäß HPLC-Analyse >99 %.
Schmelzpunkt: 218° C (Zers.)
$[\alpha]_D^{20}$ = -204.7° (c = 2.0 in Methanol)

Beispiel 63: 3-Phenyl-2-isoxazolin-5-yl-(P-methyl)-phosphinsäure (nach Verfahrensweise c))

Analog Beispiel 27 werden aus 12 g (0.05 mol) des in Beispiel 13 beschriebenen Methylesters 10.3 g der Phosphinsäure vom Schmelzpunkt 162° bis 167° erhalten.
$^1$H-NMR (DMSO-$d_6$):
δ = 1.45 (d, J = 15 Hz, 3H, P-Me), 3.3 - 4.05 (m, 2H, 4-H), 4.6 - 5.15 (mc, 1H, 5-H), 7.5 - 8.1 (m, 5H, Aryl-H), 10.0 ppm (sb, 1H, P-OH).

| $C_{10}H_{12}NO_3P$ (225.2) | | | | |
|---|---|---|---|---|
| Analyse: | Ber.:<br>Gef.: | C 53.34<br>C 53.39 | H 5.37<br>H 5.43 | N 6.22<br>N 6.20 |

Beispiel 64: 3-tert.Butyl-2-isoxazolin-5-yl-(P-methyl)-phosphinsäure (nach Verfahrensweise c))

10 g des Methylesters aus Beispiel 25 liefern bei Umsetzung gemäß Beispiel 34 und Umkristallisation der Rohsäure aus Aceton/tert.Butylmethylether 4.7 g Reinprodukt vom Schmelzpunkt 130° C.
$^1$H-NMR (DMSO-$d_6$):
δ = 1.13 (s, 9H, tBu), 1.38 (d, J = 13 Hz, 3H, P-Me), 2.7-3.4 (m, 2H, 4-H), 4.1 - 4.6 (mc, 1H, 5-H), 9.75 ppm (sb, 1H, P-OH).

| $C_8H_{16}NO_3P$ x 0.2 $H_2O$ (208.8) | | | | | |
|---|---|---|---|---|---|
| Analyse: | Ber.:<br>Gef.: | $H_2O$ 1.72<br>$H_2O$ 1.7 | C 46.02<br>C 46.02 | H 7.92<br>H 7.63 | N 6.71<br>N 6.91 |

Beispiel 65: 3-(4-Fluorphenyl)-2-isoxazolin-5-yl-phosphonsäuredimethylester

Analog Beispiel 1 erhält man aus 19.2 g (0.138 mol) 4-Fluorbenzaldoxim, 20.3 g (0.152 mol) N-Chlorsuccinimid, 18.8 g (0.138 mol) Vinylphosphonsäuredimethylester und 21.2 ml (0.152 mol) Triethylamin 23.6 g Produkt nach Umkristallisation aus tert.Butylmethylether (Schmelzpunkt: 94° C).
$^1$H-NMR (DMSO-$d_6$):
δ = 3.2 - 4.1 (m, 8H, 4-H sowie $P(OMe)_2$ als d, J = 11 Hz, bei 3.7 ppm), 4.8 - 5.25 (mc, 1H, 5-H), 7.0 - 7.9 ppm (m, 4H, Aryl-H).

| $C_{11}H_{13}FNO_4P$ (273.2) | | | | |
|---|---|---|---|---|
| Analyse: | Ber.:<br>Gef.: | C 48.36<br>C 48.24 | H 4.80<br>H 4.64 | N 5.13<br>N 5.31 |

Beispiel 66: 3-(4-Methoxycarbonyl-phenyl)-2-isoxazolin-5-yl-phosphonsäuredimethylester

Die Umsetzung von 80.6 g (0.45 mol) 4-Hydroxyiminobenzoesäuremethylester, 67.0 g (0.5 mol) N-Chlorsuccinimid, 61.25 g (0.45 mol) Vinylphosphonsäuredimethylester und 78.45 ml (0.56 mol) Triethylamin gemäß Beispiel 1 liefert nach Umkristallisation aus tert.Butylmethylether 115.2 g Reinprodukt vom Schmelzpunkt 93° C.
$^1$H-NMR ($CDCl_3$):
δ = 3.15 - 4.1 (m, 11H, 4-H, $P(OMe)_2$ bei 3.85 ppm und $CO_2Me$ bei 3.95 ppm), 4.7 - 5.2 (mc, 1H, 5-H), 7.75 und 8.1 ppm (AA'BB', 4H, Aryl-H).

| $C_{13}H_{16}NO_6P$ (313.2) | | | | |
|---|---|---|---|---|
| Analyse: | Ber.:<br>Gef.: | C 49.85<br>C 49.81 | H 5.15<br>H 5.02 | N 4.47<br>N 4.52 |

Beispiel 67: 3-(4-Fluorphenyl)-2-isoxazolin-5-yl-phosphonsäure (nach Verfahrensweise c))

14 g (51 mmol) das Dimethylesters aus Beispiel 65 werden, wie in Beispiel 34 beschrieben, in die Phosphonsäure überführt und diese aus Dichlormethan kristallisiert. Man gewinnt 11 g Produkt vom Schmelzpunkt 206° C.
$^1$H-NMR(DMSO-$d_6$):
δ = 3.0 - 3.85 (m, 2H, 4-H), 4.3 - 4.8 (mc, 1H,5-H), 6.8-7.6 (m, 4H, Aryl-H), 10.7 ppm (sb, 2H, $P(OH)_2$).

| $C_9H_9FNO_4P$ (245.2) | | | | |
|---|---|---|---|---|
| Analyse: | Ber.:<br>Gef.: | C 44.10<br>C 43.69 | H 3.70<br>H 3.53 | N 5.71<br>N 5.79 |

Beispiel 68: 3-(4-Nitrophenyl)-isoxazolin-5-yl-phosphonsäuredimethylester (nach Verfahrensweise a))

Die Herstellung erfolgt analog Beispiel 1 aus 19.6 g (0.118 mol) 4-Nitrobenzaldoxim, 17.4 g (0.13 mol) N-Chlorsuccinimid, 16.1 g (0.118 mol) Vinylphosphonsäuredimethylester und 18.7 ml (0.13 mol) Triethylamin. Kristallisation des Rohproduktes aus tert.Butylmethylether liefert analysenreine Verbindung vom Schmelzpunkt 156° bis 158° C.
$C_{11}H_{13}N_2O_6P$ (300.3)
$^1$H-NMR (DMSO-$d_6$):
$\delta$ = 3.1 - 4.05 (m, 8H, 4-H und $P(OMe)_2$ bei 3.67 ppm), 4.8 - 5.3 (mc, 1H, 5-H), 7.83 und 8.15 ppm (AA'BB', 4H, Aryl-H).

Beispiel 69: 3-(4-Nitrophenyl)-2-isoxazolin-5-yl-phosphonsäure (nach Verfahrensweise c))

7.0 g des Esters aus Beispiel 68 werden gemäß Beispiel 34 zur Phosphonsäure gespalten und aus Dichlormethan kristallisiert, wobei 5.2 g Reinprodukt vom Schmelzpunkt 194° bis 197° C (Zers.) erhalten werden.
$^1$H-NMR (DMSO-$d_6$):
$\delta$ = 2.8 - 3.85 (m, 2H, 4-H), 4.35 - 4.85 (mc, 1H, 5-H), 7.6 und 7.95 (AA'BB', 4H, Aryl-H), 10.5 ppm (sb, 2H, $P(OH)_2$).

| $C_9H_9N_2O_6P$ (272.2) | | | | |
|---|---|---|---|---|
| Analyse: | Ber.:<br>Gef.: | C 39.72<br>C 39.55 | H 3.33<br>H 3.02 | N 10.29<br>N 10.19 |

Beispiel 70: 3-(4-Dimethylamino-phenyl)-2-isoxazolin-5-yl-phosphonsäuredimethylester (nach Verfahrensweise a))

Analog Beispiel 1 werden 40 g (0.24 mol) 4-Dimethylaminobenzaldoxim, 36.17 g (0.27 mol) N-Chlorsuccinimid, 32.7 g (0.24 mol) Vinylphosphonsäuredimethylester und 52.3 ml (0.376 mol) Triethylamin umgesetzt. Das ölig anfallende Rohprodukt wird durch Chromatographie an Kieselgel und Umkristallisation aus tert.Butylmethylether gereinigt. Man erhält 33.4 g des Isoxazolins vom Schmelzpunkt 123° C (Zers.).
$C_{13}H_{19}N_2O_4P$ (298.3)
$^1$H-NMR (DMSO-$d_6$):
$\delta$ = 2.8 - 3.85 (m, 14H, 4-H, $NMe_2$ als s bei 2.85 ppm und $P(OMe)_2$ als d, J = 10 Hz, bei 3.6 ppm), 4.5 - 5.05 (mc, 1H, 5-H), 6.5 und 7.3 ppm (AA'BB', 4H, Aryl-H).

Beispiel 71: 3-(4-Dimethylamino-phenyl)-2-isoxazolin-5-yl-phosphonsäure-hydrobromid (nach Verfahrensweise c))

10 g (33. 5 mmol) des Esters aus Beispiel 70 werden entsprechend Beispiel 34 zur Phosphonsäure gespalten und diese in Methanol in 18.7 g kristallines Hydrobromid vom Schmelzpunkt 214° C (Zers.) überführt. Das Produkt gibt beim scharfen Trocknen unter vermindertem Druck ca.20 % Bromwasserstoff ab.
$^1$H-NMR (DMSO-$d_6$):
$\delta$ = 2.8 - 3.8 (m, 8H, 4-H und $NMe_2$ als sb bei 3.05 ppm), 4.4 - 4.9 (mc, 1H, 5-H), 7.15 - 7.65 (AA'BB', 4H, Aryl-H), 11.0 ppm (sb, ~ 3H, acide H).

| $C_{11}H_{15}N_2O_4P$ x 0.8 HBr (334.9) | | | | | |
|---|---|---|---|---|---|
| Analyse: | Ber.:<br>Gef.: | C 39.44<br>C 39.34 | H 4.76<br>H 4.64 | N 8.36<br>N 8.37 | Br 19.08<br>Br 18.53 |

Beispiel 72: 3-(2-Hydroxyphenyl)-2-isoxazolin-5-yl-phosphonsäuredimethylester (nach Verfahrensweise a))

41.1 g (0.3 mol) Salicylaldoxim werden in Dichlormethan mit 40.1 g (0.3 mol) N-Chlorsuccinimid und 1.5 ml Pyridin 3 Stunden unter Rückfluß erhitzt. Nach Zugabe von Eiswasser wird ausgeschüttelt und das Hydroxamoylchlorid aus Dichlormethan/Petrolether umkristallisiert. Davon werden 25.5 g (0.15 mol) mit 20.4 g (0.15 mol) Vinylphosphonsäuredimethylester und 22.9 ml (0.165 mol) Triethylamin in Dichlormethan analog Beispiel 5 zu 25.8 g öligem Produkt umgesetzt.
$C_{11}H_{14}NO_5P$ (271.3)
$^1$H-NMR ($CDCl_3$):
$\delta$ = 3.35 - 4.05 (m, 8H, 4-H und $P(OMe)_2$ bei 3.85 ppm), 4.6 - 5.1 (mc, 1H, 5-H), 6.7 - 7.55 (m, 4H, Aryl-H), 9.5 ppm (sb, 1H, OH).

Beispiel 73: 3-(2-Hydroxyphenyl)-2-isoxazolin-5-yl-phosphonsäure (nach Verfahrensweise c))

20 g des Dimethylesters aus Beispiel 72 werden gemäß Beispiel 34 in die Phosphonsäure überführt, die man aus Dichlormethan umkristallisiert (9.5 g, Schmelzpunkt 111° bis 116° C (Zers.)).
$^1$H-NMR (DMSO-$d_6$):
$\delta$ = 3.1 - 3.95 (m, 2H, 4-H), 4.35 - 4.9 (mc, 1H, 5-H), 6.7 - 8.5 ppm (m, ~7H, Aryl-H und acide H).

| $C_9H_{10}NO_5P$ (243.2) | | | | |
|---|---|---|---|---|
| Analyse: | Ber.:<br>Gef.: | C 44.46<br>C 44.14 | H 4.15<br>H 3.98 | N 5.76<br>N 5.65 |

Beispiel 74: 3-(2-Thienyl)-2-isoxazolin-5-yl-phosphonsäuredimethylester (nach Verfahrensweise a))

12.03 g (0.094 mol) Thienyl-2-aldoxim werden in Dichlormethan suspendiert und 11.23 g (0.103 mol) tert.Butylhypochlorit, gelöst in Dichlormethan, zugetropft. Unter exothermer Reaktion erfolgt die Umsetzung zum Hydroxamoylchlorid. Nach 3 Stunden werden 14.1 g (0.103 mol) Vinylphosphonsäuredimethylester und dann innerhalb von 15 Stunden 15.7 ml (0.113 mol) Triethylamin, gelöst in Dichlormethan, tropfenweise zugegeben. Aufarbeitung analog Beispiel 1 liefert 19 g öliges Rohprodukt, das durch Chromatographie an Kieselgel gereinigt wird. Man erhält 16.8 g reinen Esters.
$C_9H_{12}NO_4PS$ (261.3)
$^1$H-NMR ($CDCl_3$):
$\delta$ = 3.3 - 4.0 (m, 8H, 4-H und $P(OMe)_2$ bei 3.85 ppm), 4.65 - 5.1 (mc, 1H, 5-H), 6.8 - 7.55 ppm (m, 3H, Thienyl-H).

Beispiel 75: 3-(2-Thienyl)-2-isoxazolin-5-yl-phosphonsäure (nach Verfahrensweise c))

Aus 8.9 g des Esters aus Beispiel 74 erhält man analog Beispiel 34 und Umkristallisation aus Dichlormethan 4.5 g kristalline Phosphonsäure vom Schmelzpunkt 169° C (Zers.).
$^1$H-NMR (DMSO-$d_6$):
$\delta$ = 3.05 - 3.9 (m, 2H, 4-H), 4.45 - 4.95 (mc, 1H, 5-H), 7.0 - 7.8 (m, 3H, Thienyl-H), 9,5 ppm (sb, 2H, $P(OH)_2$).

| $C_7H_8NO_4PS$ (233.2) | | | | | |
|---|---|---|---|---|---|
| Analyse: | Ber.:<br>Gef.: | C 36.05<br>C 35.87 | H 3.46<br>H 3.11 | N 6.01<br>N 5.66 | S 13.75<br>S 13.78 |

Beispiel 76: 3-tert.Butyl-isoxazol-5-yl-phosphonsäurediethylester (nach Verfahrensweise b))

Die Herstellung erfolgt, wie in Beispiel 18 beschrieben, durch Umsetzung von 20.2 g (0.2 mol) Pivalaldoxim, 29.35 g (0.22 mol) N-Chlorsuccinimid, 53.5 g (0.22 mol) $\alpha$ = Bromvinylphosphonsäurediethylester und 61.2 ml (0.44 mol) Triethylamin in Dichlormethan. Das als Öl anfallende Rohprodukt wird durch Chromatographie an Kieselgel gereinigt. Man gewinnt 20.6 g reine ölige Verbindung.
$^1$H-NMR ($CDCl_3$):
$\delta$ = 1.0 - 1.5 (15H, $P(OEt)_2$ und tBu als s bei 1.33 ppm), 3.8 - 4.4 (m, 4H, $P(OEt)_2$), 6.65 ppm (d, J = 1.8 Hz, 1H, 4-H).

Beispiel 77: 3-tert.Butyl-isoxazol-5-yl-phosphonsäureDiammoniumsalz (nach Verfahrensweise c))

12 g (46 mmol) des Diethylesters aus Beispiel 76 werden gemäß Beispiel 34 der Esterspaltung unterzogen. Nach Überführung in das Diammoniumsalz analog Beispiel 35 und Kristallisation aus Aceton erhält man 10.3 g des kristallinen Produktes vom Schmelzpunkt 185° bis 190° C, das bei scharfem Trocknen unter vermindertem Druck bis zu 25% Ammoniak verlieren kann.
$^1$H-NMR ($D_2O$):
$\delta$ = 1.3 (s, 9H, tBu), 6.4 ppm (d, J = 1.5 Hz, 1H, 4-H).

Beispiel 78: 3-Brom-2-isoxazolin-5-yl-methyl-(P-methyl)- phosphinsäureethylester (nach Verfahrensweise a)-)

Man löst 20 g (0.135 mol) Allyl-(P-methyl)-phosphinsäure-ethylester in 570 ml Ethylacetat, versetzt mit 49.7 g (0.6 mol) Natriumhydrogencarbonat in 115 ml Wasser und tropft unter intensivem Rühren eine Lösung von 40.6 g (0.2 mol) Dibromformaldoxim in 115 ml Ethylacetat langsam hinzu. Aus der Ethylacetat-Phase werden 15 g und aus der Wasserphase durch Extraktion mit Dichlormethan bei pH 2 weitere 19 g des Produktes in öliger Form isoliert.
$^1$H-NMR ($CDCl_3$):
$\delta$ = 1.3 (t, J = 7 Hz, 3H, P-OEt), 1.55 (d, J = 14 Hz, 3H, P-Me), 1.9 - 2.4 (m, 2H, $CH_2$-P), 2.9 - 3.5 (m, 2H, 4-H), 3.65 - 4.3 (mc, 2H, P-OEt), 4.6 - 5.35 ppm (mc, 1H, 5-H).

Beispiel 79: 3-Chlor-2-isoxazolin-5-yl-methyl-(P-methyl)-phosphinsäure (nach Verfahrensweise f))

Zu einer unter Argon als Schutzgas hergestellten Lösung von 9 g (33 mmol) des Esters aus Beispiel 78 in 100 ml trockenem Dichlormethan gibt man 3.9 g (35 mmol) Chlortrimethylsilan, läßt 4 Tage bei Raumtemperatur stehen, versetzt mit Wasser bis zur Trübung, rührt 1 Stunde nach und engt ein. Nach Umkristallisation des Rückstandes aus Ethylacetat/Petrolether werden 3.3 g Reinprodukt vom Schmelzpunkt 116° C erhalten.
$^1$H-NMR (DMSO-$d_6$):
$\delta$ = 1.35 (d, J = 14 Hz, 3H, P-Me), 1.8 - 2.3 (m, 2H, $CH_2$-P), 2.75 - 3.65 (m, 2H, 4-H), 4.5 - 5.2 (mc, 1H, 5-H), 9.7 ppm (sb, 1H, P-OH).

$C_5H_9ClNO_3P$ (197.6)

| | | | | |
|---|---|---|---|---|
| Analyse: Ber.: | C 30.40 | H 4.59 | N 7.09 | Cl 17.95 |
| | C 29.93 | H 4.45 | N 7.12 | Cl 17.20 |

Alle vorgenannten Verbindungen sind mit ihren gemäß Formel I variablen Strukturelementen $R^1$, A, n, X und Y in nachstehender Tabelle 3 zusammengefaßt.

<u>Tabelle 3:</u> Verbindungen der Formel I

| Bei=spiel | $R^1$ | A | n | X | Y |
|---|---|---|---|---|---|
| 1 | 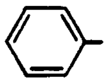 | $-CH_2-CH-$ | 0 | $-OC_2H_5$ | $-OC_2H_5$ |
| 2 | 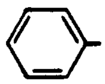 | $-CH_2-CH-$ | 1 | $-OC_2H_5$ | $-OC_2H_5$ |
| 3 | 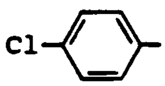 | $-CH_2-CH-$ | 0 | $-OC_2H_5$ | $-OC_2H_5$ |
| 4 | 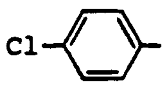 | $-CH_2-CH-$ | 1 | $-OC_2H_5$ | $-OC_2H_5$ |
| 5 | 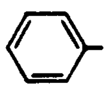 | $-CH_2-CH-$ | 0 | $-OCH_3$ | $-OCH_3$ |
| 6 | 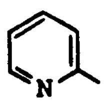 | $-CH_2-CH-$ | 0 | $-OC_2H_5$ | $-OC_2H_5$ |
| 7 | 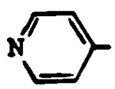 | $-CH_2-CH-$ | 0 | $-OC_2H_5$ | $-OC_2H_5$ |
| 8 | 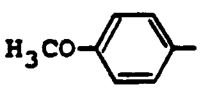 | $-CH_2-CH-$ | 0 | $-OC_2H_5$ | $-OC_2H_5$ |
| 9 | 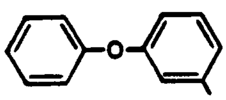 | $-CH_2-CH-$ | 0 | $-OCH_3$ | $-OCH_3$ |
| 10 | 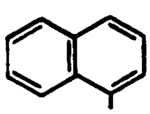 | $-CH_2-CH-$ | 0 | $-OCH_3$ | $-OCH_3$ |
| 11 | 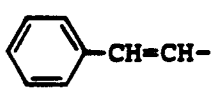 | $-CH_2-CH-$ | 0 | $-OC_2H_5$ | $-OC_2H_5$ |

| Beispiel | $R^1$ | A | n | X | Y |
|---|---|---|---|---|---|
| 12 | 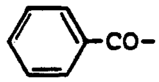 | $-CH_2-CH-$ | 0 | $-OC_2H_5$ | $-OC_2H_5$ |
| 13 | 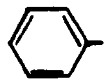 | $-CH_2-CH-$ | 0 | $-CH_3$ | $-OCH_3$ |
| 14 | 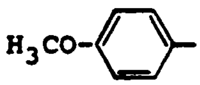 | $-CH_2-CH-$ | 0 | $-CH_3$ | $-OCH_3$ |
| 15 | 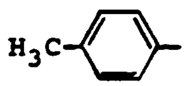 | $-CH_2-CH-$ | 0 | $-CH_3$ | $-OCH_3$ |
| 16 | 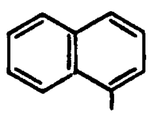 | $-CH_2-CH-$ | 0 | $-CH_3$ | $-OCH_3$ |
| 17 | 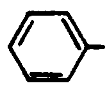 | $-CH_2-CH-$ | 0 | $-CH_3$ | $-CH_3$ |
| 18 | 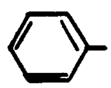 | $-CH=C-$ | 0 | $-OC_2H_5$ | $-OC_2H_5$ |
| 19 | 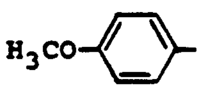 | $-CH=C-$ | 0 | $-OC_2H_5$ | $-OC_2H_5$ |
| 20 | 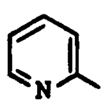 | $-CH=C-$ | 0 | $-OC_2H_5$ | $-OC_2H_5$ |
| 21 | 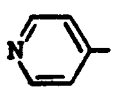 | $-CH=C-$ | 0 | $-OC_2H_5$ | $-OC_2H_5$ |
| 22 | $H_3C-(CH_2)_2-$ | $-CH_2-CH-$ | 0 | $-OC_2H_5$ | $-OC_2H_5$ |

| Bei= spiel | $R^1$ | A | n | X | Y |
|---|---|---|---|---|---|
| 23 | 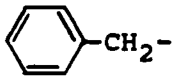 | $-CH_2-CH-$ | 0 | $-OCH_3$ | $-OCH_3$ |
| 24 | 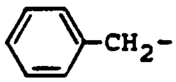 | $-CH_2-CH-$ | 0 | $-CH_3$ | $-OCH_3$ |
| 25 | $(H_3C)_3C-$ | $-CH_2-CH-$ | 0 | $-CH_3$ | $-OCH_3$ |
| 26 | $(H_3C)_3C-$ | $-CH_2-CH-$ | 0 | $-OC_2H_5$ | $-OC_2H_5$ |
| 27 | 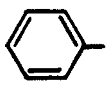 | $-CH_2-CH-$ | 0 | $-OH$ | $-OC_2H_5$ |
| 28 | 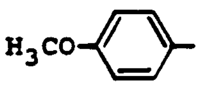 | $-CH_2-CH-$ | 0 | $-O^-NH_4^+$ | $-OC_2H_5$ |
| 29 | 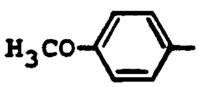 | $-CH_2-CH-$ | 0 | $-CH_3$ | $-O^-NH_4^+$ |
| 30 | 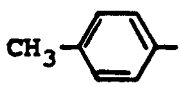 | $-CH_2-CH-$ | 0 | $-CH_3$ | $-OH$ |
| 31 | 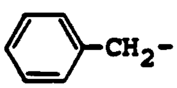 | $-CH_2-CH-$ | 0 | $-CH_3$ | $-O^-NH_4^+$ |
| 32 | 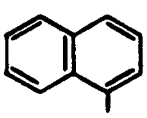 | $-CH_2-CH-$ | 0 | $-CH_3$ | $-OH$ |
| 33 | $(H_3C)_3C-$ | $-CH_2-CH-$ | 0 | $-OH$ | $-OH$ |

| Bei= spiel | $R^1$ | A | n | X | Y |
|---|---|---|---|---|---|
| 34 | 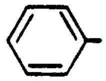 | $-CH_2-CH-$ | 0 | -OH | -OH |
| 35 | 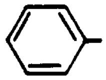 | $-CH_2-CH-$ | 0 | $-O^-NH_4^+$ | $-O^-NH_4^+$ |
| 36 | 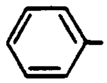 | $-CH_2-CH-$ | 0 | $-O^-Na^+$ | $-O^-Na^+$ |
| 37 | 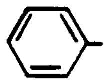 | $-CH_2-CH-$ | 0 | -OH | $-O^-K^+$ |
| 38 | 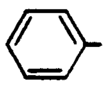 | $-CH_2-CH-$ | 0 | -OH x 2 Lysin | -OH |
| 39 | 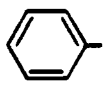 | $-CH_2-CH-$ | 1 | -OH | -OH |
| 40 | 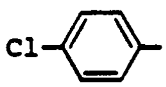 | $-CH_2-CH-$ | 0 | -OH | -OH |
| 41 | 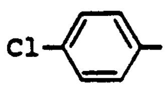 | $-CH_2-CH-$ | 1 | -OH | -OH |
| 42 | 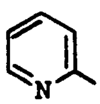 x HBr | $-CH_2-CH-$ | 0 | -OH | -OH |
| 43 | 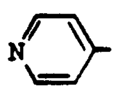 | $-CH_2-CH-$ | 0 | $-O^-NH_4^+$ | $-O^-NH_4^+$ |
| 44 | 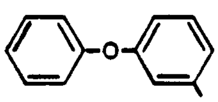 | $-CH_2-CH-$ | 0 | -OH | -OH |

| Bei= spiel | $R^1$ | A | n | X | Y |
|---|---|---|---|---|---|
| 45 | 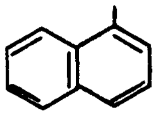 | $-CH_2-CH-$ | 0 | $-OH$ | $-OH$ |
| 46 | 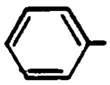 | $-CH=C-$ | 0 | $-OH$ | $-OH$ |
| 47 | $H_3C-(CH_2)_2-$ | $-CH_2-CH-$ | 0 | $-O^-NH_4^+$ | $-O^-NH_4^+$ |
| 48 | $H_3CO-$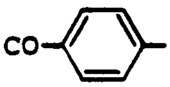 | $-CH_2-CH-$ | 0 | $-OH$ | $-OH$ |
| 49 | 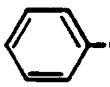$-CH_2-$ | $-CH_2-CH-$ | 0 | $-OH$ | $-OH$ |
| 50 | 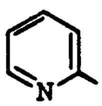 | $-CH=C-$ | 0 | $-O^-NH_4^+$ | $-O^-NH_4^+$ |
| 51 | 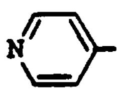 | $-CH=C-$ | 0 | $-O^-NH_4^+$ | $-O^-NH_4^+$ |
| 52 | 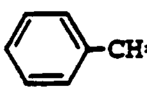$-CH=CH-$ | $-CH_2-CH-$ | 0 | $-OH$ | $-OH$ |
| 53 | 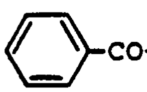$-CO-$ | $-CH_2-CH-$ | 0 | $-O^-NH_4^+$ | $-O^-NH_4^+$ |
| 54 | $H_3CO-$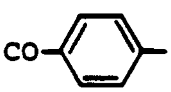 | $-CH=C-$ | 0 | $-O^-NH_4^+$ | $-O^-NH_4^+$ |
| 55 | 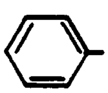 | $-CH_2-CH-$ | 0 | $-OC_2H_5$ | 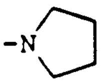 |

| Bei= spiel | $R^1$ | A | n | X | Y |
|---|---|---|---|---|---|
| 56 | 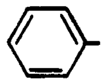 | $-CH_2-CH-$ | 0 | $-OC_2H_5$ | 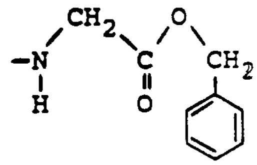 |
| 57/58 | 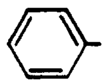 | $-CH_2-CH-$ | 0 | $-CH_3$ | 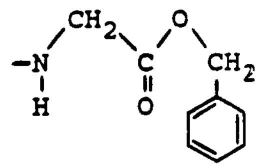 |
| 59 | 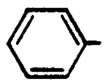 | $-CH_2-CH-$ | 0 | $-N(CH_3)_2$ | $-N(CH_3)_2$ |
| 60a | 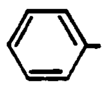<br>(+)-Enantiomer | $-CH_2-CH-$ | 0 | $-OCH_3$ | $-OCH_3$ |
| 60b | 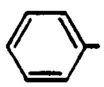<br>(-)-Enantiomer | $-CH_2-CH-$ | 0 | $-OCH_3$ | $-OCH_3$ |
| 61 | 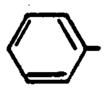<br>(+)-Enantiomer | $-CH_2-CH-$ | 0 | $-OH$ | $-OH$ |
| 62 | 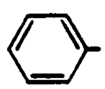<br>(-)-Enantiomer | $-CH_2-CH-$ | 0 | $-OH$ | $-OH$ |
| 63 | 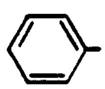 | $-CH_2-CH-$ | 0 | $-CH_3$ | $-OH$ |
| 64 | $(H_3C)_3C-$ | $-CH_2-CH-$ | 0 | $-CH_3$ | $-OH$ |

| Bei=spiel | $R^1$ | A | n | X | Y |
|---|---|---|---|---|---|
| 65 | 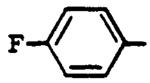 | $-CH_2-CH-$ | 0 | $-OCH_3$ | $-OCH_3$ |
| 66 | 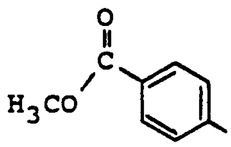 | $-CH_2-CH-$ | 0 | $-OCH_3$ | $-OCH_3$ |
| 67 | 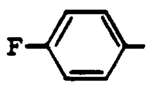 | $-CH_2-CH-$ | 0 | -OH | -OH |
| 68 | 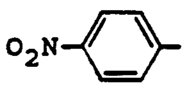 | $-CH_2-CH-$ | 0 | $-OCH_3$ | $-OCH_3$ |
| 69 | 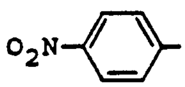 | $-CH_2-CH-$ | 0 | -OH | -OH |
| 70 | 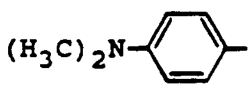 | $-CH_2-CH-$ | 0 | $-OCH_3$ | $-OCH_3$ |
| 71 | 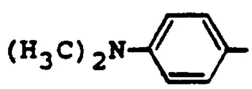 x HBr | $-CH_2-CH-$ | 0 | -OH | -OH |
| 72 | 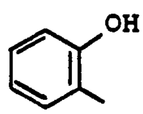 | $-CH_2-CH-$ | 0 | $-OCH_3$ | $-OCH_3$ |
| 73 | 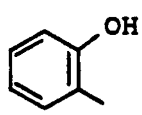 | $-CH_2-CH-$ | 0 | -OH | -OH |
| 74 | 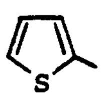 | $-CH_2-CH-$ | 0 | $-OCH_3$ | $-OCH_3$ |

| Bei= spiel | $R^1$ | A | n | X | Y |
|---|---|---|---|---|---|
| 75 | 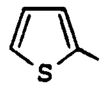 | $-CH_2-CH-$ | 0 | -OH | -OH |
| 76 | $(H_3C)_3C-$ | -CH=C- | 0 | $-OC_2H_5$ | $-OC_2H_5$ |
| 77 | $(H_3C)_3C-$ | -CH=C- | 0 | $-O^-NH_4^+$ | $-O^-NH_4^+$ |
| 78 | Br | $-CH_2-CH-$ | 1 | $-CH_3$ | $-OC_2H_5$ |
| 79 | Cl | $-CH_2-CH-$ | 1 | $-CH_3$ | -OH |

Die weiter folgenden Beispiele 80 - 105 wurden analog den vorher beschriebenen Beispielen durchgeführt (Cycloaddition der entsprechenden aliphatischen und aromatischen Nitriloxide an olefinische Phosphorverbindungen sowie ggf. Umwandlung der funktionellen Gruppen). Die erhaltenen Verbindungen der Formel I wurden durch Elementaranalysen und Kernresonanzspektren identifiziert; in Tabelle 3a sind die Verbindungen in gleicher Weise wie vorher die Verbindungen gemäß den Beispielen 1 - 79 zusammengestellt, wobei hier noch die Schmelzpunkte mit in die Tabelle aufgenommen wurden.

Ergänzende Bemerkungen zu einigen Ansprüchen sind in den Fußnoten der Tabelle 3a angegeben.

Tabelle 3a: Verbindungen der Formel I

| Beispiel | $R^1$ | A | n | X | Y | Fp (°C) |
|---|---|---|---|---|---|---|
| 80 | 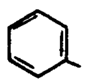 | $-CH_2-CH-$ | 0 | -OH | $-OCH_3$ | 141-144 |
| 81 | 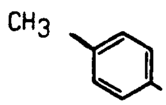 | " | 0 | -OH | -OH | 218 |
| 82 | 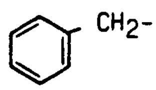 | " | 1 | -OH | -OH | 110-119 |
| 83 | 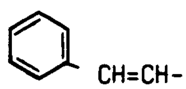 | " | 1 | -OH | -OH | 230-234 (Zers.) |
| 84(1) | 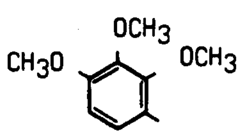 | " | 0 | $-O^- Na^+$ | $-O^- Na^+$ | > 280 |
| 85 | 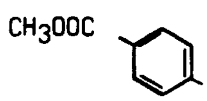 | " | 0 | -OH | -OH | 218 (Zers.) |
| 86 | 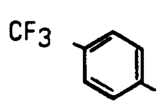 | " | 0 | -OH | -OH | 195-197 |

Tabelle 3a: Verbindungen der Formel I

| Beispiel | $R^1$ | A | n | X | Y | Fp (°C) |
|---|---|---|---|---|---|---|
| 87(2) | $Na^+$ $^-$OOC 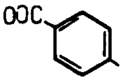 | $-CH_2-CH-$ | 0 | $-O^-$ $Na^+$ | $-O^-$ $Na^+$ | >295 |
| 88(3) | 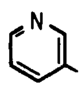 | " | 0 | -OH | -OH | 271 (Zers.) |
| 89(4) | 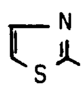 xHBr | " | 0 | -OH | -OH | 179-188 (Zers.) |
| 90(5) | 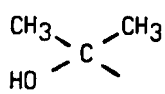 | " | 0 | $-OCH_3$ | $-OCH_3$ | Öl |
| 91(6) | HOOC - | " | 1 | $-OC_2H_5$ | $-OC_2H_5$ | 111 |
| 92(7) | HOOC- | " | 1 | -OH | $-OC_2H_5$ | 124 |
| 93 | $(H_3C)_3C-$ | " | 1 | $-O^-$ $NH_4^+$ | $-O^-$ $NH_4^+$ | >180 (Zers.) |
| 94 (8) | 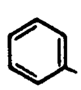 | " | 0 | $-O-(CH_2)_3-O-$ | | 152 |

Tabelle 3a: Verbindungen der Formel I

| Beispiel | $R^1$ | A | n | X | Y | Fp (°C) |
|---|---|---|---|---|---|---|
| 95 | 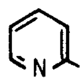 | $-CH_2-CH-$ | 0 | $-CH_3$ | -OH | 138-139 |
| 96 (9) | $CH_3OOC$ 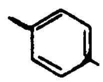 | " | 0 | $-CH_3$ | -OH | 214-216 (Zers.) |
| 97 (10) | HOOC 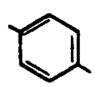 | " | 0 | $-CH_3$ | -OH | 259-261 (Zers.) |
| 98 (11) | 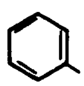 | " | 1 | $-CH_3$ | -OH | 147 |
| 99 (12) | $(CH_3)_3C-$ | " | 1 | $-CH_3$ | $-O^- NH_4^+$ | 161-165 |
| 100 | 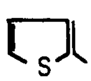 | " | 0 | $-CH_3$ | -OH | 151-158 |

Tabelle 3a: Verbindungen der Formel I

| Bei-spiel | $R^1$ | A | n | X | Y | Fp (°C) |
|---|---|---|---|---|---|---|
| 101(13) | $C_2H_5OOC$- | $-CH_2-CH-$ | 0 | $-CH_3$ | -OH | 96 (Zers.) |
| 102(14) | $Na^+$ $^-OOC$ - | " | 0 | $-CH_3$ | $-O^-$ $Na^+$ | 210-215 (Zers.) |
| 103(15) | 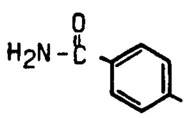 | " | 0 | $-CH_3$ | -OH | 261 (Zers.) |
| 104(16) | 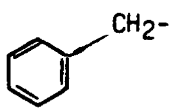 | -CH=C- | 0 | $-O^-$ $NH_4^+$ | $-O^-$ $NH_4^+$ | 197-203 (Zers.) |
| 105(17) | 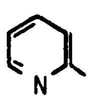 | $-CH_2-CH-$ | 0 | $-CH_3$ | $-CH_3$ | 185 |

Fußnoten zur Tabelle 3a

(1) Fällung aus methanolischer Lösung mittels 2-Ethylhexansäure-Natriumsalz

(2) Hergestellt durch alkalische Verseifung der Verbindung von Beispiel 85

(3) Hergestellt aus 3-Pyridylhydroxamoylchlorid-Hydrochlorid analog Beispiel 6 und 42

(4) Hergestellt aus Thiazolyl-2-aldoxim (vgl. A. Dondoni et al., Synthesis 1987, 998) analog Beispiel 2 und 34

(5) Synthese erfolgte ausgehend von 2-[(Trimethylsilyl)-oxy]-2-methyl-1-nitropropan (vgl. D.P. Curran et al., J. Org. Chem. 49 (1984), 3474) in Analogie zu Beispiel 22; Abspaltung der Schutzgruppe mittels Trifluoressigsäure

(6) Erzeugung des Nitriloxides aus Chloroxyiminoessigsäureethylester mittels Triethylamin analog Beispiel 1; Verseifung des entspr. Isoxazolin-3-carbonsäureethylesters mit 1 Äquivalent Natronlauge, dann Kristallisation als Säure

(7) aus Beispiel 91 mit Überschuß an Natronlauge

(8) Hergestellt analog Beispiel 56: Aktivierung mit 2 Äquivalenten $PCl_5$, dann Umsetzung mit 1,3-Propandiol und Triethylamin

(9) Selektive Spaltung des Phosphinsäureethylesters mittels HBr/Eisessig

(10) aus Beispiel 96 durch alkalische Verseifung

(11) Durchführung analog Beispiel 1 und 33; als Olefinkomponente wurde Allyl-(P-methyl)-phosphinsäureethylester eingesetzt

(12) analog Beispiel 98

(13) Herstellung siehe Beispiel 91; die Spaltung des Phosphinsäureesters erfolgte mittels HBr/Eisessig

(14) aus Beispiel 101 durch alkalische Verseifung

(15) Einführung des Carbamoylsubstituenten durch Behandlung des entsprechenden Nitrils mit Bromwasserstoff

(16) analog Beispiel 18 und 23

(17) analog Beispiel 17

Pharmakologische Prüfung und Ergebnisse

Die Verbindungen der Formel I wurden zur Charakterisierung ihrer wertvollen immunodulierenden Eigenschaften und ausgezeichneten Verträglichkeit in solchen experimentellen Testanordnungen untersucht, die sich anerkanntermaßen besonders gut für die Beurteilung der Wirkqualität von immunpharmakologisch aktiven Präparaten eignen.

1. Aktive Arthus-Reaktion an der Ratte

Als Versuchstiere dienten weibliche und männliche Sprague-Dawley Ratten mit einem Körpergewicht zwischen 80 und 100 g, denen 0,5 ml einer Emulsion von Pertussis-Vakzine und Ovalbumin in Paraffinöl subkutan in die Schwanzwurzel injiziert wurde. Nach zwei Wochen wurden die Ratten in Gruppen von jeweils 8 Tieren aufgeteilt. 24 Stunden und 1 Stunde vor Auslösung der Arthus-Reaktion durch Injektion von 0,1 ml einer 0,4 %igen Ovalbumin-Lösung in die rechte Hinterpfote erfolgte die orale Verabreichung der jeweiligen Prüfsubstanz oder des reinen Vehikels (Positivkontrolle). In die linke Pfote wurde Natriumchlorid-Lösung injiziert. Eine Gruppe nicht sensibilisierter Tiere (Negativkontrolle) wurde gleichfalls mit Ovalbumin behandelt, um unspezifische Reaktionen gegenüber dem Protein ausschliessen zu können. Als Meßparameter für die Präparatwirkung diente die prozentuale Veränderung der Pfotenvolumenzunahme gegenüber jener der zwar sensibilisierten, aber unbehandelten Kontrollgruppe (Positivkontrolle) 4 Stunden nach der Ovalbumin-Provokation, wenn die Schwellung ihren Maximalwert erreicht.

2. Akute Toxizität

Die Bestimmung der $LD_{50}$-Werte erfolgte standardgemäß über die innerhalb von 7 Tagen bei NMRI-(Naval Medical Research Institute)-Mäusen (6 Tiere pro dosi) nach einmaliger intraperitonealer (i.p.) oder intravenöser (i.v.) Gabe auftretende Mortalität.

Die Ergebnisse aus den Versuchen zur Arthus-Reaktion und Toxizität sind in nachstehender Tabelle 4 zusammengefaßt.

<u>Tabelle 4:</u> Wirkung auf die aktive Arthus-Reaktion und akute Toxizität

| Verbindung aus Beispiel | Aktive Arthus-Reaktion Dosis in mg/kg p.o. | % Änderung | Akute Toxizität $LD_{50}$ (mg/kg) i.p. | i.v. |
|---|---|---|---|---|
| 5 | 100 | - 20 | 300-600 | |
| 13 | 50 | - 13 | | >200 |
| 17 | 35 | - 34 | | >200 |
| 27 | 70 | + 108 | | >200 |
| 28 | 25 | - 12 | | >200 |
| 31 | 50 | + 66 | | >200 |
| 32 | 50 | + 42 | 600-1200 | |
| 33 | 100 | - 15 | | >200 |
| 34 | 35 | - 16 | | 200-300 |
| 35 | 35 | - 16 | | >200 |
| 39 | 50 | + 24 | 600-1200 | |
| 40 | 50 | - 23 | | >200 |
| 41 | 100 | + 26 | 150-300 | |
| 42 | 35 | - 33 | | >200 |
| 43 | 50 | - 14 | | >200 |
| 45 | 50 | - 25 | 75-150 | |
| 46 | 50 | - 36 | | >200 |
| 47 | 25 | - 35 | | >200 |
| 48 | 100 | + 10 | 300-600 | |
| 49 | 50 | - 59 | | >200 |
| 50 | 25 | - 18 | | >200 |
| 51 | 50 | + 17 | | >200 |
| 52 | 50 | - 15 | | |
| 54 | 40 | - 57 | | >200 |

Tabelle 4 (Fortsetzung)

| Verbindung aus Beispiel | Aktive Arthus-Reaktion Dosis in mg/kg p.o. | % Änderung | Akute Toxizität $LD_{50}$ (mg/kg) i.p. | i.v. |
|---|---|---|---|---|
| 55 | 70 | - 23 | | >200 |
| 56 | 100 | + 35 | >1200 | |
| 57/58 | 12 | + 22 | | 50-100 |
| 59 | 50 | - 25 | | >200 |
| 61 | 50 | + 52 | | |
| 62 | 50 | + 16 | | |
| 63 | 50 | - 48 | 300-600 | |
| 64 | 20 | - 59 | | >200 |
| 65 | 25 | - 25 | >300 | |
| 66 | 50 | - 28 | | |
| 67 | 50 | - 35 | | |
| 69 | 50 | - 20 | | >100 |
| 71 | 70 | - 10 | 150-300 | |
| 73 | 35 | - 44 | 150-300 | |
| 75 | 35 | - 26 | | >100 |
| 77 | 35 | - 20 | | |
| 79 | 70 | - 24 | | |
| 87 | 35 | - 29 | | |
| 88 | 50 | - 50 | | |
| 90 | 50 | - 31 | | |
| 99 | 35 | - 32 | | >100 |
| 101 | 70 | - 32 | | >100 |
| 102 | 35 | - 35 | | |
| 105 | 35 | - 23 | | >100 |

3. Chronische Graft-versus-Host(cGvH)-Reaktion an der Maus

Die Graft-versus-Host-Krankheit, der eine vom Transplantat ausgehende, gegen das Wirtsgewebe gerichtete Immunreaktion zugrunde liegt, ist bei akuter, fast immer tödlich endender Verlaufsform durch Milzvergrößerung, Leberschwellung, Hypertrophie der Lymphknoten, hämolytische Anämie, erniedrigte Immunglobulin- und Komplementspiegel sowie verminderte Immunreaktivität gekennzeichnet. Die etwas milder verlaufende chronische Krankheitsform führt zu Lymphadenopathie, Immunkomplexglomerulonephritis und zur exzessiven Bildung von organunspezifischen Autoantikörpern. Ein ähnliches Krankheitsbild kennzeichnet den systemischen Lupus erythematodes (SLE), der ebenfalls zum Formenkreis der Autoimmunerkrankungen gehört.
Die Untersuchung der erfindungsgemäß verwendeten Verbindungen auf den Krankheitsverlauf der durch zwei Injektionen von miteinander gemischten Milz- und Thymuszellen an weiblichen Mäusen der (DBA/2 x C57Bl/6)F1-Generation ausgelösten cGvH-Reaktion erfolgte in der von S. Popovic und R. R. Bartlett beschriebenen Versuchsanordnung (Agents and Actions 21 (1987), 284 - 286), wobei im zeitlichen Abstand von 7 Tagen jeweils 5 x $10^7$ ebenfalls von weiblichen Spendertieren gewonnene DBA/2-Zellen in 0,2 ml Kulturmedium intravenös verabreicht wurden. Zur sicheren Beurteilung des Krankheitsausbruches und -verlaufes wurde bei allen Versuchen ein Kollektiv gesunder Tiere als Negativkontrolle mitgeführt. Die 6-wöchige orale Behandlung der kranken Tiere erfolgte ab dem 21. Tage nach der ersten Donorzellen-Injektion, wobei die Prüfsubstanzen oder das reine Vehikel (Positivkontrolle) einnmal täglich appliziert wurden. Als Vehikel diente eine wäßrige CMC(Carboxymethylcellulose-Natriumsalz)-Lösung mit einem Gehalt von 100 mg CMC pro 1. Das Applikationsvolumen betrug 10 ml pro kg Körpergewicht. Die einzelnen

Versuchskollektive umfaßten jeweils 10 Tiere.
Die Präparatwirkung wurde anhand der Hemmung der Proteinurie und des cGvH-Index beurteilt. Die kranken Tiere entwickeln infolge der Zerstörung von Nephronen durch Immunkomplexablagerung an der Basalmembrane der Glomeruli eine ausgeprägte Proteinurie, die mit dem Ausmaß der Glomerulonephritis korreliert und die sich über den Anstieg der mit dem Urin ausgeschiedenen Proteinmenge leicht quantifizieren läßt. Der zweite Meßparameter, der cGvH-Index, bezieht sich auf die durch die cGvH-Reaktion hervorgerufene starke Milzvergrößerung (Splenomegalie). Er ist definiert als der Quotient aus dem Produkt von Milz- und Körpergewicht der kranken Tiere und dem Produkt der entsprechenden Gewichte gesunder, unbehandelter Tiere aus der Negativkontrollgruppe und stellt ein verläßliches Maß für die Krankheitsintensität dar (je größer dieser Index, desto stärker die Krankheit).

Die in Tabelle 5 zusammengefaßten Ergebnisse dieser Untersuchungen belegen, daß Verbindungen der Formel I die cGvH-Krankheit durch modulierenden Eingriff in die Autoimmunprozesse nachhaltig zu lindern vermögen.

Tabelle 5

| Hemmung der Proteinurie und des cGvH-Index | | | |
|---|---|---|---|
| Verbindung aus Beispiel | Dosis in mg/kg/Tag p.o | % Hemmung | |
| | | Proteinurie | cGvH-Index |
| 54 | 15<br>50 | 27<br>64 | 16<br>18 |
| 63 | 15<br>30 | 76<br>61 | 54<br>48 |

4. <u>Hemmwirkung auf die Enzymaktivität von Aminopeptidasen</u>

Die Enzymaktivität der Aminopeptidase B wurde mit dem Substrat L-Lysin-2-naphthylamid bei Raumtemperatur photometrisch bestimmt. Zusatz der Prüfsubstanzen in Konzentrationen von 0.1 - 100 µg/ml zum Enzymansatz führte zu einer dosisabhängigen Suppression der Enzymaktivität. Aus diesen Daten wurden jene Substanzkonzentrationen berechnet, die eine 50 %ige Inhibition der Enzymaktivität ($IC_{50}$) bewirken. In gleicher Weise wurde die Inhibition der Leucin-Aminopeptidase unter Verwendung von L-Leucin-4-nitroanilid als Enzymsubstrat untersucht.
Die $IC_{50}$-Wert sind in der Tabelle 6 zusammengestellt.

Tabelle 6

| Inhibition der Aminopeptidasen | | |
|---|---|---|
| Verbindung aus Beispiel | Aminopeptidase B $IC_{50}$ ($\mu$g/ml) | Leucin-Aminopeptidase $IC_{50}$ ($\mu$g/ml) |
| 1 | | 65 |
| 13 | 100 | |
| 34 | 22.5 | 24 |
| 35 | | 57 |
| 36 | | 56 |
| 37 | | 52 |
| 45 | | 92 |
| 47 | | 35 |
| 48 | | 100 |
| 53 | | 65 |
| 61 | | 26 |
| 62 | | 130 |
| 67 | | 57 |
| 69 | | 27 |
| 71 | | 37 |
| 75 | | 79 |

5. Wirkung auf die zelluläre Immunreaktion vom verzögerten Typ gegen Schaferythrozyten [DTH(delayed-type hypersensitivity)-Reaktion]

Es wurden Gruppen von jeweils 5 weiblichen NMRI-Mäusen mit einem Körpergewicht von 18 bis 20 g gebildet und jedem Tier $10^6$ bzw. $10^9$ rote Blutkörperchen vom Schaf intravenös verabreicht. Schaferythrozyten gelten in der Immunologie als Standardantigen, mit dem sich die zelluläre und humorale Immunreaktivität, insbesondere die Funktionsfähigkeit der T-Zell-abhängigen Komponente des Immunsystems, der sogenannten T-Helferzellen, überprüfen läßt.
Gleichzeitig mit dem Antigen wurden die Prüfsubstanzen in Dosen von 5 bis 100 mg/kg in physiologischer Kochsalzlösung intraperitoneal appliziert. Nach 5 Tagen wurden allen Tieren jeweils 2 x $10^8$ Schaferythrozyten in die Fußsohle injiziert. 24 Stunden später wurde die Schwellung des Fusses gemessen. Die Fußschwellung wird durch eine Hautreaktion vom verzögerten Typ (DTH-Reaktion) ausgelöst und ist, wie dem Fachmann bekannt, ein Maß für die zelluläre Immunantwort (J. Immunol. 101 (1968), 830 - 845).
Die exemplarisch mit dem Präparat des Beispiels 34 erhaltenen Testergebnisse sind in Tabelle 7 zusammengestellt und veranschaulichen, daß die Verbindungen der Formel I - prophylaktisch verabreicht - die zelluläre Immunantwort nach Immunisierung mit dem Antigen durch Stimulation des T-Zell-Systems zu steigern vermögen, wobei in diesem Experiment die stimulierende Wirkung ihr Optimum bei einer Dosis von 25 mg/kg erreichte.

Tabelle 8 zeigt die relative Wirkung weiterer Prüfsubstanzen bei einer Dosis von 40 mg/kg, bezogen auf jene der Verbindung aus Beispiel 34, deren maximale Stimulation (Differenz zwischen behandelten und unbehandelten Tieren) 100 % entspricht.

6. Stimulation der unspezifischen Immunität - Aktivierung von mononukleären Phagozyten

Makrophagen spielen bei allen Immunprozessen, einschließlich der Abwehr von Infektionserregern, eine zentrale Rolle. Einerseits wirken sie selbst bei der Elimination der Krankheitserreger mit, andererseits üben sie Kontrollfunktionen bei der Regulation des humoralen (B-Zell-abhängigen) und des zellulären (T-Zell-abhängigen) Immunsystems aus.
Hier wurde der stimulierende Einfluß der erfindungsgemäß verwendeten Verbindungen auf Peritonealmakrophagen bei 6

Tabelle 7

| Wirkung auf die zelluläre Immunantwort (DTH-Reaktion) | | | |
|---|---|---|---|
| Testsubstanz | Dosis in mg/kg (1x i.p.) | % Fußschwellung nach Immunisierung mit | |
| | | $10^6$ Erythrozyten | $10^9$ Erythrozyten |
| PBS* (Vehikel) | | 15.6 ± 4.1 | 16.9 ± 4.0 |
| Verbindung aus Beispiel 34 | 5.0 | 25.3 ± 5.8 | 22.8 ± 3.5 |
| | 10.0 | 28.9 ± 7.7 | 24.1 ± 5.7 |
| | 12.5 | 29.9 ± 3.5 | 26.1 ± 8.9 |
| | 20.0 | 32.5 ± 3.0 | 30.5 ± 6.5 |
| | 25.0 | 33.7 ± 6.6 | 34.1 ± 7.4 |
| | 40.0 | 29.7 ± 4.7 | 32.8 ± 5.6 |
| | 50.0 | 29.2 ± 6.9 | 28.1 ± 4.1 |
| | 100.0 | 27.3 ± 4.6 | 26.6 ± 5.0 |

* PBS = Phosphat-gepufferte Kochsalzlösung (NaCl: 8 g/l, KCl: 0.2 g/l, $Na_2HPO_4 \cdot 2\ H_2O$: 1.44 g/l, $KH_2PO_4$: 0.2 g/l)

Tabelle 8

| Stimulation der DTH-Reaktion | |
|---|---|
| Verbindung aus Beispiel | Relative Stimulation der DTH-Reaktion nach Einmalgabe von 40 mg/kg i.p. in % |
| 34 | 100 |
| 27 | 94 |
| 30 | 76 |
| 35 | 98 |
| 36 | 102 |
| 37 | 77 |
| 39 | 136 |
| 53 | 68 |
| 61 | 108 |
| 62 | 83 |

bis 8 Wochen alten weiblichen NMRI-Mäusen untersucht. Die Tiere erhielten die Prüfsubstanzen in Dosen von 5, 10, 20 und 40 mg/kg parenteral oder oral. Den Tieren der Kontrollgruppe wurde physiologische Kochsalzlösung verabreicht. Drei Tage nach der Substanzgabe wurden die Peritonealmakrophagen der Tiere anhand der Sekretion von lysosomalen Enzymen und der Chemilumineszenz als Maß für die Kapazität des oxidativen Stoffwechsels auf ihren Aktivierungszustand hin untersucht. Zu diesem Zweck wurden entweder 3 x $10^6$ Makrophagen mit 1 ml TC 199-Kulturmedium in Petrischalen mit einem Durchmesser von 3 cm oder aber $10^6$ Makrophagen mit 0,1 ml Kulturmedium in Rundboden-Polyethylen-Röhrchen bei 37° C unter einer Atmosphäre mit einem $CO_2$-Gehalt von 5 % kultiviert. Nach einstündiger Bebrütung wurden die Kulturen gewaschen, um schwimmende Zellen zu entfernen. Danach dienten die Röhrchen-Kulturen zur Bestimmung der Chemilumineszenz mit Hilfe eines Biolumaten. Die Zellkulturen in den Petrischalen wurden erneut 24 Stunden lang bei 37° C bebrütet und anschließend für die Aktivitätsbestimmung der durch Exozytose freigesetzten lysosomalen Enzyme verwendet.

Hierbei zeigte sich, daß Verbindungen der Formel I sowohl nach intraperitonealer (i.p.) als auch oraler (p.o.) Verabreichung die Makrophagenaktivität stimulieren und somit eine immunitätssteigernde Wirkung besitzen.

So bewirkte beispielsweise die zur Dosisfindung breiter geprüfte Verbindung des Beispiels 34 gemäß Tabelle 9 bei beiden Applikationsformen eine markante, dosisabhängige Steigerung der Chemilumineszenz als Folge der Aktivierung des oxidativen Makrophagen-Stoffwechsels mit verstärkter Sauerstoffradikalbildung und damit erhöhter Lichtemission. Aus der Tabelle 10 geht hervor, daß die Makrophagen der

Kontrolltiere nur geringe Mengen an lysosomalen Enzymen (β-Glucuronidase (β-Glu), β-Galactosidase (β-Gal) und N-Acetyl-β-D-Glucosaminidase (N-Ac-Glu)) in den Kulturüberstand abgeben. Demgegenüber wurde die Freisetzung dieser sauren Hydrolasen aus den mononukleären Phagozyten der beispielsweise mit der Verbindung des Beispiels 34 intraperitoneal oder oral behandelten Tiere dosisabhängig gesteigert. Tabelle 11 zeigt die relative Wirkung weiterer Prüfsubstanzen bei einer Dosis von 40 mg/kg i.p., bezogen auf jene der Verbindung des Beispiels 34, deren jeweilige maximale Aktivierung (Differenz zwischen behandelten und unbehandelten Tieren) 100 % entspricht.

Tabelle 9

| Wirkung auf den oxidativen Stoffwechsel von Peritonealmakrophagen der Maus | | | |
|---|---|---|---|
| Testsubstanz | Dosis in mg/kg | Chemilumineszenz in (RLE */15 min) x $10^3$ nach einmaliger Präparatgabe | |
| | | i.p. | p.o. |
| PBS (Vehikel) | | 368 ± 31 | 359 ± 48 |
| Verbindung aus Beispiel 34 | 5 | 842 ± 42 | 728 ± 101 |
| | 10 | 2842 ± 223 | 2140 ± 156 |
| | 20 | 4935 ± 516 | 3286 ± 283 |
| | 40 | 6990 ± 290 | 4405 ± 197 |

* RLE = Relative Lichteinheiten

Tabelle 10

| Stimulation der Freisetzung lysosomaler Enzyme aus Peritonealmakrophagen der Maus | | | | | | | |
|---|---|---|---|---|---|---|---|
| Testsubstanz | Dosis in mg/kg | Enzymaktivität in mU/ml nach einmaliger Präparatgabe | | | | | |
| | | ß-Glu | | ß-Gal | | N-Ac-Glu | |
| | | i.p. | p.o. | i.p. | p.o. | i.p. | p.o. |
| PBS (Vehikel) | | 751 | 678 | 1179 | 1051 | 1867 | 1701 |
| Verbinddung aus Beispiel 34 | 5 | 1197 | 973 | 2357 | 2216 | 2607 | 2071 |
| | 10 | 1542 | 1393 | 3956 | 3513 | 4822 | 4283 |
| | 20 | 2067 | 1979 | 6918 | 6011 | 6812 | 6128 |
| | 40 | 2547 | 2286 | 9318 | 9262 | 9281 | 8432 |

Tabelle 11

| Stimulation der Makrophagenaktivität | | |
|---|---|---|
| Verbindung aus Beispiel | Relative Stimulation der Makrophagenaktivität in % nach Einmalgabe von 40 mg/kg i.p. | |
| | Chemilumineszenz | Exozytose |
| 34 | 100 | 100 |
| 5 | | 54 |
| 13 | 64 | 51 |
| 27 | 63 | 78 |
| 28 | 31 | 47 |
| 29 | | 38 |
| 35 | 107 | 113 |
| 36 | 88 | 99 |
| 37 | 97 | 104 |
| 39 | 42 | 86 |
| 40 | 38 | 29 |
| 41 | | 43 |
| 42 | 35 | 41 |
| 46 | 27 | 56 |
| 47 | 44 | 32 |
| 53 | 72 | 94 |
| 55 | 46 | 67 |
| 56 | | 43 |
| 60a | 51 | 88 |
| 60b | 24 | 63 |
| 61 | 76 | 81 |
| 62 | 94 | 96 |
| 67 | 56 | 72 |
| 69 | 49 | 84 |
| 71 | 37 | 35 |
| 79 | 44 | 57 |

Die Verbindungen der Formel I wurden weiterhin in verschiedenen experimentellen Infektionsmodellen untersucht, wobei sich auch hier ihr therapeutisches Potential aufgrund der immunmodulierenden Eigenschaften eindrucksvoll demonstrieren ließ.
Im folgenden werden exemplarisch die mit der Verbindung des Beispiels 34 erhaltenen Versuchsergebnisse beschrieben.

7. Wirkung auf die Hautreaktion vom verzögerten Typ (DTH-Reaktion) in mit Listeria monocytogenes infizierten Mäusen

In diesem Experiment wurde die spezifische, zellvermittelte Immunität gegen das Bakterium Listeria monocytogenes mit Hilfe der DTH-Reaktion untersucht.
Weibliche NMRI-Mäuse wurden mit 2 x $10^2$ lebenden Bakterien infiziert und danach in Gruppen zu je 10 Tieren aufgeteilt. Am gleichen Tage wurde mit der Behandlung der Tiere durch intraperitoneale Verabreichung des Präparates in Dosen von 0.1, 1 bzw. 10 mg/kg begonnen. Diese Behandlung wurde nach 2, 4 und 6 Tagen wiederholt. Infizierte Tiere eines vierten Versuchskollektives, die anstelle des Präparates lediglich das reine Vehikel i.p. erhielten, dienten als Positivkontrolle. Am 13. Versuchstag erfolgte die Auslösung der DTH-Reaktion durch Injektion eines aus Listeria monocytogenes gewonnenen, löslichen Antigens in die Fußsohle. 10 nicht infizierte Tiere einer 5. Versuchsgruppe (Negativkontrolle) wurden ebenfalls mit dem Antigen behandelt, um unspezifische Reaktionen auf die Antigen-Provokation auszuschließen. 24 Stunden nach der Antigengabe wurde die prozentuale Zunahme des Pfotenvolumens als Maß für die provozierte DTH-Reaktion bestimmt.
Die in Tabelle 12 zusammengefaßten Versuchsergebnisse zeigen, daß die Prüfsubstanz die DTH-Reaktion und damit die zellvermittelte Immunität dosisabhängig und ab Dosen über 1 mg/kg signifikant steigert.

Tabelle 12

| Wirkung auf die DTH-Reaktion gegen Listeria monocytogenes | | |
|---|---|---|
| Versuchskollektiv | Dosis in mg/kg (4 x i.p.) | DTH-Reaktion Zunahme des Pfotenvolumens in % |
| Negativkontrolle<br>Positivkontrolle | | 0.8 ± 1.8<br>9.8 ± 6.4 |
| Verumgruppen (mit Verbindung aus Beispiel 34 behandelt) | 0.1<br>1.0<br>10.0 | 13.3 ± 9.7<br>15.0 ± 7.0<br>19.2 ± 9.4* |

* Signifikanz $p < 0.05$ (Student t-Test)

Mit derselben Versuchsanordnung wurde auch der Einfluß der Prüfsubstanz bei i.p. Applikation von 10 mg/kg auf die DTH-Reaktion von weiblichen NMRI-Mäusen nach Infektion mit unterschiedlichen Mengen des Bakteriums Listeria monocytogenes untersucht. Die Tiere erhielten entweder 2 x $10^2$ oder 5 x $10^2$ Keime. Die Ergebnisse sind in Tagelle 13 dargestellt. Danach wird die DTH-Reaktion zwar bei beiden Keimkonzentrationen durch die Prüfsubstanz erhöht, doch fällt diese Stimulation bei dem mit der niedrigeren Keimzahl infizierten Tierkollektiv deutlich stärker aus.

Tabelle 13

| Wirkung auf die DTH-Reaktion mit Listeria monocytogenes infizierter Mäuse | | |
|---|---|---|
| Applizierte Keimzahl | Versuchskollektiv | DTH-Reaktion Zunahme des Pfotenvolumens in % |
| 2 x $10^2$ | Positivkontrolle<br>Verumgruppe* | 11.3 ± 15.3<br>30.9 ± 15.3 |
| 5 x $10^2$ | Positivkontrolle<br>Verumgruppe* | 12.3 ± 11.5<br>17.9 ± 12.1 |

* 4 x 10 mg/kg i.p. (Verbindung aus Beispiel 34)

8. Wirkung auf die Mortalität und Organbesiedlung bei mit Listeria monocytogenes infizierten Mäusen

Weibliche NMRI-Mäuse (10 Tiere/Gruppe) wurden mit einer niedrigen Dosis Listeria monocytogenes (2 x $10^2$) infiziert. Diese Dosis ist für die Tiere subletal, d. h. sie sterben nicht an der Infektion, entwickeln aber, wie in dem voranstehenden Experiment beschrieben, eine spezifische, zellvermittelte Immunität, die durch die Verbindungen der Formel I verstärkt wird. Es wurde nun untersucht, wie sich dieser Effekt auf den Krankheitsverlauf nach einer 15 Tage nach der Erstinfektion durchgeführten zweiten Belastung mit $10^6$ Keimen des Bakteriums Listeria monocytogenes auswirkt. Wie aus der Tabelle 14 hervorgeht, starben in der Kontrollgruppe 4 der 10 Tiere und alle 6 überlebenden Tiere (100 %) zeigten 5 Tage nach der Zweitinfektion eine Besiedlung der Leber mit Listeria monocytogenes. In der Verumgruppe dagegen, deren Tiere nach dem Behandlungsschema des voranstehend beschriebenen Versuches die Verbindung des Beispiels 34 (4 x 10 mg/kg i.p.) nach der Erstinfektion erhalten hatten, überlebten alle 10 Tiere die Zweitinfektion und lediglich bei 2 von ihnen (20 %) ließen sich Keime in der Leber nachweisen.

Tabelle 14

| Wirkung auf den Krankheitsverlauf der Listeria monocytogenes-Infektion bei Mäusen | | |
|---|---|---|
| Versuchskollektiv | Krankheitsverlauf nach Sekundärinfektion | |
| | Mortalität | Anteil der überlebenden Tiere mit Keimbefall der Leber in % |
| Kontrollgruppe (unbehandelt) | 4/10 | 100 |
| Verumgruppe (Verbindung des Beispiels 34; 4 x 10 mg/kg i.p.) | 0/10 | 20 |

Die Prüfsubstanz vermittelt demzufolge einen deutlichen Schutz vor den fatalen Folgen einer Sekundärinfektion.

9. Wirkung auf die Staphylococcus aureus-Infektion immunsupprimierter Mäuse

Es ist bekannt, daß sich bei Versuchstieren durch Mehrfachgabe eines Zytostatikums eine deutliche Immunsuppression hervorrufen läßt, die sich in einer erhöhten Infektionsanfälligkeit mit drastischem Anstieg der Mortalität äußert.
Es wurde nun untersucht, ob sich die Sterblichkeitsrate durch Behandlung mit den Verbindungen der Formel I nachhaltig senken läßt. Dazu wurden weibliche B6D2F1-Mäuse an drei aufeinander folgenden Tagen mit jeweils 7.5 mg/kg Adriamycin (ADM) intravenös behandelt, am 5. Tag mit 2 x $10^6$ Keimen des Bakteriums Staphylococcus aureus infiziert und die Mortalität bis zum 45. Versuchstag bestimmt (Positivkontrolle). Ein weiteres Kollektiv zwar infizierter, aber nicht durch Vorbehandlung mit ADM immunsupprimierter Tiere wurde als Negativkontrolle mitgeführt. Die immunsupprimierten Tiere der drei Verumgruppen erhielten an 4 aufeinander folgenden Tagen, einen Tag vor der Infektion beginnend, die Prüfsubstanz, wobei jeweils Dosen von 0.1, 1 bzw. 10 mg/kg intraperitoneal verabreicht wurden. Alle Versuchskollektive umfaßten jeweils 20 Tiere.

Die in Tabelle 15 dargestellten Testergebnisse belegen, daß die Mortalität bei den Tieren der Positivkontrolle mit ADM-indduzierter Immunschwäche gegenüber jenen der Negativkontrolle mit intakter Immunabwehr drastisch ansteigt, und daß die Behandlung mit der Prüfsubstanz zu einer deutlichen Senkung der Sterblichkeitsrate bei den immunsupprimierten Tieren führt.

Tabelle 15

| Wirkung auf die Staphylococcus aureus-Infektion immunsupprimierter Mäuse | | |
|---|---|---|
| Versuchskollektiv | Dosis in mg/kg (4 x i.p.) | Mortalität |
| Negativkontrolle | | 0/20 |
| Positivkontrolle | | 13/20 |
| Verumgruppen (mit Verbindung aus Beispiel 34 behandelt) | 0.1 | 5/20 |
| | 1.0 | 8/20 |
| | 10.0 | 8/20 |

10. Wirkung auf die Antikörperantwort der Maus gegen Escherichia(E.) coli-Totkeime und Tetanus-Toxoid

Es wurden Gruppen von jeweils 5 weiblichen NMRI-Mäusen mit einem Körpergewicht von 18 bis 20 g gebildet und den Tieren entweder jeweils $10^8$ durch Hitze abgetötete E. coli-Bakterien intravenös oder jeweils 300 Lf (Limes floculationis) Tetanus-Toxoid subcutan verabreicht. Gleichzeitig mit der Antigengabe erfolgte die orale Verabreichung der Prüfsubstanz in physiologischer Kochsalz-Lösung (PBS) in Dosen von 5, 10, 20, 40 bzw. 80 mg/kg oder des reinen Vehikels (Kontrollgruppen).
Nach 10 und 20 Tagen wurde den Mäusen aus dem retroorbitalen Venengeflecht Blut entnommen und in

den daraus gewonnenen Seren die IgG- und IgM-Antikörper gegen E. coli-Totkeime bzw. Tetanus-Toxoid mit Hilfe der dem Fachmann bekannten ELISA-Technik bestimmt, wobei homologes Lipopolysaccharid von E. coli bzw. Tetanus-Toxoid als Antigen benutzt wurde. Die Höhe der photometrisch gemessenen Extinktionswerte ist ein Maß für die Menge der gebildeten Antikörper.
Die Ergebnisse sind in Tabelle 16 zusammengestellt. Danach ist die Antikörperantwort auf beide Antigene nach oraler Behandlung mit der Prüfsubstanz gegenüber jener der unbehandelten Tiere signifikant erhöht.

Tabelle 16: Stimulation der Antikörperantwort der Maus gegen E. coli-Totkeime und Tetanus-Toxoid

| Test-substanz | Dosis in mg/kg (1 x p.o.) | Antikörperantwort ($mE_{492nm}$-ELISA-Test) gegen E. coli-Totkeime IgM* | IgG** | Tetanus-Toxoid IgG** |
|---|---|---|---|---|
| PBS (Vehikel) | | 925 ± 118 | 1115 ± 141 | 566 ± 270 |
| Verbindung aus Beispiel 34 | 5 | 1217 ± 264 | 1377 ± 324 | 865 ± 182 |
| | 10 | 1411 ± 179 | 1663 ± 191 | 1213 ± 255 |
| | 20 | 1657 ± 231 | 1951 ± 468 | 1852 ± 261 |
| | 40 | 1523 ± 288 | 2434 ± 312 | 1357 ± 348 |
| | 80 | 1459 ± 208 | 2217 ± 273 | 1154 ± 232 |

* 10 Tage-Wert ** 20 Tage-Wert

11. Wirkung auf die chronische Salmonella typhimurium-Infektion an der Maus

Weibliche NMRI-Mäuse (20 Tiere/Gruppe) wurden durch intravenöse Verabreichung von 5 x $10^3$ Salmonella typhimurium-Keimen infiziert. Die Tiere entwickelten daraufhin eine chronische Infektion, die durch persistierende, bakterielle Besiedlung der Organe, wie der Leber und der Milz, mit Nekrosenbildung charakterisiert war. Die Prüfsubstanz wurde in einer Dosis von 5 mg/kg vom 3. bis zum 21. Tage nach der Infektion in zweitägigem Abstand intraperitoneal verabreicht. Die Tiere einer Kontrollgruppe erhielten nur das Vehikel. Am 22. Tag nach der Infektion wurden die Mortalität in beiden Versuchskollektiven bestimmt und die Organe der Tiere, die überlebt hatten, auf Keimbefall und Nekrosenbildung untersucht. Die Daten in Tabelle 17 zeigen, daß die Mortalität, die Anzahl der Tiere mit Keim-positiver Leber und die Häufigkeit von Leber- und Milznekrosen bei den mit der Prüfsubstanz behandelten Tieren gegenüber den unbehandelten Tieren der Kontrollgruppe gesenkt wird.

Tabelle 17

| Wirkung auf den Krankheitsverlauf der chronischen Salmonella typhimurium-Infektion bei Mäusen | | | | | |
|---|---|---|---|---|---|
| Versuchsgruppe | Mortalität | Anteil der überlebenden Tiere in % mit | | | |
| | | Keim-positiver Leber | Lebernekrosen | starken Lebernekrosen | Milznekrosen |
| Kontrolle | 14/20 | 83 | 83 | 67 | 17 |
| Präparatgruppe* | 8/20 | 67 | 58 | 42 | 0 |

* behandelt mit der Verbindung des Beispiels 34 (10 x 5 mg/kg i.p.)

12. Stimulation der Abwehrkraft gegen das B16 Melanom an der Maus

Bei weiblichen C57B1/6-Mäusen mit einem Körpergewicht von 18 bis 20 g wurde mit 2 x $10^5$ lebenden B16 Melanomzellen eine Primärtumor erzeugt, der nach dem Heranwachsen bis auf einen Durchmesser von 0.65 cm operativ entfernt wurde. Die Tiere starben danach an Metastasen in der Lunge. Es wurde nun untersucht, ob sich durch intraperitoneale Behandlung mit den Verbindungen der Formel I die mittlere Überlebenszeit nach Entfernung des Primärtumors, also die Zeit, zu der noch 50 % der Tiere leben, verlängern läßt. Dazu wurden die Mäuse nach Amputation des Primärtumors in Gruppen von jeweils 10 Tieren eingeteilt und ab dem 4. bis zum 100. Tag in zweitägigem Abstand mit der Prüfsubstanz in Dosen von jeweils 1.25 bzw. 2.5 mg/kg i.p. therapiert. Die Tiere der Kontrollgruppe erhielten nach demselben Therapieschema lediglich das reine Vehikel PBS (physiologische Kochsalz-Lösung).
Die Versuchsergebnisse sind in Tabelle 18 wiedergegeben. Danach starben in der Kontrollgruppe 50 % der Tiere nach 22 Tagen, während die mit der Prüfsubstanz behandelten Mäuse eine signifikante Verlängerung der mittleren Überlebenszeit auf 41 bzw. 43 Tage zeigten.

Tabelle 18

| Stimulation der Abwehrkraft gegen das B16 Melanom | | | |
|---|---|---|---|
| Testsubstanz | Dosis mg/kg i.p. | Überlebensrate nach 100 Tagen in % | mittlere Überlebenszeit in Tagen |
| PBS (Vehikel) | | 0 | 22 |
| Verbindung des Beispiels 34 | 1.25<br>2.50 | 20<br>30 | 41<br>43 |

**Patentansprüche**
**Patentansprüche für folgende Vertragsstaaten : AT, BE, CH, DE, FR, GB, IT, LI, LU, NL, SE**

**1.** Arzneimittel, gekennzeichnet durch einen Gehalt an - oder bestehend aus - mindestens einer Verbindung der Formel I und/oder gegebenenfalls einem von deren physiologisch verträglichen Salzen, wobei

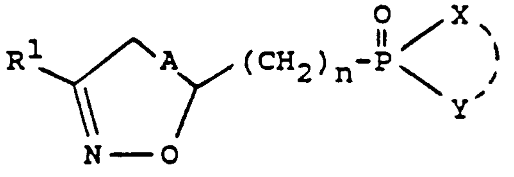

(I)

$R^1$ für

a) eine geradkettige oder verzweigte Alkyl- oder Alkenylgruppen mit 1 bis 6 C-Atomen, deren Kohlenstoffkette mit Halogen, Hydroxy, $(C_1$-$C_4)$Alkoxy, $(C_1$-$C_4)$Acyloxy oder gegebenenfalls mit $(C_1$-

$C_4$)Alkoxy oder Halogen substituiertem Aryl substituiert sein kann, oder
b) eine ein- oder zweikernige aromatische oder heteroaromatische Gruppe mit 1 bis 2 Stickstoffatomen und/oder einem Schwefel- oder Sauerstoffatom im Ringsystem, wobei diese Gruppe ein- oder mehrfach und gleich oder verschieden mit geradkettigem oder verzweigtem ($C_1$-$C_4$)Alkyl, ($C_3$-$C_6$)-Cycloalkyl, Hydroxy, ($C_1$-$C_3$)Alkoxy, Aryloxy, ($C_1$-$C_4$)Acyloxy oder Benzoyloxy, Halogen, Trifluormethyl, Nitro, gegebenenfalls mono- oder disubstituiertem Amino, ($C_1$-$C_4$)Alkoxycarbonyl, Carboxy, Carbamoyl, ($C_1$-$C_4$)Alkylcarbonyl, deren Carbonylgruppe auch in ketalisierter Form vorliegen kann, oder gegebenenfalls mit ($C_1$-$C_4$)Alkyl, Halogen, oder ($C_1$-$C_3$)Alkoxy ringsubstituiertem Phenyl oder Benzyl substituiert sein kann, oder
c) Carboxy oder Alkoxycarbonyl mit 1 bis 4 C-Atomen im Alkylteil oder
d) Arylcarbonyl, das gegebenenfalls im Arylteil mit ($C_1$-$C_4$)Alkyl, Halogen oder ($C_1$-$C_3$)Alkoxy substituiert ist, oder
e) Halogen steht,
A eine C,C-Einfach- oder eine C,C-Doppelbindung,
n eine ganze Zahl von 0 bis 2 und
X und Y, die gleich oder verschieden sein können, jeweils unabhängig voneinander eine geradkettige oder verzweigte ($C_1$-$C_4$)Alkylgruppe, den Rest -$OR^2$ oder die Gruppe -$NR^2R^3$ bedeuten, wobei $R^2$ und $R^3$ für Wasserstoff oder gegebenenfalls substituierte ($C_1$-$C_6$)Alkylreste stehen, die in der Gruppe -$NR^2R^3$ zusammen mit dem Stickstoffatom auch einen fünf- bis siebengliedrigen Ring oder im Strukturelement -P(O)($OR^2$)$_2$ zusammen mit dem Phosphoratom einen gegebenenfalls noch mit ($C_1$-$C_3$)Alkyl, ($C_1$-$C_4$)Alkoxycarbonyl oder Carboxy substituierten Heterocyclus der Formel

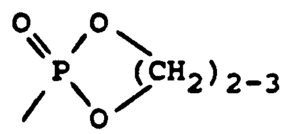

bilden können,
und wobei die Verbindungen der Formel I gegebenenfalls als reine Stereoisomere oder als deren Gemische vorliegen können.

**2.** Arzneimittel nach Anspruch 1, gekennzeichnet durch einen Gehalt an - oder bestehend aus - mindestens einer Verbindung der Formel I und/oder gegebenenfalls einem von deren Salzen, bei denen $R^1$ für
a) gegebenenfalls verzweigtes ($C_1$-$C_4$)Alkyl oder ($C_1$-$C_4$)Hydroxyalkyl oder Phenyl($C_1$-$C_2$)alkyl oder Phenyl($C_1$-$C_3$)alkenyl,
b) unsubstituiertes oder ein- oder mehrfach mit ($C_1$-$C_4$)Alkyl, Hydroxy, ($C_1$-$C_2$)Alkoxy, Phenoxy, Halogen, Trifluormethyl, Nitro, Di($C_1$-$C_2$)alkylamino, ($C_1$-$C_2$)Alkoxycarbonyl, Carboxy oder Phenyl substituiertes Phenyl, Naphthyl, Pyridyl oder Thienyl,
c) Carboxy, Meth- oder Ethoxycarbonyl,
d) Benzoyl,
e) Chlor oder Brom steht,
A eine C,C-Einfach- oder eine C,C-Doppelbindung bedeutet,
n für 0 oder 1 steht und
X und Y, die gleich oder verschieden sein können, unabhängig voneinander eine Methyl- oder Ethylgruppe oder die Reste -$OR^2$ oder -$NR^2R^3$ darstellen, wobei $R^2$ für Wasserstoff, Methyl oder Ethyl und $R^3$ ebenfalls für Wasserstoff, Methyl oder Ethyl oder aber für das Kohlenstoffgerüst einer gegebenenfalls carboxygeschützten Aminosäure stehen, die Reste $R^2$ und $R^3$ in der Gruppe -$NR^2R^3$ zusammen mit dem Stickstoffatom auch einen Pyrrolidin-, Piperidin- oder Morpholinring und die Reste -$OR^2$ im Strukturelement -P(O)($OR^2$)$_2$ zusammen mit dem Phosphoratom einen gegebenenfalls mit ($C_1$-$C_2$)Alkyl substituierten 2-Oxo-1,3,2-dioxaphospholan- oder 2-Oxo-1,3,2-dioxaphosphiran-Ring bilden können,
und wobei diese Verbindungen gegebenenfalls als reine Stereoisomere oder als deren Gemische vorliegen können.

**3.** Arzneimittel nach Anspruch 2, gekennzeichnet durch einen Gehalt an - oder bestehend aus - mindestens einer Verbindung der Formel I und/oder gegebenenfalls einem von deren Salzen, bei denen

entweder
$R^1$ für tert.Butyl, Benzyl, Phenyl, Naphthyl, Pyridyl, Thienyl oder mit Methyl, Hydroxy, Methoxy, Phenoxy, Chlor, Fluor, Trifluormethyl, Nitro, Dimethylamino, Methoxycarbonyl oder Carboxy substituiertes Phenyl steht oder
X und Y unabhängig voneinander Hydroxy, Methoxy oder Ethoxy bzw.
X Methyl und Y Hydroxy, Methoxy oder Ethoxy bedeuten, und wobei diese Verbindungen gegebenenfalls als reine Stereoisomere oder als deren Gemische vorliegen können.

**4.** Arzneimittel nach Anspruch 3, gekennzeichnet durch einen Gehalt an - oder bestehend aus - mindestens einer Verbindung der Formel I und/oder gegebenenfalls einem von deren Salzen, bei denen $R^1$, X und Y gleichzeitig
die im Anspruch 3 definierten Bedeutungen haben, und wobei diese Verbindungen gegebenenfalls als reine Stereoisomere oder als deren Gemische vorliegen können.

**5.** Arzneimittel nach Anspruch 4, gekennzeichnet durch einen Gehalt an - oder bestehend aus - mindestens einer Verbindung der Formel I und/oder gegebenenfalls eine von deren Salzen, bei denen A für eine C,C-Einfachbindung steht und n den Wert 0 hat, wobei diese Verbindungen als reine Stereoisomere oder als deren Gemische vorliegen können.

**6.** Arzneimittel nach Anspruch 5, gekennzeichnet durch einen Gehalt an - oder bestehend aus - mindestens einer Verbindung der Formel I und/oder einem von deren Salzen, bei denen $R^1$ für tert.Butyl oder Phenyl steht, X und Y jeweils Hydroxy oder X Methyl und Y Hydroxy bedeuten, wobei diese Verbindungen als reine Stereoisomere oder als deren Gemische vorliegen können.

**7.** Arzneimittel nach Anspruch 6, gekennzeichnet durch einen Gehalt an - oder bestehend aus - 3-Phenyl-2-isoxazolin-5-yl-phosphonsäure und/oder 3-Phenyl(oder 3-tert.Butyl)-2-isoxazolin-5-yl-(P-methyl)-phosphinsäure und/oder mindestens einem von deren Salzen, wobei diese Verbindungen als reine Stereoisomere oder als deren Gemische vorliegen können.

**8.** Arzneimittel nach Ansprüchen 1 bis 7, dadurch gekennzeichnet, daß sie zur Prophylaxe und/oder Behandlung von Erkrankungen des Immunsystems bestimmt sind.

**9.** Arzneimittel nach Anspruch 8, dadurch gekennzeichnet, daß sie zur Prophylaxe und/oder Behandlung von Tumoren, Infektionen und/oder Autoimmunerkrankungen und für die Adjuvierung von Impfstoffen verwendet werden.

**10.** Verfahren zur Herstellung eines Arzneimittels gemäß den Ansprüchen 1 bis 9, dadurch gekennzeichnet, daß man mindestens eine Verbindung der Formel I, gegebenenfalls in stereoisomerenreiner Form und/oder als physiologisch verträgliches Salz, mit einem physiologisch annehmbaren Träger und gegebenenfalls weiteren Zusatz- und/oder Hilfsstoffen in eine geeignete pharmazeutische Darreichungsform bringt.

**11.** Verbindungen der Formel I, ihre gegebenenfalls stereoisomeren Formen und gegebenenfalls ihre physiologisch verträglichen Salze, dadurch gekennzeichnet, daß $R^1$, A, n, X und Y die in Anspruch 1 definierten Bedeutungen haben, wobei die Verbindungen 3-Phenyl-2-isoxazolin-5-yl-phosphonsäure, 3-Methyl( und Phenyl)-2-isoxazolin-5-yl-phosphonsäuredimethylester, 3-(3-Nitrophenyl)-2-isoxazolin-5-yl-phosphonsäuredipropylester, 3-(2-Nitro-5-(2-chlor-4-trifluormethyl-phenoxy)-phenyl)-2-isoxazolin-5-yl-phosphonsäurediethylester, 3-Methyl(und Phenyl)-2-isoxazolin-5-yl-phosphonsäuretetramethyldiamid,3-Phenyl-2-isoxazolin-5-yl-methyl-phosphonsäure und deren Diethylester, 3-Methyl(Ethyl, Isopropyl, tert.Butyl, Methoxymethyl, Phenyl und Ethoxycarbonyl)-isoxazol-5-yl-methyl-phosphonsäurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegebenenfalls racemischen Formen handelt, ausgenommen sind.

**12.** Verbindungen nach Anspruch 11, ihre gegebenenfalls stereoisomeren Formen und gegebenenfalls ihre Salze, dadurch gekennzeichnet, daß $R^1$, A, n, X und Y die in Anspruch 2 definierten Reste darstellen, wobei die Verbindungen 3-Phenyl-2-isoxazolin-5-yl-phosphonsäure, 3-Methyl(und Phenyl)-2-isoxazolin-5-yl-phosphonsäuredimethylester, 3-Methyl(und Phenyl)-2-isoxazolin-5-yl-phosphonsäuretetramethyldiamid,3-Phenyl-2-isoxazolin-5-yl-methyl-phosphonsäure und deren Diethylester, 3-Methyl(Ethyl, Isopropyl, tert.Butyl, Phenyl und Ethoxycarbonyl)-isoxazol-5-yl-methyl-phosphon-säurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegebenenfalls racemischen Formen handelt, ausgenommen sind.

**13.** Verbindungen nach Anspruch 12, ihre gegebenenfalls stereoisomeren Formen und gegebenenfalls ihre Salze, dadurch gekennzeichnet, daß $R^1$, X und Y die in Anspruch 3 definierten Reste darstellen und A und n die in Anspruch 2 genannten Bedeutungen haben, wobei die Verbindungen 3-Phenyl-2-isoxazolin-5-yl-phosphonsäure, 3-Methyl(und Phenyl)-2-isoxazolin-5-yl-phosphonsäuredimethylester, 3-Phenyl-2-isoxazolin-5-yl-phosphonsäuretetramethyldiamid, 3-Phenyl-2-isoxazolin-5-yl-methyl-phosphonsäure und deren Diethylester, 3-Methyl(Ethyl, Isopropyl, tert.Butyl, Phenyl und Ethoxycarbonyl)-isoxazol-5-yl-methyl-phosphonsäurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegebenenfalls racemischen Formen handelt, ausgenommen sind.

**14.** Verbindungen nach Anspruch 13, ihre gegebenenfalls stereoisomeren Formen und gegebenenfalls ihre Salze, dadurch gekennzeichnet, daß $R^1$, X und Y die in Anspruch 4 definierten Reste darstellen und A und n die in Anspruch 2 genannten Bedeutungen haben, wobei die Verbindungen 3-Phenyl-2-isoxazolin-5-yl-phosphonsäure und deren Dimethylester, 3-Phenyl-2-isoxazolin-5-yl-methyl-phosphonsäure und deren Diethylester, 3-tert.Butyl(und Phenyl)-isoxazol-5-yl-methyl-phosphonsäurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegebenenfalls racemischen Formen handelt, ausgenommen sind.

**15.** Verbindungen nach Anspruch 14, ihre stereoisomeren Formen und gegebenenfalls ihre Salze, dadurch gekennzeichnet, daß $R^1$, X und Y die in Anspruch 4 definierten Reste darstellen und A und n die in Anspruch 5 genannten Bedeutungen haben, wobei die Verbindungen 3-Phenyl-2-isoxazolin-5-yl-phosphonsäure und deren Dimethylester, sofern es sich um deren racemischen Formen handelt, ausgenommen sind.

**16.** Verbindungen nach Anspruch 15, ihre stereoisomeren Formen und ihre Salze, dadurch gekennzeichnet, daß $R^1$, X und Y die in Anspruch 6 definierten Reste darstellen und A und n die in Anspruch 5 genannten Bedeutungen haben, wobei die racemische 3-Phenyl-2-isoxazolin-5-yl-phosphonsäure ausgenommen ist.

**17.** Verbindungen nach Anspruch 16, ihre stereoisomeren Formen und ihre Salze, dadurch gekennzeichnet, daß sie 3-Phenyl- oder 3-tert.Butyl-2-isoxazolin-5-yl-(P-methyl)-phosphinsäure darstellen.

**18.** Verbindungen nach Anspruch 16 und ihre Salze, dadurch gekennzeichnet, daß sie die enantiomeren Formen der 3-Phenyl-2-isoxazolin-5-yl-phosphonsäure darstellen.

**19.** Verfahren zur Herstellung von phosphorhaltigen 2-Isoxazolinen und Isoxazolen der allgemeinen Formel I gemäß den Ansprüchen 11 bis 18, gegebenenfalls stereoisomeren Formen und gegebenenfalls ihrer physiologisch verträglichen Salze, dadurch gekennzeichnet, daß man ein Nitriloxid der Formel II

$R^1$-C≡N→O (II)

a) für den Fall, daß A in Formel I eine C,C-Einfachbindung bedeutet, mit einer olefinischen Phosphorverbindung der Formel III

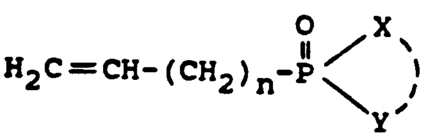

(III)

umsetzt oder
b) für den Fall, daß A in Formel I eine C,C-Doppelbindung bedeutet, mit einer olefinischen Phosphorverbindung der Formel IV

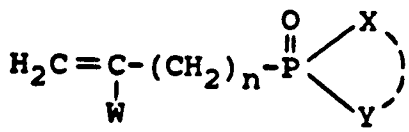
(IV)

zu einem 2-Isoxazolin der Formel V

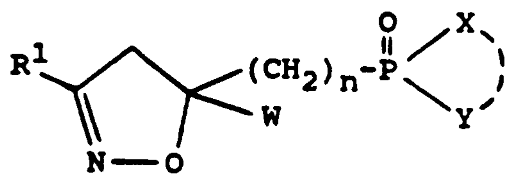
(V)

umsetzt und aus diesem Zwischenprodukt unter basischen Bedingungen oder thermischer Belastung HW eliminiert,wobei in den Formeln II bis V $R^1$, n, X und Y die vorgenannten Bedeutungen haben und W für eine Abgangsgruppe, insbesondere Halogen, steht, und gegebenenfalls
c) einen nach a) oder b) erhaltenen Phosphon- oder Phosphinsäureester der Formel I zum Phosphonsäurehalbester oder zur Phosphon- bzw. Phosphinsäure der Formel I spaltet oder
d) einen nach a) oder b) gewonnenen Phosphonsäuredialkylester der Formel I mit einem Amin der Formel $HNR^2R^3$ (VI) unter Austausch einer der beiden phosphorständigen Alkoxygruppen gegen den Rest $-NR^2R^3$ zu einem Halbesterhalbamid der Formel I umsetzt, wobei $R^2$ und $R^3$ die vorgenannten Bedeutungen haben und Verbindungen, in denen $R^1$ Halogen bedeutet, ausgenommen sind, oder
e) eine nach a), b) oder c) hergestellte Phosphonsäure der Formel I zunächst in ein am Phosphoratom aktiviertes Säurederivat überführt und dieses anschließend mit Alkoholen der Formel $R^2OH$ (VII), oder einem Diol der Formel $HO-(CH_2)_{2-3}-OH$ (VIII) und/oder Aminen der Formel VI wahlweise zu einem Mono- oder gegebenenfalls gemischten Diester, einem cyclischen Ester, einem Halbesterhalbamid oder einem Mono- oder gegebenenfalls gemischten Diamid der Formel I umsetzt oder
einen nach a), b) oder c) erhaltenen Phosphonsäurehalbester der Formel I nach Aktivierung am Phosphoratom mit einem Alkohol VII oder einem Amin VI zu einem gegebenenfalls gemischten Diester bzw. einem Halbesterhalbamid der Formel I umsetzt oder
eine nach a), b) oder c) hergestellte Phosphinsäure der Formel I nach Aktivierung am Phosphoratom mit einem Alkohol VII oder einem Amin VI zu einem Ester bzw. Amid der Formel I umsetzt,
wobei $R^2$ und $R^3$ in Formel VI die vorgenannten Bedeutungen haben, $R^2$ in Formel VII für gegebenenfalls substituiertes $(C_1-C_6)$Alkyl steht und die Alkylenkette $-(CH_2)_{2-3}-$ in Formel VIII noch mit $(C_1-C_3)$Alkyl, $(C_1-C_4)$Alkoxycarbonyl oder Carboxy substituiert sein kann, oder
f) einen nach a) hergestellten 3-Chlor(oder Brom)-2-isoxazolin-phosphonsäuredi- oder monoester oder -phosphinsäureester der Formel I, in der n eine ganze Zahl von 0 bis 2 bedeutet, mit $Tri(C_1-C_4)$alkylhalogensilanen unter Esterspaltung und gleichzeitigem Austausch von Chlor bzw. Brom gegen das Halogenatom des jeweils eingesetzten Silans zu den entsprechenden 3-Halogen-2-isoxazolinphosphon- oder -phosphinsäuren der Formel I umsetzt oder
g) eine nach a) bis f) erhaltene Verbindung der Formel I, die aufgrund ihrer chemischen Struktur in diastereomeren oder enantiomeren Formen auftritt, in die reinen Stereoisomeren spaltet,
wobei man die nach a) bis g) hergestellten Verbindungen der Formel I entweder in freier Form isoliert oder gegebenenfalls in physiologisch verträgliche Salze umwandelt.

**20.** Verbindungen der Formel I, ihre gegebenenfalls stereoisomeren Formen und/oder gegebenenfalls ihre physiologisch verträglichen Salze zur Anwendung in einem Verfahren zur prophylaktischen und/oder therapeutischen Behandlung des menschlichen oder tierischen Körpers.

**21.** Ausführungsform gemäß Anspruch 20 zur prophylaktischen und/oder therpateutischen Behandlung von Erkrankungen des Immunsystems des menschlichen oder tierischen Körpers.

**22.** Ausführungsform gemäß Anspruch 20 zur prophylaktischen und/oder therapeutischen Behandlung von Tumoren, Infektionen und/oder Antiimmunerkrankungen des menschlichen oder tierischen Körpers sowie für die Adjuvierung von Impfstoffen.

**23.** Verwendung von Verbindungen der Formel I, ihrer gegebenenfalls stereoisomeren Formen und/oder gegebenenfalls ihrer physiologisch verträglichen Salze zur Herstellung von Arzneimitteln gemäß den Ansprüchen 1 bis 7.

**24.** Verwendung von Verbindungen der Formel I, ihrer gegebenenfalls stereoisomeren Formen und/oder gegebenenfalls ihrer physiologisch verträglichen Salze zur Herstellung von Arzneimitteln gemäß Anspruch 23, die zur Prophylaxe und/oder Behandlung von Erkrankungen des Immunsystems bestimmt sind.

**25.** Verwendung der Verbindungen der Formel I, ihrer gegebenenfalls stereoisomeren Formen und/oder gegebenenfalls ihrer physiologisch verträglichen Salze zur Herstellung von Arzneimitteln gemäß Anspruch 24, die zur Prophylaxe und/oder Behandlung von Tumoren, Infektionen und/oder Autoimmunerkrankungen und für die Adjuvierung von Impfstoffen bestimmt sind.

**Patentansprüche für folgende Vertragsstaaten : ES, GR**

**1.** Verfahren zur Herstellung eines Arzneimittels, dadurch gekennzeichnet, daß man mindestens eine Verbindung der nachstehenden Formel I, gegebenenfalls in stereoisomerenreiner Form und/oder als physiologisch verträgliches Salz, mit einem physiologisch annehmbaren Träger und gegebenenfalls weiteren Zusatz- und/oder Hilfsstoffen in eine geeignete pharmazeutische Darreichungsform bringt:

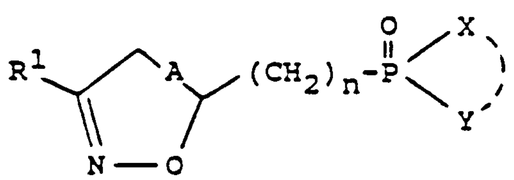

(I)

worin $R^1$ für

a) eine geradkettige oder verzweigte Alkyl- oder Alkenylgruppe mit 1 bis 6 C-Atomen, deren Kohlenstoffkette mit Halogen, Hydroxy, $(C_1\text{-}C_4)$Alkoxy, $(C_1\text{-}C_4)$Acyloxy oder gegebenenfalls mit $(C_1\text{-}C_4)$Alkoxy oder Halogen substituiertem Aryl substituiert sein kann, oder

b) eine ein- oder zweikernige aromatische oder heteroaromatische Gruppe mit 1 bis 2 Stickstoffatomen und/oder einem Schwefel- oder Sauerstoffatom im Ringsystem, wobei diese Gruppe ein- oder mehrfach und gleich oder verschieden mit geradkettigem oder verzweigtem $(C_1\text{-}C_4)$Alkyl, $(C_3\text{-}C_6)$-Cycloalkyl, Hydroxy, $(C_1\text{-}C_3)$Alkoxy, Aryloxy, $(C_1\text{-}C_4)$ Acyloxy oder Benzoyloxy, Halogen, Trifluormethyl, Nitro, gegebenenfalls mono- oder disubstituiertem Amino, $(C_1\text{-}C_4)$Alkoxycarbonyl, Carboxy, Carbamoyl, $(C_1\text{-}C_4)$Alkylcarbonyl, deren Carbonylgruppe auch in ketalisierter Form vorliegen kann, oder gegebenenfalls mit $(C_1\text{-}C_4)$Alkyl, Halogen, oder $(C_1\text{-}C_3)$Alkoxy ringsubstituiertem Phenyl oder Benzyl substituiert sein kann, oder

c) Carboxy oder Alkoxycarbonyl mit 1 bis 4 C-Atomen im Alkylteil oder

d) Arylcarbonyl, das gegebenenfalls im Arylteil mit $(C_1\text{-}C_4)$Alkyl, Halogen oder $(C_1\text{-}C_3)$Alkoxy substituiert ist, oder

e) Halogen steht,

A eine C,C-Einfach- oder eine C,C-Doppelbindung,

n eine ganze Zahl von 0 bis 2 und

X und Y, die gleich oder verschieden sein können, jeweils unabhängig voneinander eine geradkettige oder verzweigte $(C_1\text{-}C_4)$Alkylgruppe, den Rest $-OR^2$ oder die Gruppe $-NR^2R^3$ bedeuten, wobei $R^2$ und $R^3$ für Wasserstoff oder gegebenenfalls substituierte $(C_1\text{-}C_6)$Alkylreste stehen, die in der Gruppe $-NR^2R^3$ zusammen mit dem Stickstoffatom auch einen fünf- bis siebengliedrigen Ring oder im Strukturelement $-P(O)(OR^2)_2$ zusammen mit dem Phosphoratom einen gegebenenfalls noch mit $(C_1$-

$C_3$)Alkyl, ($C_1$-$C_4$)Alkoxycarbonyl oder Carboxy substituierten Heterocyclus der Formel

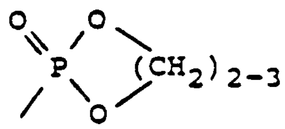

bilden können.

**2.** Verfahren nach Anspruch 1, dadurch gekennzeichnet, daß in Formel I
$R^1$ für
a) gegebenenfalls verzweigtes ($C_1$-$C_4$)Alkyl oder ($C_1$-$C_4$)Hydroxyalkyl oder Phenyl($C_1$-$C_2$)alkyl oder Phenyl($C_1$-$C_3$)alkenyl,
b) unsubstituiertes oder ein- oder mehrfach mit ($C_1$-$C_4$)Alkyl, Hydroxy, ($C_1$-$C_2$)Alkoxy, Phenoxy, Halogen, Trifluormethyl, Nitro, Di($C_1$-$C_2$)alkylamino, ($C_1$-$C_2$)Alkoxycarbonyl, Carboxy oder Phenyl substituiertes Phenyl, Naphthyl, Pyridyl oder Thienyl,
c) Carboxy, Meth- oder Ethoxycarbonyl,
d) Benzoyl,
e) Chlor oder Brom steht,
A eine C,C-Einfach- oder eine C,C-Doppelbindung bedeutet,
n für 0 oder 1 steht und
X und Y, die gleich oder verschieden sein können, unabhängig voneinander eine Methyl- oder Ethylgruppe oder die Reste $-OR^2$ oder $-NR^2R^3$ darstellen, wobei $R^2$ für Wasserstoff, Methyl oder Ethyl und $R^3$ ebenfalls für Wasserstoff, Methyl oder Ethyl oder aber für das Kohlenstoffgerüst einer gegebenenfalls carboxygeschützten Aminosäure stehen, die Reste $R^2$ und $R^3$ in der Gruppe $-NR^2R^3$ zusammen mit dem Stickstoffatom auch einen Pyrrolidin-, Piperidin- oder Morpholinring und die Reste $-OR^2$ im Strukturelement $-P(O)(OR^2)_2$ zusammen mit dem Phosphoratom einen gegebenenfalls mit ($C_1$-$C_2$)Alkyl substituierten 2-Oxo-1,3,2-dioxaphospholan- oder 2-Oxo-1,3,2-dioxaphosphiran-Ring bilden können.

**3.** Verfahren nach Anspruch 2, dadurch gekennzeichnet, daß in Formel I entweder
$R^1$ für tert.Butyl, Benzyl, Phenyl, Naphthyl, Pyridyl, Thienyl oder mit Methyl, Hydroxy, Methoxy, Phenoxy, Chlor, Fluor, Trifluormethyl, Nitro, Dimethylamino, Methoxycarbonyl oder Carboxy substituiertes Phenyl steht oder
X und Y unabhängig voneinander Hydroxy, Methoxy oder Ethoxy bzw.
X Methyl und Y Hydroxy, Methoxy oder Ethoxy bedeuten.

**4.** Verfahren nach Anspruch 3, dadurch gekennzeichnet, daß in Formel I $R^1$, X und Y gleichzeitig die im Anspruch 3 definierten Bedeutungen haben.

**5.** Verfahren nach Anspruch 4, dadurch gekennzeichnet, daß in Formel I A für eine C,C-Einfachbindung steht und n den Wert 0 hat.

**6.** Verfahren nach Anspruch 5, dadurch gekennzeichnet, daß in Formel I $R^1$ für tert.Butyl oder Phenyl steht, X und Y jeweils Hydroxy oder X Methyl und Y Hydroxy bedeuten.

**7.** Verfahren nach Anspruch 6, dadurch gekennzeichnet, daß die Verbindung(en) der Formel I 3-Phenyl-2-isoxazolin-5-yl-phosphonsäure und/oder 3-Phenyl (oder 3-tert.Butyl)-2-isoxazolin-5-yl-(P-methyl)-phosphinsäure ist (sind).

**8.** Verfahren nach einem oder mehreren der Ansprüche 1 bis 7, dadurch gekennzeichnet, daß die verfahrensgemäß hergestellten Arzneimittel zur Prophylaxe und/oder Behandlung von Erkrankungen des Immunsystems bestimmt sind.

**9.** Verfahren nach Anspruch 8, dadurch gekennzeichnet, daß die verfahrensgemäß hergestellten Arzneimittel zur Prophylaxe und/oder Behandlung von Tumoren, Infektionen und/oder Autoimmunerkrankungen und für die Adjuvierung von Impfstoffen verwendet werden.

**10.** Verfahren zur Herstellung von phosphorhaltigen 2-Isoxazolinen und Isoxazolen der allgemeinen Formel I gemäß der Definition in Anspruch 1, ihrer gegebenenfalls stereoisomeren Formen und gegebenenfalls ihrer physiologisch verträglichen Salze wobei die Verbindungen 3-Phenyl-2-isoxazolin-5-yl-phosphonsäure, 3-Methyl( und Phenyl)-2-isoxazolin-5-yl-phosphonsäuredimethylester, 3-(3-Nitrophenyl)-2-isoxazolin-5-yl-phosphonsäuredipropylester, 3-(2-Nitro-5-(2-chlor-4-trifluormethyl-phenoxy)-phenyl)-2-isoxazolin-5-yl-phosphonsäurediethylester, 3-Methyl(und Phenyl)-2-isoxazolin-5-yl-phosphonsäuretetramethyldiamid,3-Phenyl-2-isoxazolin-5-yl-methyl-phosphonsäure und deren Diethylester, 3-Methyl(Ethyl, Isopropyl, tert.Butyl, Methoxymethyl, Phenyl und Ethoxycarbonyl)-isoxazol-5-yl-methyl-phosphonsäurediethylester, 3-(4-Fluor- und 4-Chlorphenyl)-isoxazol-5-yl-(P-methyl-phosphinsäure und 3-Phenyl-isoxazol-5-yl-(P-methyl)-phosphinsäuremethylester, sofern es sich um deren gegebenenfalls racemischen Formen handelt, ausgenommen sind,
dadurch gekennzeichnet, daß man ein Nitriloxid der Formel II

$R^1$-C≡N→O (II)

a) für den Fall, daß A in Formel I eine C,C-Einfachbindung bedeutet, mit einer olefinischen Phosphorverbindung der Formel III

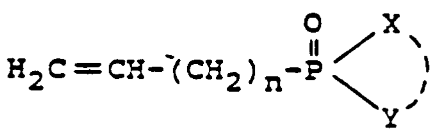

(III)

umsetzt oder
b) für den Fall, daß A in Formel I eine C,C-Doppelbindung bedeutet, mit einer olefinischen Phosphorverbindung der Formel IV

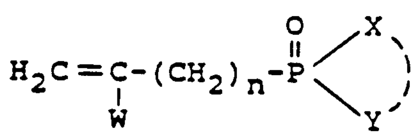

(IV)

zu einem 2-Isoxazolin der Formel V

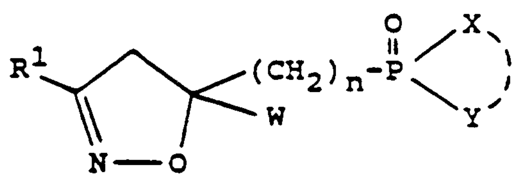

(V)

umsetzt und aus diesem Zwischenprodukt unter basischen Bedingungen oder thermischer Belastung HW eliminiert,wobei in den Formeln II bis V $R^1$, n, X und Y die vorgenannten Bedeutungen haben und W für eine Abgangsgruppe, insbesondere Halogen, steht, und gegebenenfalls
c) einen nach a) oder b) erhaltenen Phosphon- oder Phosphinsäureester der Formel I zum Phosphonsäurehalbester oder zur Phosphon- bzw. Phosphinsäure der Formel I spaltet oder
d) einen nach a) oder b) gewonnenen Phosphonsäuredialkylester der Formel I mit einem Amin der Formel $HNR^2R^3$ (VI) unter Austausch einer der beiden phosphorständigen Alkoxygruppen gegen den Rest $-NR^2R^3$ zu einem Halbesterhalbamid der Formel I umsetzt, wobei $R^2$ und $R^3$ die vorgenannten Bedeutungen haben und Verbindungen, in denen $R^1$ Halogen bedeutet, ausgenommen sind, oder
e) eine nach a), b) oder c) hergestellte Phosphonsäure der Formel I zunächst in ein am Phosphoratom aktiviertes Säurederivat überführt und dieses anschließend mit Alkoholen der Formel $R^2OH$ (VII), oder einem Diol der Formel $HO-(CH_2)_{2-3}-OH$ (VIII) und/oder Aminen der Formel VI wahlweise zu einem Mono- oder gegebenenfalls gemischten Diester, einem cyclischen Ester, einem Halbesterhalbamid oder einem Mono- oder gegebenenfalls gemischten Diamid der Formel I umsetzt oder einen nach a), b) oder c) erhaltenen Phosphonsäurehalbester der Formel I nach Aktivierung am Phosphoratom mit einem Alkohol VII oder einem Amin VI zu einem gegebenenfalls gemischten Diester bzw. einem Halbesterhalbamid der Formel I umsetzt oder
eine nach a), b) oder c) hergestellte Phosphinsäure der Formel I nach Aktivierung am Phosphoratom mit einem Alkohol VII oder einem Amin VI zu einem Ester bzw. Amid der Formel I umsetzt,
wobei $R^2$ und $R^3$ in Formel VI die vorgenannten Bedeutungen haben, $R^2$ in Formel VII für gegebenenfalls substituiertes $(C_1\text{-}C_6)$Alkyl steht und die Alkylenkette $-(CH_2)_{2-3}-$ in Formel VIII noch mit $(C_1\text{-}C_3)$Alkyl, $(C_1\text{-}C_4)$Alkoxycarbonyl oder Carboxy substituiert sein kann, oder
f) einen nach a) hergestellten 3-Chlor(oder Brom)-2-isoxazolin-phosphonsäuredi- oder monoester oder -phosphinsäureester der Formel I, in der n eine ganze Zahl von 0 bis 2 bedeutet, mit Tri$(C_1\text{-}C_4)$alkylhalogensilanen unter Esterspaltung und gleichzeitigem Austausch von Chlor bzw. Brom gegen das Halogenatom des jeweils eingesetzten Silans zu den entsprechenden 3-Halogen-2-isoxazolinphosphon- oder -phosphinsäuren der Formel I umsetzt oder
g) eine nach a) bis f) erhaltene Verbindung der Formel I, die aufgrund ihrer chemischen Struktur in diastereomeren oder enantiomeren Formen auftritt, in die reinen Stereoisomeren spaltet,
wobei man die nach a) bis g) hergestellten Verbindungen der Formel I entweder in freier Form isoliert oder gegebenenfalls in physiologisch verträgliche Salze umwandelt.

**11.** Verfahren nach Anspruch 10, dadurch gekennzeichnet, daß man Verbindungen der Formel I herstellt, in welcher $R^1$, A, n, X und Y die in Anspruch 2 definierten Reste darstellen, wobei die Verbindungen 3-Phenyl-2-isoxazolin-5-yl-phosphonsäure, 3-Methyl(und Phenyl)-2-isoxazolin-5-yl-phosphonsäuredimethylester, 3-Methyl(und Phenyl)-2-isoxazolin-5-yl-phosphonsäuretetramethyldiamid,3-Phenyl-2-isoxazolin-5-yl-methyl-phosphonsäure und deren Diethylester, 3-Methyl(Ethyl, Isoprospyl, tert.Butyl, Phenyl und Ethoxycarbonyl)-isoxazol-5-yl-methyl-phosphon-säurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegebenenfalls racemischen Formen handelt, ausgenommen sind.

**12.** Verfahren nach Anspruch 11, dadurch gekennzeichnet, daß man Verbindungen der Formel I herstellt, in welcher $R^1$, X und Y die in Anspruch 3 definierten Reste darstellen und A und n die in Anspruch 2 genannten Bedeutungen haben, wobei die Verbindungen 3-Phenyl-2-isoxazolin-5-yl-phosphonsäure, 3-Methyl (und Phenyl)-2-isoxazolin-5-yl-phosphonsäuredimethylester, 3-Phenyl-2-isoxazolin-5-yl-phosphonsäuretetramethyldiamid, 3-Phenyl-2-isoxazolin-5-yl-methyl-phosphonsäure und deren Diethylester, 3-Methyl(Ethyl, Isopropyl, tert.Butyl, Phenyl und Ethoxycarbonyl)-isoxazol-5-yl-methyl-phosphonsäurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegebenenfalls reacemischen Formen handelt, ausgenommen sind.

**13.** Verfahren nach Anspruch 12, dadurch gekennzeichnet, daß man Verbindungen der Formel I herstellt, in welcher $R^1$, X und Y die in Anspruch 4 definierten Reste darstellen und A und n die in Anspruch 2 genannten Bedeutungen haben, wobei die Verbindungen 3-Phenyl-2-isoxazolin-5-yl-phosphonsäure und deren Dimethylester, 3-Phenyl-2-isoxazolin-5-yl-methyl-phosphonsäure und deren Diethylester, 3-tert.Butyl(und Phenyl)-isoxazol-5-yl-methyl-phosphonsäurediethylester, 3-(4-Fluor- und 4-Chlorphenyl)-isoxazol-5-yl-(P-methyl)-phosphinsäure und 3-Phenyl-isoxazol-5-yl-(P-methyl)-phosphinsäuremethylester, sofern es sich um deren gegebenenfalls racemischen Formen handelt, ausgenommen sind.

**14.** Verfahren nach Anspruch 13, dadurch gekennzeichnet, daß man Verbindungen der Formel I herstellt, in welcher $R^1$, X und Y die in Anspruch 4 definierten Reste darstellen und A und n die in Anspruch 5 genannten Bedeutungen haben, wobei die Verbindungen 3-Phenyl-2-isoxazolin-5-yl-phosphonsäure und deren Dimethylester, sofern es sich um deren racemischen Formen handelt, ausgenommen sind.

**15.** Verfahren nach Anspruch 14, dadurch gekennzeichnet, daß man Verbindungen der Formel I herstellt, in welcher $R^1$, X und Y die in Anspruch 6 definierten Reste darstellen und A und n die in Anspruch 5 genannten Bedeutungen haben, wobei die racemische 3-Phenyl-2-isoxazolin-5-yl-phosphonsäure ausgenommen ist.

**16.** Verfahren nach Anspruch 15, dadurch gekennzeichnet, daß man 3-Phenyl- oder 3-tert.-Butyl-2-isoxazolin-5-yl-(P-methyl)-phosphinsäure herstellt.

**17.** Verfahren nach Anspruch 15, dadurch gekennzeichnet, daß man die enantiomeren Formen der 3-Phenyl-2-isoxazolin-5-yl-phosphonsäure herstellt.

**18.** Verfahren zur Herstellung von Verbindungen der Formel I gemäß der Definition in Anspruch 1, ihrer gegebenenfalls stereoisomeren Formen und/oder gegebenenfalls ihrer physiologisch verträglichen Salze zur Anwendung in einem Verfahren zur prophylaktischen und/oder therapeutischen Behandlung des menschlichen oder tierischen Körpers.

**19.** Verfahren nach Anspruch 18, dadurch gekennzeichnet, daß die Verbindungen zur Anwendung einem Verfahren zur prophylaktischen und/oder therapeutischen Behandlung von Erkrankungen des Immunsystems des menschlichen oder tierischen Körpers hergestellt werden.

**20.** Verfahren nach Anspruch 18, dadurch gekennzeichnet, daß die Verbindungen zur Anwendung in einem Verfahren zur prophylaktischen und/oder therapeutischen Behandlung von Tumoren, Infektionen und/oder Autoimmunerkrankungen des menschlichen oder tierischen Körpers sowie für die Adjuvierung von Impfstoffen hergestellt werden.

**21.** Verwendung von Verbindungen der Formel I gemäß der Definition in Anspruch 1, ihrer gegebenenfalls stereoisomeren Formen und/oder gegebenenfalls ihrer physiologisch verträglichen Salze zur Herstellung von Arzneimitteln.

**22.** Verwendung von Verbindungen der Formel I gemäß der Definition in Anspruch 1, ihrer gegebenenfalls stereoisomeren Formen und/oder gegebenenfalls ihrer physiologisch verträglichen Salze zur Herstellung von Arzneimitteln gemäß Anspruch 21, die zur Prophylaxe und/oder Behandlung von Erkrankungen des Immunsystems bestimmt sind.

**23.** Verwendung der Verbindungen der Formel I gemäß der Definition in Anspruch 1, ihrer gegebenenfalls stereoisomeren Formen und/oder gegebenenfalls ihrer physiologisch verträglichen Salze zur Herstellung von Arzneimitteln gemäß Anspruch 22, die zur Prophylaxe und/oder Behandlung von Tumoren, Infektionen und/oder Autoimmunerkrankungen und für die Adjuvierung von Impfstoffen bestimmt sind.

**Claims**
**Claims for the following Contracting States : AT, BE, CH, DE, FR, GB, IT, LI, LU, NL, SE**

**1.** A pharmaceutical which contains or is composed of at least one compound of the formula I and/or one of its physiologically tolerated salts where appropriate,

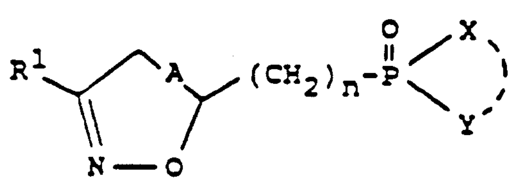

(I)

where $R^1$ represents

a) a straight-chain or branched alkyl or alkenyl group which has 1 to 6 carbon atoms and whose carbon chain can be substituted by halogen, hydroxyl, $(C_1-C_4)$alkoxy, $(C_1-C_4)$acyloxy or aryl which is optionally substituted by $(C_1-C_4)$alkoxy or halogen, or

b) a mono- or binuclear aromatic or heteroaromatic group having 1 or 2 nitrogen atoms and/or one sulfur or oxygen atom in the ring system, it being possible for this group to be substituted one or more times and identically or differently by straight-chain or branched $(C_1-C_4)$alkyl, $(C_3-C_6)$-cycloalkyl, hydroxyl, $(C_1-C_3)$alkoxy, aryloxy, $(C_1-C_4)$acyloxy or benzoyloxy, halogen, trifluoromethyl, nitro, optionally mono- or disubstituted amino, $(C_1-C_4)$-alkoxycarbonyl, carboxyl, carbamoyl, $(C_1-C_4)$-alkylcarbonyl, whose carbonyl group can in each case also be in ketalized form, or benzyl or phenyl which is optionally ring-substituted by $(C_1-C_4)$ alkyl, halogen or $(C_1-C_3)$ alkoxy, or

c) carboxyl or alkoxycarbonyl having 1 to 4 carbon atoms in the alkyl moiety or

d) arylcarbonyl which is optionally substituted in the aryl moiety by $(C_1\text{-}C_4)$alkyl, halogen or $(C_1\text{-}C_3)$-alkoxy, or
e) halogen,
A denotes a C,C single bond or a C,C double bond,
n denotes an integer from 0 to 2, and
X and Y, which can be identical or different, each denote, independently of one another, a straight-chain or branched $(C_1\text{-}C_4)$ all group, the radical $-OR^2$ or the group $-NR^2R^3$, where $R^2$ and $R^3$ represent hydrogen or optionally substituted $(C_1\text{-}C_6)$alkyl radicals which, in the group $-NR^2R^3$, can also form together with the nitrogen atom a five- to seven-membered ring or, in the structural element $-P(O)(OR^2)_2$, can form together with the phosphorus atom a heterocycle of the formula

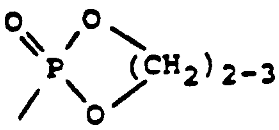

which is optionally also substituted by $(C_1\text{-}C_3)$alkyl, $(C_1\text{-}C_4)$alkoxycarbonyl or carboxyl,
and where the compounds of the formula I can, where appropriate, be in the form of pure stereoisomers or mixtures thereof.

**2.** A pharmaceutical as claimed in claim 1, which contains or is composed of at least one compound of the formula I and/or one of its salts where appropriate, in which $R^1$ represents
a) optionally branched $(C_1\text{-}C_4)$alkyl or $(C_1\text{-}C_4)$hydroxyalkyl or phenyl$(C_1\text{-}C_2)$alkyl or phenyl$(C_2\text{-}C_3)$-alkenyl,
b) phenyl, naphthyl, pyridyl or thienyl, each of which is unsubstituted or substituted one or more times by $(C_1\text{-}C_4)$alkyl, hydroxyl, $(C_1\text{-}C_2)$alkoxy, phenoxy, halogen, trifluoromethyl, nitro, di$(C_1\text{-}C_2)$-alkylamino, $(C_1\text{-}C_2)$alkoxycarbonyl, carboxyl or phenyl,
c) carboxyl or meth- or ethoxycarbonyl,
d) benzoyl,
e) chlorine or bromine,
A denotes a C,C single bond or a C,C double bond,
n represents 0 or 1, and
X and Y, which can be identical or different, represent independently of one another a methyl or ethyl group or the radicals $-OR^2$ or $-NR^2R^3$, where $R^2$ represents hydrogen, methyl or ethyl, and $R^3$ likewise represents hydrogen, methyl or ethyl, or else represent the carbon skeleton of an optionally carboxyl-protected amino acid, the radicals $R^2$ and $R^3$ in the group $-NR^2R^3$ can also form together with the nitrogen atom a pyrrolidine, piperidine or morpholine ring, and the radicals $-OR^2$ in the structural element $-P(O)(OR^2)_2$ can form together with the phosphorus atom a 2-oxo-1,3,2-dioxaphospholane or 2-oxo-1,3,2-dioxaphosphorinane ring, each of which is optionally substituted by $(C_1\text{-}C_2)$alkyl, and where these compounds can, where appropriate, be in the form of pure stereoisomers or mixtures thereof.

**3.** A pharmaceutical as claimed in claim 2, which contains or is composed of at least one compound of the formula I and/or one of its salts where appropriate, in which either $R^1$ represents tert.butyl, benzyl, phenyl, naphthyl, pyridyl or thienyl, or phenyl which is substituted by methyl, hydroxyl, methoxy, phenoxy, chlorine, fluorine, trifluoromethyl, nitro, dimethylamino, methoxy-carbonyl or carboxyl, or X and Y denote, independently of one another, hydroxyl, methoxy or ethoxy, or X denotes methyl and Y denotes hydroxyl, methoxy or ethoxy, and where these compounds can, where appropriate, be in the form of pure stereoisomers or mixtures thereof.

**4.** A pharmaceutical as claimed in claim 3, which contains or is composed of at least one compound of the formula I and/or one of its salts where appropriate, in which $R^1$, X and Y all have the meanings defined in claim 3, and where these compounds can, where appropriate, be in the form of pure stereoisomers or mixtures thereof.

**5.** A pharmaceutical as claimed in claim 4, which contains or is composed of at least one compound of the formula I and/or one of its salts where appropriate, in which A represents a C,C single bond, and n

has the value 0, where these compounds can be in the form of pure stereoisomers or mixtures thereof.

**6.** A pharmaceutical as claimed in claim 5, which contains or is composed of at least one compound of the formula I and/or one of its salts, in which $R^1$ represents tert.butyl or phenyl, X and Y each denote hydroxyl, or X denotes methyl and Y denotes hydroxyl, where these compounds can be in the form of pure stereoisomers or mixtures thereof.

**7.** A pharmaceutical as claimed in claim 6, which contains or is composed of 3-phenyl-2-isoxazolin-5-ylphosphonic acid and/or 3-phenyl(or 3-tert.butyl)-2-isoxazolin-5-yl(P-methyl)phosphinic acid and/or at least one of their salts, where these compounds can be in the form of pure stereoisomers or mixtures thereof.

**8.** A pharmaceutical as claimed in claims 1 to 7, which is intended for the prophylaxis and/or treatment of diseases of the immune system.

**9.** A pharmaceutical as claimed in claim 8, which is used for the prophylaxis and/or treatment of tumors, infections and/or autoimmune diseases and as adjuvant in vaccines.

**10.** A process for the preparation of a pharmaceutical as claimed in claims 1 to 9, which comprises converting at least one compound of the formula I, where appropriate in the form of a pure stereoisomer and/or as physiologically tolerated salt, with a physiologically acceptable vehicle and, where appropriate, further additives and/or auxiliaries into a suitable pharmaceutical administration form.

**11.** A compound of the formula I, its stereoisomeric forms where appropriate and its physiologically tolerated salts where appropriate, wherein $R^1$, A, n, X and Y have the meanings defined in claim 1, excepting the compounds 3-phenyl-2-isoxazolin-5-ylphosphonic acid, dimethyl 3-methyl(and phenyl)-2-isoxazolin-5-ylphosphonate, dipropyl 3-(3-nitrophenyl)-2-isoxazolin-5-ylphosphonate, diethyl 3-(2-nitro-5-(2-chloro-4-trifluoromethylphenoxy)phenyl)-2-isoxazolin-5-ylphosphonate, 3-methyl(and phenyl)-2-isoxazolin-5-ylphosphonic tetramethyldiamide, 3-phenyl-2-isoxazolin-5-ylmethylphosphonic acid and the diethyl ester thereof, diethyl 3-methyl (ethyl, isopropyl, tert.-butyl, methoxymethyl, phenyl and ethoxycarbonyl)-5-isoxazolylmethylphosphonate, 3-(4-fluoro- and 4-chloro-phenyl)-5-isoxazolyl(P-methyl)-phosphinic acid and methyl 3-phenyl-5-isoxazolyl(P-methyl)phosphinate, where the racemic forms are being dealt with where appropriate.

**12.** A compound as claimed in claim 11, its stereoisomeric forms where appropriate and its salts where appropriate, wherein $R^1$, A, n, X and Y represent the radicals defined in claim 2, excepting the compounds 3-phenyl-2-isoxazolin-5-ylphosphonic acid, dimethyl 3-methyl(and phenyl)-2-isoxazolin-5-ylphosphonate, 3-methyl-(and phenyl)-2-isoxazolin-5-ylphosphonic tetramethyldiamide, 3-phenyl-2-isoxazolin-5-ylmethylphosphonic acid and the diethyl ester thereof, diethyl 3-methyl(ethyl, isopropyl, tert.butyl, phenyl and ethoxycarbonyl)-5-isoxazolyl-methylphosphonate, 3-(4-fluoro- and 4-chlorophenyl)-5-isoxazolyl(P-methyl)phosphinic acid and methyl 3-phenyl-5-isoxazolyl(P-methyl)-phosphinate, where the racemic forms are being dealt with where appropriate.

**13.** A compound as claimed in claim 12, its stereoisomeric forms where appropriate and its salts where appropriate, wherein $R^1$, X and Y represent the radicals defined in claim 3, and A and n have the meanings mentioned in claim 2, excepting the compounds 3-phenyl-2-isoxazolin-5-ylphosphonic acid, dimethyl 3-methyl(and phenyl)-2-isoxazolin-5-ylphosphonic acid, 3-phenyl-2-isoxazolin-5-ylphosphonic tetramethyldiamide, 3-phenyl-2-isoxazolin-5-ylmethylphosphonic acid and the diethyl ester thereof, diethyl 3-methyl(ethyl, isopropyl, tert.-butyl, phenyl and ethoxycarbonyl)-5-isoxazolylmethylphosphonate, 3-(4-fluoro-and 4-chlorophenyl)-5-isoxazol-yl(P-methyl)phosphinicacid and methyl 3-phenyl-5-isoxazolyl(P-methyl)phosphinate, where the racemic forms thereof are being dealt with where appropriate.

**14.** A compound as claimed in claim 13, its stereoisomeric forms where appropriate and its salts where appropriate, wherein $R^1$, X and Y represent the radicals defined in claim 4, and A and n have the meanings mentioned in claim 2, excepting the compounds 3-phenyl-2-isoxazolin-5-ylphosphonic acid and the dimethyl ester thereof, 3-phenyl-2-isoxazolin-5-ylmethylphosphonic acid and the diethyl ester thereof, diethyl 3-tert.butyl(and phenyl)-5-isoxazolylmethylphosphonate, 3-(4-fluoro- and 4-chlorophenyl)-5-isoxazolyl(P-methyl)phosphinic acid and methyl 3-phenyl-5-isoxazolyl(P-methyl)-phosphinate, where the racemic forms thereof are being dealt with where appropriate.

**15.** A compound as claimed in claim 14, its stereoisomeric forms and its salts where appropriate, wherein $R^1$, X, and Y represent the radicals defined in claim 4, and A and n have the meanings mentioned in claim 5, excepting the compounds 3-phenyl-2-isoxazolin-5-ylphosphonic acid and its dimethyl ester, where the racemic forms thereof are being dealt with where appropriate.

**16.** A compound as claimed in claim 15, its stereoisomeric forms and its salts, wherein $R^1$, X, and Y represent the radicals defined in claim 6, and A and n have the meanings mentioned in claim 5, excepting racemic 3-phenyl-2-isoxazolin-5-ylphosphonic acid.

**17.** A compound as claimed in claim 16, its stereoisomeric forms and its salts, which represents 3-phenyl or 3-tert.butyl-2-isoxazolin-5-yl(P-methyl)phosphinic acid.

**18.** A compound as claimed in claim 16, and its salts, which represents an enantiomeric form of 3-phenyl-2-isoxazolin-5-ylphosphonic acid.

**19.** A process for the preparation of phosphorus-containing 2-isoxazolines and isoxazoles of the formula I as claimed in claims 11 to 18, their stereoisomeric forms where appropriate and their physiologically tolerated salts where appropriate, which comprises reacting a nitrile oxide of the formula II

$R^1$-C≡N→O (II)

a) in the case where A in formula I denotes a C,C single bond,
with an olefinic phosphorus compound of the formula III

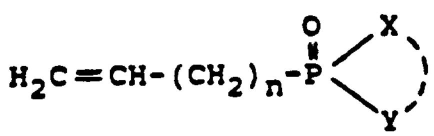

(III)

or
b) in the case where A in formula I denotes a C,C double bond,
with an olefinic phosphorus compound of the formula IV

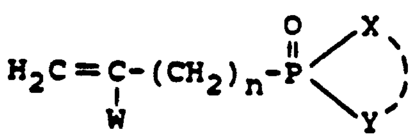

(IV)

to give a 2-isoxazoline of the formula V

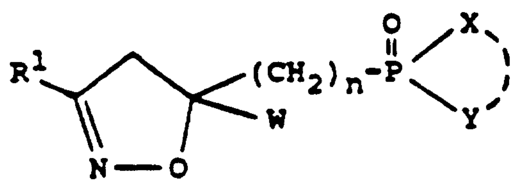

(V)

and eliminating HW from this intermediate under basic conditions or by exposure to heat, where $R^1$, n, X and Y in formulae II to V have the abovementioned meanings, and W represents a leaving group, in particular halogen, and, where appropriate,

c) cleaving a phosphonic or phosphinic ester of the formula I obtained as in a) or b) to give the phosphonic monoester or to give the phosphonic or phosphinic acid of the formula I, or

d) reacting a dialkyl phosphonate of the formula I obtained as in a) or b) with an amine of the formula $HNR^2R^3$ (VI) with replacement of one of the two alkoxy groups on the phosphorus by the radical $-NR^2R^3$ to give a monoester monoamide of the formula I, where $R^2$ and $R^3$ have the abovementioned meanings, and compounds in which $R^1$ denotes halogen are excepted, or

e) initially converting a phosphonic acid of the formula I prepared as in a), b) or c) into an acid derivative activated on the phosphorus atom, and subsequently reacting the latter with alcohols of the formula $R^2OH$ (VII) or a diol of the formula $HO-(CH_2)_{2-3}-OH$ (VIII) and/or amines of the formula VI, as selected, to give a mono- or optionally mixed diester, a cyclic ester, a monoester monoamide or a mono- or optionally mixed diamide of the formula I, or

reacting a phosphonic monoester of the formula I obtained as in a), b) or c), after activation on the phosphorus atom, with an alcohol VII or an amine VI to give an optionally mixed diester or a monoester monoamide of the formula I, or

reacting a phosphinic acid of the formula I prepared as in a), b) or c), after activation on the phosphorus atom, with an alcohol VII or an amine VI to give an ester or amide of the formula I,

where $R^2$ and $R^3$ in formula VI have the abovementioned meanings, $R^2$ in formula VII represents optionally substituted $(C_1-C_6)$ alkyl, and the alkylene chain $-(CH_2)_{2-3}-$ in formula VIII can also be substituted by $(C_1-C_3)$alkyl, $(C_1-C_4)$alkoxycarbonyl or carboxyl, or

f) reacting a 3-chloro(or bromo)-2-isoxazoline-phosphonic di- or monoester or -phosphinic ester of the formula I, which has been prepared as in a) and in which n denotes an integer from 0 to 2, with tri$(C_1-C_4)$alkylhalogeno-silanes to give, with ester cleavage and simultaneous replacement of chlorine or bromine by the halogen atom of the particular silane used, the corresponding 3-halogeno-2-isoxazoline-phosphonic or -phosphinic acids of the formula I or

g) resolving a compound of the formula I which has been obtained as in a) to f), which, by reason of its chemical structure, occurs in diastereomeric or enantiomeric forms, into the pure stereoisomers with the compounds of the formula I prepared as in a) to g) being either isolated in free form or, where appropriate, converted into physiologically tolerated salts.

**20.** A compound of the formula I, its stereoisomeric forms where appropriate and/or its physiologically tolerated salts where appropriate, for use in a method for the prophylactic and/or therapeutic treatment of the human or animal body.

**21.** The embodiment as claimed in claim 20 for the prophylactic and/or therapeutic treatment of diseases of the immune system of the human or animal body.

**22.** The embodiment as claimed in claim 20 for the prophylactic and/or therapeutic treatment of tumors, infections and/or autoimmune diseases of the human or animal body and for use as adjuvant in vaccines.

**23.** The use of compounds of the formula I, their stereoisomeric forms where appropriate and/or their physiologically tolerated salts where appropriate, for the preparation of pharmaceuticals as claimed in claims 1 to 7.

**24.** The use of compounds of the formula I, their stereoisomeric forms where appropriate and/or their physiologically tolerated salts where appropriate, for the preparation of pharmaceuticals as claimed in claim 23, which are intended for the prophylaxis and/or treatment of diseases of the immune system.

**25.** The use of compounds of the formula I, their stereoisomeric forms where appropriate and/or their physiologically tolerated salts where appropriate, for the preparation of pharmaceuticals as claimed in claim 24, which are intended for the prophylaxis and/or treatment of tumors, infections and/or autoimmune diseases and for providing adjuvants for vaccines.

**Claims for the following Contracting States : ES, GR**

**1.** A process for the preparation of a pharmaceutical, which comprises converting an effective amount of at least one compound of the formula I below 1, where appropriate in the form of the pure stereoisomer and/or as physiologically tolerated salt, with a physiologically acceptable vehicle and, where appropriate, further additives and/or auxiliaries into a suitable pharmaceutical administration form:

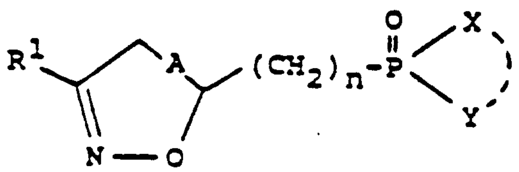

(I)

where $R^1$ represents

a) a straight-chain or branched alkyl or alkenyl group which has 1 to 6 carbon atoms and whose carbon chain can be substituted by halogen, hydroxyl, $(C_1\text{-}C_4)$alkoxy, $(C_1\text{-}C_4)$acyloxy or aryl which is optionally substituted by $(C_1\text{-}C_4)$alkoxy or halogen, or

b) a mono- or binuclear aromatic or heteroaromatic group having 1 or 2 nitrogen atoms and/or one sulfur or oxygen atom in the ring system, it being possible for this group to be substituted one or more times and identically or differently by straight-chain or branched $(C_1\text{-}C_4)$alkyl, $(C_3\text{-}C_6)$-cycloalkyl, hydroxyl, $(C_1\text{-}C_3)$alkoxy, aryloxy, $(C_1\text{-}C_4)$acyloxy or benzoyloxy, halogen, trifluoromethyl, nitro, optionally mono- or disubstituted amino, $(C_1\text{-}C_4)$-alkoxycarbonyl, carboxyl, carbamoyl, $(C_1\text{-}C_4)$-alkylcarbonyl, whose carbonyl group can in each case also be in ketalized form, or benzyl or phenyl which is optionally ring-substituted by $(C_1\text{-}C_4)$alkyl, halogen or $(C_1\text{-}C_3)$alkoxy, or

c) carboxyl or alkoxycarbonyl having 1 to 4 carbon atoms in the alkyl moiety or

d) arylcarbonyl which is optionally substituted in the aryl moiety by $(C_1\text{-}C_4)$alkyl, halogen or $(C_1\text{-}C_3)$-alkoxy, or

e) halogen,

A denotes a C,C single bond or a C,C double bond,

n denotes an integer from 0 to 2, and

X and Y, which can be identical or different, each denote, independently of one another, a straight-chain or branched $(C_1\text{-}C_4)$alkyl group, the radical $-OR^2$ or the group $-NR^2R^3$, where $R^2$ and $R^3$ represent hydrogen or optionally substituted $(C_1\text{-}C_6)$alkyl radicals which, in the group $-NR^2R^3$, can also form together with the nitrogen atom a five- to seven-membered ring or, in the structural element $-P(O)(OR^2)_2$, can form together with the phosphorus atom a heterocycle of the formula

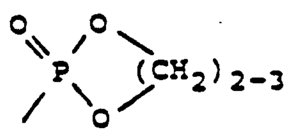

which is optionally also substituted by $(C_1\text{-}C_3)$alkyl, $(C_1\text{-}C_4)$alkoxycarbonyl or carboxyl.

**2.** The process as claimed in claim 1, wherein in formula I

$R^1$ represents

a) optionally branched $(C_1\text{-}C_4)$alkyl or $(C_1\text{-}C_4)$hydroxyalkyl or phenyl $(C_1\text{-}C_2)$alkyl or phenyl $(C_2\text{-}C_3)$-alkenyl,

b) phenyl, naphthyl, pyridyl or thienyl, each of which is unsubstituted or substituted one or more times by $(C_1\text{-}C_4)$alkyl, hydroxyl, $(C_1\text{-}C_2)$alkoxy, phenoxy, halogen, trifluoromethyl, nitro, di $(C_1\text{-}C_2)$-alkylamino, $(C_1\text{-}C_2)$alkoxycarbonyl, carboxyl or phenyl,

c) carboxyl or meth- or ethoxycarbonyl,

d) benzoyl,

e) chlorine or bromine,

A denotes a C,C single bond or a C,C double bond,

n represents 0 or 1, and

X and Y, which can be identical or different, represent independently of one another a methyl or ethyl group or the radicals $-OR^2$ or $-NR^2R^3$, where $R^2$ represents hydrogen, methyl or ethyl, and $R^3$ likewise represents hydrogen, methyl or ethyl, or else represent the carbon skeleton of an optionally carboxyl-protected amino acid, the radicals $R^2$ and $R^3$ in the group $-NR^2R^3$ can also form together with the nitrogen atom a pyrrolidine, piperidine or morpholine ring, and the radicals $-OR^2$ in the

structural element $-P(O)(OR^2)_2$ can form together with the phosphorus atom a 2-oxo-1,3,2-dioxaphospholane or 2-oxo-1,3,2-dioxaphosphorinane ring, each of which is optionally substituted by $(C_1-C_2)$alkyl.

**3.** The process as claimed in claim 2, wherein in formula I either $R^1$ represents tert.butyl, benzyl, phenyl, naphthyl, pyridyl or thienyl, or phenyl which is substituted by methyl, hydroxyl, methoxy, phenoxy, chlorine, fluorine, trifluoromethyl, nitro, dimethylamino, methoxycarbonyl or carboxyl, or X and Y denote, independently of one another, hydroxyl, methoxy or ethoxy, or X denotes methyl and Y denotes hydroxyl, methoxy or ethoxy.

**4.** The process as claimed in claim 3, wherein in formula I $R^1$, X and Y all have the meanings defined in claim 3.

**5.** The process as claimed in claim 4, wherein in formula I A represents a C,C single bond and n has the value 0.

**6.** The process as claimed in claim 5, wherein in formula I $R^1$ represents tert.butyl or phenyl, X and Y each denote hydroxyl, or X denotes methyl and Y denotes hydroxyl.

**7.** The process as claimed in claim 6, wherein the compound(s) of the formula I is (are) 3-phenyl-2-isoxazolin-5-ylphosphonic acid and/or 3-phenyl(or 3-tert.butyl)-2-isoxazolin-5-yl(P-methyl)phosphinic acid.

**8.** The process as claimed in one or more of claims 1 to 7, wherein the pharmaceuticals prepared by the process are intended for the prophylaxis and/or treatment of diseases of the immune system.

**9.** The process as claimed in claim 8, wherein the pharmaceuticals prepared by the process are used for the prophylaxis and/or treatment of tumors, infections and/or autoimmune diseases and as adjuvant in vaccines.

**10.** A process for the preparation of phosphorus-containing 2-isoxazolines and isoxazoles of the formula I as defined in claim 1, their stereoisomeric forms where appropriate and their physiologically tolerated salts where appropriate, excepting the compounds 3-phenyl-2-isoxazolin-5-ylphosphonic acid, dimethyl 3-methyl(and phenyl)-2-isoxazolin-5-ylphosphonate, dipropyl 3-(3-nitrophenyl)-2-isoxazolin-5-ylphosphonate, diethyl 3-(2-nitro-5-(2-chloro-4-trifluoromethylphenoxy)phenyl)-2-isoxasolin-5-ylphosphonate, 3-methyl(and phenyl)-2-isoxazolin-5-ylphosphonic tetramethyldiamide, 3-phenyl-2-isoxazolin-5-ylmethylphosphonic acid and the diethyl ester thereof, diethyl 3-methyl(ethyl, isopropyl, tert.-butyl, methoxymethyl, phenyl and ethoxycarbonyl)-5-isoxazolylmethylphosphonate, 3-(4-fluoro- and 4-chlorophenyl)-5-isoxazolyl(P-methyl)phosphinic acid and methyl 3-phenyl-5-isoxasolyl(P-methyl)-phosphinate, where the racemic forms are being dealt with where appropriate, which comprises reacting a nitrile oxide of the formula II

$R^1$-C≡N→O (II)

a) in the case where A in formula I denotes a C,C single bond, with an olefinic phosphorus compound of the formula III

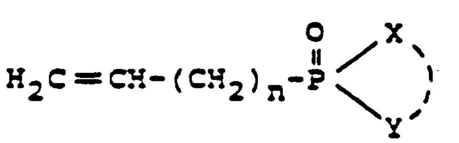

(III)

or

b) in the case where A in formula I denotes a C,C double bond, with an olefinic phosphorus compound of the formula IV

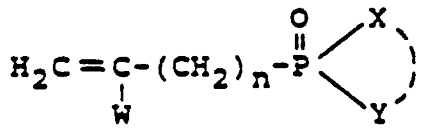

(IV)

to give a 2-isoxazoline of the formula V

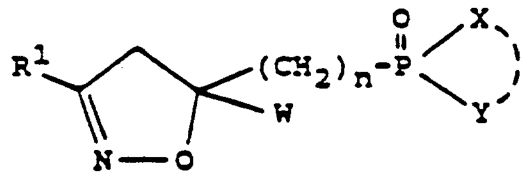

(V)

and eliminating HW from this intermediate under basic conditions or by exposure to heat, where $R^1$, n, X and Y in formulae II to V have the abovementioned meanings, and W represents a leaving group, in particular halogen, and, where appropriate,

c) cleaving a phosphonic or phosphinic ester of the formula I obtained as in a) or b) to give the phosphonic monoester or to give the phosphonic or phosphinic acid of the formula I, or

d) reacting a dialkyl phosphonate of the formula I obtained as in a) or b) with an amine of the formula $HNR^2R^3$ (VI) with replacement of one of the two alkoxy groups on the phosphorus by the radical $-NR^2R^3$ to give a monoester monoamide of the formula I, where $R^2$ and $R^3$ have the abovementioned meanings, and compounds in which $R^1$ is halogen are excepted, or

e) initially converting a phosphonic acid of the formula I prepared as in a), b) or c) into an acid derivative activated on the phosphorus atom, and subsequently reacting the latter with alcohols of the formula $R^2OH$ (VII) or a diol of the formula $HO-(CH_2)_{2-3}-OH$(VIII) and/or amines of the formula VI, as selected, to give a mono- or optionally mixed diester, a cyclic ester, a monoester monoamide or a mono- or optionally mixed diamide of the formula I, or

reacting a phosphonic monoester of the formula I obtained as in a), b) or c), after activation on the phosphorus atom, with an alcohol VII or an amine VI to give an optionally mixed diester or a monoester monoxide of the formula I, or

reacting a phosphinic acid of the formula I prepared as in a), b) or c), after activation on the phosphorus atom, with an alcohol VII or an amine VI to give an ester or amide of the formula I,

where $R^2$ and $R^3$ in formula VI have the abovementioned meanings, $R^2$ in formula VII is optionally substituted ($C_1$-$C_6$)alkyl, and the alkylene chain $-(CH_2)_{2-3}-$ in formula VIII can also be substituted by ($C_1$-$C_3$)alkyl, ($C_1$-$C_4$)alkoxycarbonyl or carboxyl, or

f) reacting a 3-chloro(or bromo)-2-isoxazoline-phosphonic di- or monoester or -phosphinic ester of the formula I, which has been prepared as in a) and in which n denotes an integer from 0 to 2, with tri($C_1$-$C_4$)alkylhalogeno-silanes to give, with ester cleavage and simultaneous replacement of chlorine or bromine by the halogen atom of the particular silane used, the corresponding 3-halogeno-2-isoxazoline-phosphonic or -phosphinic acids of the formula I, or

g) resolving a compound of the formula I which has been obtained as in a) to f), which, by reason of its chemical structure, occurs in diastereomeric or enantiomeric forms, into the pure stereoisomers with the compounds of the formula I prepared as in a) to g) being either isolated in free form or, where appropriate, converted into physiologically tolerated salts.

**11.** The process as claimed in claim 10, which comprises preparing compounds of the formula I in which $R^1$, A, n, X and Y represent the radicals defined in claim 2, excepting the compounds 3-phenyl-2-isoxazolin-5-ylphosphonic acid, dimethyl 3-methyl(and phenyl)-2-isoxazolin-5-ylphosphonate, 3-methyl (and phenyl)-2-isoxazolin-5-ylphosphonic tetramethyldiamide, 3-phenyl-2-isoxazolin-5-ylmethylphosphonic acid and the diethyl ester thereof, diethyl 3-methyl(ethyl, isopropyl, tert.-butyl, phenyl and ethoxycarbonyl)-5-isoxazolylmethyiphosphonate, 3-(4-fluoro-and-4-chlorophenyl)-5-isoxazolyl(P-methyl)-phosphinic acid and methyl 3-phenyl-5-isoxazolyl-(P-methyl)phosphinate, where the racemic forms are being dealt with where appropriate.

**12.** The process as claimed in claim 11, which comprises preparing compounds of the formula I in which $R^1$, X and Y represent the radicals defined in claim 3, and A and n have the meanings mentioned in claim 2, excepting the compounds 3-phenyl-2-isoxazolin-5-ylphosphonic acid, dimethyl 3-methyl (and phenyl)-2-isoxasolin-5-ylphosphonate, 3-phenyl-2-isoxazolin-5-ylphosphonic tetramethyldiamide, 3phenyl-2-isoxazolin-5-ylmethylphosphonic acid and the dimethyl ester thereof, diethyl 3-methyl-(ethyl, isopropyl, tert.butyl, phenyl and ethoxycarbonyl)-5-isoxasolylmethylphosphonate, 3-(4-fluoro- and 4-chlorophenyl)-5-isoxazolyl(P-methyl)phosphinic acid and methyl 3-phenyl-5-isoxazolyl(P-methyl)-phosphinate, where the racemic forms thereof are being dealt with where appropriate.

**13.** The process as claimed in claim 12, which comprises preparing compounds of the formula I in which $R^1$, X and Y represent the radicals defined in claim 4, and A and n have the meanings mentioned in claim 2, excepting the compounds 3-phenyl-2-isoxasolin-5-ylphosphonic acid and the dimethyl ester thereof, 3-phenyl-2-isoxazolin-5-ylmethylphosphonic acid and the diethyl ester thereof, diethyl 3-tert.butyl(and phenyl)-5-isoxazolylmethyl-phosphonate, 3-(4-fluoro- and 4-chlorophenyl)-5-isoxasolyl(P-methyl)phosphinic acid and methyl 3-phenyl-5-isoxazolyl(P-methyl)phosphinate, where the racemic forms thereof are being dealt with where appropriate.

**14.** The process as claimed in claim 13, which comprises preparing compounds of the formula I in which $R^1$, X and Y are the radicals defined in claim 4, and A and n have the meanings mentioned in claim 5, excepting the compounds 3-phenyl-2-isoxazolin-5-ylphosphonic acid and the dimethyl ester thereof, where the racemic forms thereof are being dealt with where appropriate.

**15.** The process as claimed in claim 14, which comprises preparing compounds of the formula I in which $R^1$, X and Y are the radicals defined in claim 6, and A and n have the meanings mentioned in claim 5, excepting racemic 3-phenyl-2-isoxazolin-5-ylphosphonic acid.

**16.** The process as claimed in claim 15, which comprises preparing 3-phenyl- or 3-tert.butyl-2-isoxazolin-5-yl(P-methyl)phosphinic acid.

**17.** The process as claimed in claim 15, which comprises preparing enantiomeric forms of 3-phenyl-2-isoxazolin-5-ylphosphonic acid.

**18.** A process for the preparation of compounds of the formula I as defined in claim 1, their stereoisomeric forms where appropriate and/or their physiologically tolerated salts where appropriate, for use in a method for the prophylactic and/or therapeutic treatment of the human or animal body.

**19.** The process as claimed in claim 18, which comprises preparing the compounds for use in a method for the prophylactic and/or therapeutic treatment of diseases of the immune system of the human or animal body.

**20.** The process as claimed in claim 18, which comprises preparing the compounds for use in a method for the prophylactic and/or therapeutic treatment of tumors, infections and/or autoimmune diseases of the human or animal body and for use as adjuvant in vaccines.

**21.** The use of compounds of the formula I as defined in claim 1, their stereoisomeric forms where appropriate and/or their physiologically tolerated salts where appropriate for preparing pharmaceuticals.

**22.** The use of compounds of the formula I as defined in claim 1, their stereoisomeric forms where appropriate and/or their physiologically tolerated salts where appropriate for preparing pharmaceuticals, as in claim 21, which are intended for the prophylaxis and/or treatment of diseases of the immune system.

**23.** The use of compounds of the formula I as defined in claim 1, their stereoisomeric forms where appropriate and/or their physiologically tolerated salts where appropriate for preparing pharmaceuticals, as in claim 22, which are intended for the prophylaxis and/or treatment of tumors, infections and/or autoimmune diseases and for providing adjuvants for vaccines.

**Revendications**

**Revendications pour les Etats contractants suivants : AT, BE, CH, DE, FR, GB, IT, LI, LU, NL, SE**

**1.** Médicaments caractérisés par une teneur en - ou consistant en - au moins un composé de formule I et/ou éventuellement un de ses sels physiologiquement acceptables,

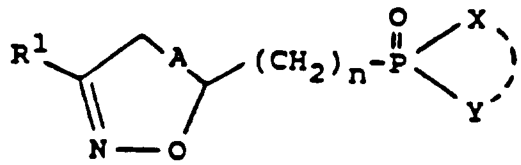

(I)

formule dans laquelle $R^1$ représente

a) un groupe alkyle ou alcényle à chaîne droite ou ramifiée ayant de 1 à 6 atomes de carbone, dont la chaîne carbonée peut être substituée par un atome d'halogène ou par un groupe hydroxy, alcoxy en $C_1$-$C_4$, acyloxy en $C_1$-$C_4$ ou par un radical aryle éventuellement substitué par un atome d'halogène ou par un groupe alcoxy en $C_1$-$C_4$, ou

b) un groupe aromatique ou hétéroaromatique mono- ou binucléaire comportant 1 ou 2 atomes d'azote et/ou un atome d'oxygène ou de soufre dans le système cyclique, ce groupe pouvant être une ou plusieurs fois substitué et de façon identique ou différente par un ou des atomes d'halogène ou groupes alkyle en $C_1$-$C_4$, cycloalkyle en $C_3$-$C_6$, hydroxy, alcoxy en $C_1$-$C_3$, aryloxy, acyloxy en $C_1$-$C_4$, benzoyloxy, halogène, trifluorométhyle, nitro, amino éventuellement mono- ou disubstitué, (alcoxy en $C_1$-$C_4$)-carbonyle, carboxy, carbamoyle, (alkyl en $C_1$-$C_4$)-carbonyle, dont le groupe carbonyle peut également se trouver sous forme cétalisée, ou phényle ou benzyle éventuellement substitués sur le noyau par un atome d'halogène ou par un groupe alkyle en $C_1$-$C_4$ ou alcoxy en $C_1$-$C_3$, ou

c) un groupe carboxy ou alcoxycarbonyle ayant de 1 à 4 atomes de carbone dans le fragment alkyle, ou

d) un groupe arylcarbonyle qui est éventuellement substitué sur le fragment aryle par un atome d'halogène ou par un groupe alkyle en $C_1$-$C_4$ ou alcoxy en $C_1$-$C_3$, ou

e) un atome d'halogène,

A représente une simple liaison C-C ou une double liaison C-C,

n est un nombre entier allant de 0 à 2, et

X et Y, qui peuvent être identiques ou différents, représentent chacun, indépendamment l'un de l'autre, un groupe alkyle en $C_1$-$C_4$ à chaîne droite ou ramifiée, un radical $-OR^2$ ou $-NR^2R^3$, $R^2$ et $R^3$ représentant des atomes d'hydrogène ou des radicaux alkyle en $C_1$-$C_6$ éventuellement substitués qui, dans le groupe $-NR^2R^3$, peuvent également former, conjointement avec l'atome d'azote, un cycle à 5-7 chaînons, ou, dans l'élément structural $-P(O)(OR^2)_2$, peuvent former, conjointement avec l'atome de phosphore, un hétérocycle de formule

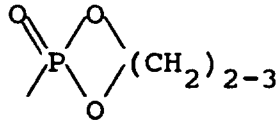

éventuellement encore substitué par un groupe alkyle en $C_1$-$C_3$, (alcoxy en $C_1$-$C_4$)-carbonyle ou carboxy,

et les composés de formule I pouvant éventuellement se trouver sous forme de stéréoisomères purs ou de mélanges de ceux-ci.

**2.** Médicaments selon la revendication 1, caractérisés par une teneur en - ou consistant en - au moins un composé de formule I et/ou éventuellement un de ses sels, dans lesquels
$R^1$ représente

a) un groupe alkyle en $C_1$-$C_4$ ou hydroxyalkyle en $C_1$-$C_4$ ou phényl-alkyle($C_1$-$C_2$) ou phénylalcényle($C_1$-$C_3$), éventuellement ramifié,

b) un groupe phényle, naphtyle, pyridyle ou thiényle non substitué ou substitué une ou plusieurs fois par un ou des atomes d'halogène ou groupes alkyle en $C_1$-$C_4$, hydroxy, alcoxy en $C_1$-$C_2$, phénoxy, trifluorométhyle, nitro, di(alkyl en $C_1$-$C_2$)-amino, (alcoxy en $C_1$-$C_2$)-carbonyle, carboxy ou phényle,

c) le groupe carboxy, méthoxycarbonyle ou éthoxycarbonyle,

d) le groupe benzoyle,

e) un atome de chlore ou de brome,

A représente une simple liaison C-C ou une double liaison C-C,

n est 0 ou 1, et

X et Y, qui peuvent être identiques ou différents, représentent, indépendamment l'un de l'autre, le groupe méthyle ou éthyle ou les radicaux $-OR^2$ ou $-NR^2R^3$, $R^2$ représentant un atome d'hydrogène ou le groupe méthyle ou éthyle et $R^3$ représentant également un atome d'hydrogène ou le groupe méthyle ou éthyle, ou bien le squelette carboné d'un aminoacide à groupe carboxy éventuellement protégé, les radicaux $R^2$ et $R^3$, dans le groupe $-NR^2R^3$, pouvant également former, conjointement avec l'atome d'azote, un cycle pyrrolidine, pipéridine ou morpholine, et les radicaux $-OR^2$, dans l'élément structural $-P(O)(OR^2)_2$ pouvant former, conjointement avec l'atome de phosphore, un cycle 2-oxo-1,3,2-dioxaphospholane ou 2-oxo-1,3,2-dioxaphosphirane évenuellement substitué par un groupe alkyle en $C_1$-$C_2$,

et ces composés pouvant éventuellement se trouver sous forme de stéréoisomères pure ou de mélanges de ceux-ci.

**3.** Médicaments selon la revendication 2, caractérisés par une teneur en - ou consistant en - au moins un composé de formule I et/ou éventuellement un de ses sels, dans lesquels, soit

$R^1$ représente le groupe tert-butyle, benzyle, phényle, naphtyle, pyridyle, thiényle ou un groupe phényle substitué par l'atome de chlore ou de fluor ou par le groupe méthyle, hydroxy, méthoxy, phénoxy, trifluorométhyle, nitro, diméthylamino, méthoxycarbonyle ou carboxy, soit

X et Y représentent, indépendamment l'un de l'autre, le groupe hydroxy, méthoxy ou éthoxy, ou

X représente le groupe méthyle et Y représente le groupe hydroxy, méthoxy ou éthoxy,

et ces composés pouvant éventuellement se trouver sous forme de stéréoisomères purs ou de mélanges de ceux-ci.

**4.** Médicaments selon la revendication 3, caractérisés par une teneur en - ou consistant en - au moins un composé de formule I et/ou éventuellement un de ses sels, dans lesquels, $R^1$, X et Y ont en même temps les significations définies dans la revendication 3, et ces composés pouvant éventuellement se trouver sous forme de stéréoisomères purs ou de mélanges de ceux-ci.

**5.** Médicaments selon la revendication 4, caractérisés par une teneur en - ou consistant en - au moins un composé de formule I et/ou éventuellement un de ses sels, dans lesquels A représente une simple liaison C-C et n a la valeur 0, ces composés pouvant se trouver sous forme de stéréoisomères purs ou de mélanges de ceux-ci.

**6.** Médicaments selon la revendication 5, caractérisés par une teneur en - ou consistant en - au moins un composé de formule I et/ou éventuellement un de ses sels, dans lesquels $R^1$ représente le groupe tert-butyle ou phényle, X et Y représentent chacun le groupe hydroxy, ou X représente le groupe méthyle et Y le groupe hydroxy, ces composés pouvant se trouver sous forme de stéréoisomères purs ou de mélanges de ceux-ci.

**7.** Médicaments selon la revendication 6, caractérisés par une teneur en - ou consistant en - l'acide 3-phényl-2-isoxazoline-5-yl-phosphonique et/ou l'acide 3-phényl(ou 3-tert-butyl)-2-isoxazoline-5-yl-(P-méthyl)-phosphinique et/ou au moins un de leurs sels, ces composés pouvant se trouver sous forme de stéréoisomères purs ou de mélanges de ceux-ci.

**8.** Médicaments selon les revendications 1 à 7, caractérisés en ce qu'ils sont destinés à la prophylaxie et/ou au traitement de maladies du système immunitaire.

**9.** Médicaments selon la revendication 8, caractérisés en ce qu'ils sont utilisés pour la prophylaxie et/ou le traitement de tumeurs, d'infections et/ou de maladies autoimmunes et pour l'addition d'adjuvant à des vaccins.

**10.** Procédé pour la fabrication d'un médicament selon les revendications 1 à 9, caractérisé en ce que l'on met sous une forme pharmaceutique appropriée au moins un composé de formule I, éventuellement sous forme de stéréoisomère pur et/ou sous forme de sel physiologiquement acceptable, avec un véhicule physiologiquement acceptable et éventuellement d'autres adjuvants et/ou additifs.

**11.** Composés de formule I, leurs formes éventuellement stéréoisomères, et éventuellement leurs sels physiologiquement acceptables, caractérisés en ce que $R^1$, A, n, X et Y ont les significations données dans la revendication 1, à l'exception des composés acide 3-phényl-2-isoxazoline-5-yl-phosphonique, 3-méthyl(et phényl)-2-isoxazoline-5-yl-phosphonate de diméthyle, 3-(3-nitrophényl)-2-isoxazoline-5-yl-phosphonate de dipropyle, 3-[2-nitro-5-(2-chloro-4-trifluorométhyl-phénoxy)-phényl]-2-isoxazoline-5-yl-phosphonate de diéthyle, 3-méthyl(et phényl)-2-isoxazoline-5-yl-phosphotétraméthyldiamide, acide 3-phényl-2-isoxazoline-5-yl-méthyl-phosphonique et son ester diéthylique, 3-méthyl(éthyl, isopropyl, tert-butyl, méthoxyméthyl, phényl et éthoxycarbonyl)-isoxazol-5-yl-méthyl-phosphonate de diéthyle, acide 3-(4-fluoro- et 4-chlorophényl)-isoxazol-5- yl-(P-méthyl)-phosphinique et 3-phényl-isoxazol-5-yl- (P-méthyl)-phosphinate de méthyle, dans la mesure où il s'agit de leurs formes éventuellement racémiques.

**12.** Composés selon la revendication 11, leurs formes éventuellement stéréoisomères et éventuellement leurs sels, caractérisés en ce que $R^1$, A, n, X et Y sont tels que définis dans la revendication 2, à l'exception des composés acide 3-phényl-2-isoxazoline-5-yl-phosphonique, 3-méthyl(et phényl)-2-isoxazoline-5-yl-phosphonate de diméthyle, 3-méthyl(et phényl)-2-isoxazoline-5-yl-phosphotétraméthyldiamide, acide 3-phényl-2-isoxazoline-5-yl-méthyl-phosphonique et son ester diéthylique, 3-méthyl(éthyl, isopropyl, tert-butyl, phényl et éthoxycarbonyl)-isoxazol-5-yl-méthyl-phosphonatede diéthyle, acide 3-(4-fluoro- et 4-chlorophényl)-isoxazol-5-yl-(P-méthyl)-phosphinique et 3-phényl-isoxazol-5-yl-(P-méthyl)-phosphinate de méthyle, dans la mesure où il s'agit de leurs formes éventuellement racémiques.

**13.** Composés selon la revendication 2, leurs formes éventuellement stéréoisomères et éventuellement leurs sels, caractérisés en ce que $R^1$, X et Y représentent les radicaux définis dans la revendication 3, et A et n ont les signications données dans la revendication 2, à l'exception des composés acide 3-phényl-2-isoxazoline-5-yl-phosphonique, 3-méthyl(et phényl)-2-isoxazoline-5-yl-phosphonate de diméthyle, 3-phényl-2-isoxazoline-5-yl-phosphotétraméthyldiamide, acide 3-phényl-2-isoxazoline-5-yl-méthyl-phosphonique et son ester diéthylique, 3-méthyl(éthyl, isopropyl, tert-butyl, phényl et éthoxycarbonyl)-isoxazol-5-yl-méthyl-phosphonatede diéthyle, acide 3-(4-fluoro- et 4-chlorophényl)-isoxazol-5-yl-(P-méthyl)-phosphinique et 3-phényl-isoxazol-5-yl-(P-méthyl)-phosphinate de méthyle, dans la mesure où il s'agit de leurs formes éventuellement racémiques.

**14.** Composés selon la revendication 13, leurs formes éventuellement stéréoisomères et éventuellement leurs sels, caractérisés en ce que $R^1$, X et Y représentent les radicaux définis dans la revendication 4, et A et n ont les significations données dans la revendication 2, à l'exception des composés acide 3-phényl-2-isoxazoline-5-yl-phosphonique et son ester diméthylique, acide 3-phényl-2-isoxazoline-5-yl-méthyl- phosphonique et son ester diéthylique, 3-tert-butyl-(et phényl)-isoxazol-5-yl-méthyl-phosphonate de diéthyle, acide 3-(4-fluoro- et 4-chlorophényl)-isoxazol-5-yl-(P-méthyl)-phosphinique et 3-phényl-isoxazol-5-yl-(P-méthyl)-phosphinate de méthyle, dans la mesure où il s'agit de leurs formes éventuellement racémiques.

**15.** Composés selon la revendication 14, leurs formes stéréoisomères et éventuellement leurs sels, caractérisés en ce que $R^1$, X et Y représentent les radicaux définis dans la revendication 4 et A et n ont les significations données dans la revendication 5, à l'exception de l'acide 3-phényl-2-isoxazoline-5-yl-phosphonique et son ester diméthylique, dans la mesure où il s'agit de leurs formes racémiques.

**16.** Composés selon la revendication 15, leurs formes stéréoisomères et leurs sels, caractérisés en ce que $R^1$, X et Y représentent des radicaux définis dans la revendication 6 et A et n ont les significations données dans la revendication 5, à l'exception de l'acide 3-phényl-2-isoxazoline-5-yl-phosphonique racémique.

**17.** Composés selon la revendication 16, leurs formes stéréoisomères et leurs sels, caractérisés en ce qu'il représentent l'acide 3-phényl- ou 3-tert-butyl-2-isoxazoline-5-yl-(P-méthyl)-phosphinique.

**18.** Composés selon la revendication 16 et leurs sels, caractérisés en ce qu'ils représentent les formes énantiomères de l'acide 3-phényl-2-isoxazoline-5-yl-phosphonique.

**19.** Procédé pour la préparation de 2-isoxazoline et d'isoxazole phosphorés de formule générale I selon les revendications 11 à 18, de leurs formes éventuellement stéréoisomères, et éventuellement de leurs sels physiologiquement acceptables, caractérisé en ce que on fait réagir un oxyde de nitrile de formule II

$R^1$-C≡N→O (II)

a) dans le cas où A, dans la formule I, représente une simple liaison C-C, avec un composé phosphoré oléfinique de formule III

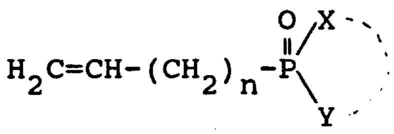

(III)

ou

b) dans le cas où A, dans la formule I, représente une double liaison C-C, avec un composé phosphoré oléfinique de formule IV

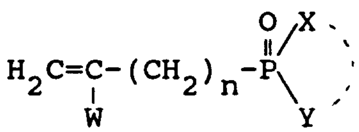

(IV)

pour aboutir à une 2-isoxasoline de formule V

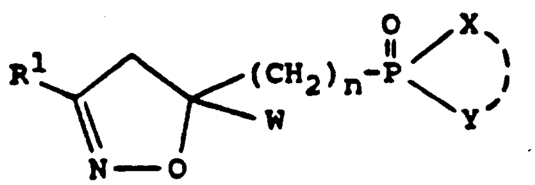

(V)

et, à partir de ce composé intermédiaire, on élimine HW dans des conditions basiques ou sous contrainte thermique, dans les formules II à V, $R^1$, n, X et Y ayant les significations données précédemment et W représentant un groupe séparable, en particulier un atome d'halogène, et éventuellement

c) on coupe un ester d'acide phosphonique ou phosphinique de formule I, obtenu selon a) ou b), pour obtenir l'hémiester d'acide phosphonique ou l'acide phosphonique ou phosphinique de formule I, ou

d) on fait réagir un phosphonate de dialkyle de formule I, obtenu selon a) ou b), avec une amine de formule $HNR^2R^3$ (VI), avec échange de l'un des deux groupes alcoxy situés sur l'atome de phosphore, contre le radical $-NR^2R^3$, pour aboutir à un hémiester-hémiamide de formule I, $R^2$ et $R^3$ ayant les significations données précédemment, et à l'exception des composés dans lesquels $R^1$ représente un atome d'halogène, ou

e) on convertit un acide phosphonique de formule I, préparé selon a), b) ou c), en un dérivé d'acide activé au niveau de l'atome de phosphore, et, si on le désire, on fait ensuite réagir celui-ci avec des alcools de formule $R^2OH$ (VII) ou avec un diol de formule

$HO-(CH_2)_{2-3}-OH$ (VIII) et/ou des amines de formule VI, pour aboutir à un mono- ou diester éventuellement mixte, à un ester cyclique, à un hémiester-hémiamide ou à un mono- ou diamide éventuellement mixte de formule I, ou on fait réagir un hémiester phosphonique de formule I, obtenu selon a) b) ou c), après activation au niveau de l'atome de phosphore, avec un alcool VII ou une amine VI, pour aboutir à un diester éventuellement mixte ou à un hémiester-hémiamide de formule I, ou

on fait réagir un acide phosphinique de formule I, préparé selon a), b) ou c), après activation au niveau de l'atome de phosphore, avec un alcool VII ou une amine VI, pour aboutir à un ester ou amide de formule I,

$R^2$ et $R^3$ dans la formule VI ayant les significations données précédemment, $R^2$ dans la formule VII représentant un groupe alkyle en $C_1$-$C_6$ éventuellement substitué, et la chaîne alkylène $-(CH_2)_{2-3}-$ dans la formule VIII pouvant être encore substituée par un groupe alkyle en $C_1$-$C_3$, (alcoxy en $C_1$-$C_4$)-carbonyle ou carboxy, ou

f) on fait réagir un mono- ou diester d'acide 3-chloro(ou bromo)2-isoxazoline-phosphonique ou ester d'acide 3-chloro(ou bromo)-2-isoxazoline-phosphinique de formule I, dans lequel n représente un nombre entier allant de 0 à 2, avec des tri(alkyl en $C_1$-$C_4$)-halogénosilanes, avec coupure de l'ester et échange simultané de l'atome de chlore ou de brome contre l'atome d'halogène du silane utilisé dans chaque cas, pour aboutir aux acides 3-halogéno-2-isoxazoline-phosphoniques ou -phosphiniques de formule I correspondants, ou

g) on scinde en les stéréoisomères purs un composé de formule I, obtenu selon a) à f), qui, en raison de sa structure chimique apparaît sous formes diastéréoisomères ou énantiomères,

les composés de formule I préparés selon a) à g) étant soit isolés sous forme libre soit éventuellement convertis en sels physiologiquement acceptables.

**20.** Composés de formule I, leurs formes éventuellement stéréoisomères et/ou éventuellement leurs sels physiologiquement acceptables, pour utilisation dans un procédé pour le traitement prophylactique et/ou thérapeutique de l'organisme humain ou animal.

**21.** Mode de réalisation selon la revendication 20, pour le traitement prophylactique et/ou thérapeutique de maladies du système immunitaire de l'organisme humain ou animal.

**22.** Mode de réalisation selon la revendication 20, pour le traitement prophylactique et/ou thérapeutique de tumeurs, d'infections et/ou de maladies auto-immunes de l'organisme humain ou animal, ainsi que pour l'addition d'adjuvant à des vaccins.

**23.** Utilisation de composés de formule I, de leurs formes éventuellement stéréoisomères et/ou éventuellement de leurs sels physiologiquement acceptables pour la fabrication de médicaments selon les revendications 1 à 7.

**24.** Utilisation de composés de formule I, de leurs formes éventuellement stéréoisomères et/ou éventuellement de leurs sels physiologiquement acceptables, pour la fabrication de médicaments selon la revendication 23, qui sont destinés à la prophylaxie et/ou au traitement de maladies du système immunitaire.

**25.** Utilisation des composés de formule I de leurs formes éventuellement stéréoisomères et/ou éventuellement de leurs sels physiologiquement acceptables, pour la fabrication de médicaments selon la revendication 24, qui sont destinés à la prophylaxie et/ou au traitement de tumeurs, d'infections et/ou de maladies auto-immunes et pour l'addition d'adjuvant à des vaccins.

**Revendications pour les Etats contractants suivants : ES, GR**

**1.** Procédé pour la fabrication d'un médicament, caractérisé en ce que l'on met sous une forme pharmaceutique appropriée au moins un composé de formule I ci-après, éventuellement sous forme de stéréoisomère pur et/ou sous forme de sel physiologiquement acceptable avec un véhicule physiologiquement acceptable et éventuellement d'autres additifs et/ou adjuvants:

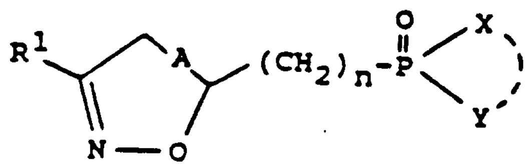

(I)

formule dans laquelle $R^1$ représente

a) un groupe alkyle ou alcényle à chaîne droite ou ramifiée ayant de 1 à 6 atomes de carbone, dont la chaîne carbonée peut être substituée par un atome d'halogène ou par un groupe hydroxy alcoxy en $C_1$-$C_4$, acyloxy en $C_1$-$C_4$ ou par un radical aryle éventuellement substitué par un atome d'halogène ou par un groupe alcoxy en $C_1$-$C_4$, ou

b) un groupe aromatique ou hétéroaromatique mono- ou binucléaire comportant 1 ou 2 atomes d'azote et/ou un atome d'oxygène ou de soufre dans le système cyclique, ce groupe pouvant être une ou plusieurs fois substitué et de façon identique ou différente par un ou des atomes d'halogène ou groupes alkyle en $C_1$-$C_4$, cycloalkyle en $C_3$-$C_6$, hydroxy, alcoxy en $C_1$-$C_3$, aryloxy, acyloxy en $C_1$-$C_4$, benzoyloxy, halogène, trifluorométhyle, nitro, amino eventuellement mono- ou disubstitué, (alcoxy en $C_1$-$C_4$)-carbonyle, carboxy, carbamoyle, (alkyl en $C_1$-$C_4$)-carbonyle, dont le groupe carbonyle peut également se trouver sous forme cétalisée ou phényle ou benzyle éventuellement substitués sur le noyau par un atome d'halogène ou par un groupe alkyle en $C_1$-$C_4$ ou alcoxy en $C_1$-$C_3$, ou

c) un groupe carboxy ou alcoxycarbonyle ayant de 1 à 4 atomes de carbone dans le fragment alkyle, ou

d) un groupe arylcarbonyle qui est éventuellement substitué sur le fragment aryle par un atome d'halogène ou par un groupe alkyle en $C_1$-$C_4$ ou alcoxy en $C_1$-$C_3$, ou

e) un atome d'halogène,

A représente une simple liaison C-C ou une double liaison C-C,

n est un nombre entier allant de 0 à 2, et

X et Y qui peuvent être identiques ou différents, représentent chacun, indépendamment l'un de l'autre, un groupe alkyle en $C_1$-$C_4$ à chaîne droite ou ramifiée, un radical $-OR^2$ ou $-NR^2R^3$, $R^2$ et $R^3$ représentant des atomes d'hydrogène ou des radicaux alkyle en $C_1$-$C_6$ éventuellement substitués qui, dans le groupe $-NR^2R^3$, peuvent également former, conjointement avec l'atome d'azote, un cycle à 5-7 chaînons ou, dans l'élément structural $-P(O)(OR^2)_2$, peuvent former, conjointement avec l'atome de phosphore, un hétérocycle de formule

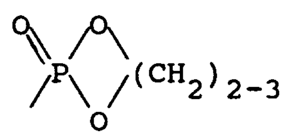

éventuellement encore substitué par un groupe alkyle en $C_1$-$C_3$, (alcoxy en $C_1$-$C_4$)-carbonyle ou carboxy.

**2.** Procédé selon la revendication 1, caractérisé en ce que dans la formule I

$R^1$ représente

a) un groupe alkyle en $C_1$-$C_4$ ou hydroxyalkyle en $C_1$-$C_4$ ou phényl-alkyle($C_1$-$C_2$) ou phénylalcényle($C_1$-$C_3$), éventuellement ramifié,

b) un groupe phényle, naphtyle, pyridyle ou thiényle non substitué ou substitué une ou plusieurs fois par un ou des atomes d'halogène ou groupes alkyle en $C_1$-$C_4$, hydroxy, alcoxy en $C_1$-$C_2$, phénoxy, trifluorométhyle, nitro, di(alkyl en $C_1$-$C_2$)-amino, (alcoxy en $C_1$-$C_2$)-carbonyle, carboxy ou phényle,

c) le groupe carboxy, méthoxycarbonyle ou éthoxycarbonyle,

d) le groupe benzoyle,

e) un atome de chlore ou de brome,

A représente une simple liaison C-C ou une double liaison C-C,

n est 0 ou 1, et

X et Y, qui peuvent être identiques ou différents, représentent, indépendamment l'un de l'autre, le groupe méthyle ou éthyle ou les radicaux $-OR^2$ ou $-NR^2R^3$, $R^2$ représentant un atome d'hydrogène ou le groupe méthyle ou éthyle et $R^3$ représentant également un atome d'hydrogène ou le groupe méthyle ou éthyle ou bien le squelette carboné d'un aminoacide à groupe carboxy éventuellement protégé, les radicaux $R^2$ et $R^3$, dans le groupe $-NR^2R^3$, pouvant également former, conjointement avec l'atome d'azote, un cycle pyrrolidine, pipéridine ou morpholine, et les radicaux $-OR^2$, dans l'élément structural $-P(O)(OR^2)_2$ pouvant former, conjointement avec l'atome de phosphore, un cycle 2-oxo-1,3,2-dioxaphospholane ou 2-oxo-1,3,2-dioxaphosphirane évenuellement substitué par un groupe alkyle en $C_1$-$C_2$.

**3.** Procédé selon la revendication 2, caractérisé en ce que, dans la formule I soit

$R^1$ représente le groupe tert-butyle, benzyle, phényle, naphtyle, pyridyle, thiényle ou un groupe phényle substitué par l'atome de chlore ou de fluor ou par le groupe méthyle, hydroxy, méthoxy, phénoxy, trifluorométhyle, nitro, diméthylamino, méthoxycarbonyle ou carboxy, soit

X et Y représentant, indépendamment l'un de l'autre le groupe hydroxy, méthoxy ou éthoxy, ou

X représente le groupe méthyle et Y représente le groupe hydroxy, méthoxy ou éthoxy.

**4.** Procédé selon la revendication 3, caractérisé en ce que, dans la formule I, $R^1$, X et Y ont en même temps les significations définies dans la revendication 3.

**5.** Procédé selon la revendication 4, caractérisé en ce que, dans la formule I A représente une simple liaison C-C et n a la valeur 0.

**6.** Procédé selon la revendication 5, caractérisé en ce que dans la formule I, $R^1$ représente le groupe tert-butyle ou phényle, X et Y représentent chacun le groupe hydroxy ou X représente le groupe méthyle et Y le groupe hydroxy.

**7.** Procédé selon la revendication 6, caractérisé en ce que, le(s) composé(s) de formule I est (sont) l'acide 3-phényl-2-isoxazoline-5-yl-phosphonique et/ou l'acide 3-phényl(ou 3-tert-butyl)-2-isoxazoline-5-yl-(P-méthyl)-phosphinique.

**8.** Procédé selon une ou plusieurs des revendications 1 à 7, caractérisé en ce que les médicaments fabriqués selon le procédé sont destinés à la prophylaxie et/ou au traitement de maladies du système immunitaire.

**9.** Procédé selon la revendication 8, caractérisé en ce que les médicaments fabriqués selon le procédé sont utilisés pour la prophylaxie et/ou le traitement de tumeurs, d'infections et/ou de maladies auto-immunes et pour l'addition d'adjuvant à des vaccins.

**10.** Procédé pour la préparation de 2-oxazoline et isoxazole phosphorés de formule générale I, selon la définition dans la revendication 1, de leurs formes éventuellement stéréoisomères et éventuellement de leurs sels physiologiquement acceptables, à l'exception des composés acide 3-phényl-2-isoxazoline-5-yl-phosphonique, 3-méthyl(et phényl)-2-isoxazoline-5-yl-phosphonate de diméthyle, 3-(3-nitrophényl)-2-isoxazoline-5-yl-phosphonate de dipropyle, 3-[2-nitro-5-(2-chloro-4-trifluorométhyl-phénoxy)-phényl]-2-isoxazoline-5-ylphosphonate de diéthyle, 3-méthyl(et phényl)-2-isoxazoline-5-yl-phosphotétraméthyldiamide, acide 3-phényl-2-isoxazoline-5-yl-méthyl-phosphonique et son ester diéthylique, 3-méthyl(éthyl, isopropyl, tert-butyl, méthoxyméthyl, phényl et éthoxycarbonyl)-isoxazol-5-yl-méthyl-phosphonate de diéthyle, acide 3-(4-fluoro- et 4-chlorophényl)-isoxazol-5-yl-(P-méthyl)-phosphinique et 3-phényl-isoxazol-5-yl-(P-méthyl)-phosphinate de méthyle, dans la mesure où il s'agit de leurs formes éventuellement racémiques,
caractérisé en ce que l'on fait réagir un oxyde de nitrile de formule II

$$R^1\text{-}C{\equiv}N{\rightarrow}O \qquad (II)$$

a) dans le cas où A, dans la formule I, représente une simple liaison C-C, avec un composé phosphoré oléfinique de formule III

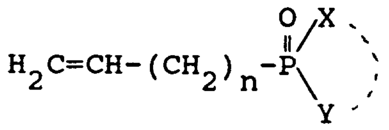

(III)

ou

b) dans le cas où A, dans la formule I, représente une double liaison C-C, avec un composé phosphoré oléfinique de formule IV

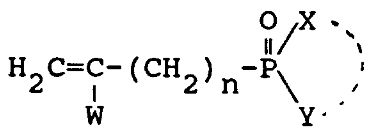

(IV)

pour aboutir à une 2-isoxazoline de formule v

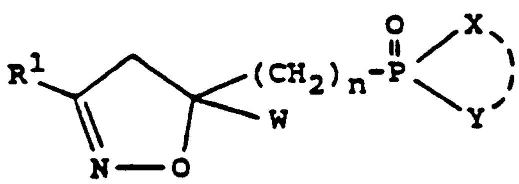

(V)

et, à partir de ce composé intermédiaire, on élimine HW dans des conditions basiques ou sous contrainte thermique, dans les formules II à V, $R^1$, n, X et Y ayant les significations données précédemment et W représentant un groupe séparable, en particulier un atome d'halogène, et éventuellement

c) on coupe un ester d'acide phosphonique ou phosphinique de formule I, obtenu selon a) ou b), pour obtenir l'hémiester d'acide phosphonique ou l'acide phosphonique ou phosphinique de formule I, ou

d) on fait réagir un phosphonate de dialkyle de formule I, obtenu selon a) ou b), avec une amine de formule $HNR^2R^3$ (VI), avec échange de l'un des deux groupes alcoxy situés sur l'atome de phosphore, contre le radical $-NR^2R^3$, pour aboutir à un hémiester-hémiamide de formule I, $R^2$ et $R^3$ ayant les significations données précédemment, et à l'exception des composés dans lesquels $R^1$ représente un atome d'halogène, ou

e) on convertit un acide phosphonique de formule I, préparé selon a), b) ou c), en un dérivé d'acide activé au niveau de l'atome de phosphore, et, si on le désire, on fait ensuite réagir celui-ci avec des alcools de formule $R^2OH$ (VII) ou avec un diol de formule

$HO-(CH_2)_{2-3}-OH$ (VIII) et/ou des amines de formule VI, pour aboutir à un mono- ou diester éventuellement mixte, à un ester cyclique, à un hémiester-hémiamide ou à un mono- ou diamide éventuellement mixte de formule I, ou

on fait réagir un hémiester phosphonique de formule I, obtenu selon a), b) ou c), après activation au niveau de l'atome de phosphore, avec un alcool VII ou une amine VI, pour aboutir à un diester éventuellement mixte ou à un hémiester-hémiamide de formule I, ou

on fait réagir un acide phosphinique de formule I, préparé selon a), b) ou c), après activation au niveau de l'atome de phosphore, avec un alcool VII ou une amine VI, pour aboutir à un ester ou amide de formule I,

$R^2$ et $R^3$ dans la formule VI ayant les significations données précédemment, $R^2$ dans la formule VII représentant un groupe alkyle en $C_1$-$C_6$ éventuellement substitué, et la chaîne alkylène $-(CH_2)_{2-3}-$ dans la formule VII pouvant être encore substituée par un groupe alkyle en $C_1$-$C_3$, (alcoxy en $C_1$-$C_4$)-carbonyle ou carboxy, ou

f) on fait réagir un mono- ou diester d'acide 3-chloro(ou bromo)-2-isoxazoline-phosphonique ou ester d'acide 3-chloro(ou bromo)-2-isoxazoline-phosphinique de formule I, dans lequel n représente un nombre entier allant de 0 à 2, avec des tri(alkyl en $C_1$-$C_4$)-halogénosilanes, avec coupure de l'ester et échange simultané de l'atome de chlore ou de brome contre l'atome d'halogène du silane utilisé dans chaque cas, pour aboutir aux acides 3-halogéno-2-isoxazoline-phosphoniques ou -phosphiniques de formule I correspondants, ou

g) on scinde en les stéréoisomères purs un composé de formule I, obtenu selon a) à f) qui, en raison de sa structure chimique, apparait sous formes diastéréoisomères ou énantiomères,

les composés de formule I, préparés selon a) à g), étant soit isolés sous forme libre, soit éventuellement convertis en sels physiologiquement acceptables.

**11.** Procédé selon la revendication 10, caractérisé en ce que l'on prépare des composés de formule I dans lesquels $R^1$, A, n, X et Y sont tels que définis dans la revendication 2, à l'exception des composés acide 3-phényl-2-isoxazoline-5-yl-phosphonique, 3-méthyl(et phényl)-2-isoxazoline-5-yl-phosphonate de diméthyle, 3-méthyl(et phényl)-2-isoxazoline-5-yl-phosphotétraméthyl-diamide, acide 3-phényl-2-isoxazoline-5-yl-méthyl-phosphonique et son ester diéthylique, 3-méthyl(éthyl, isopropyl, tert-butyl, phényl et éthoxycarbonyl)-isoxazol-5-yl-méthyl-phosphonate de diéthyle, acide 3-(4-fluoro- et 4-chlorophényl)-isoxazol-5-yl-(P-méthyl)-phosphinique et 3-phényl-isoxazol-5-yl-(P-méthyl)-phosphinate de méthyle, dans la mesure où il s'agit de leurs formes éventuellement racémiques.

**12.** Procédé selon la revendication 11, caractérisé en ce que l'on prépare des composés de formule I dans lesquels $R^1$, X et Y représentent les radicaux définis dans la revendication 3, et A et n ont les significations données dans la revendication 2, à l'exception des composés acide 3-phényl-2-isoxazoline-5-yl-phosphonique, 3-méthyl(et phényl)-2-isoxazoline-5-yl-phosphonate de diméthyle, 3-phényl-2-isoxazoline-5-yl-phosphotétraméthyl-diamide, acide 3-phényl-2-isoxazoline-5-yl-méthyl-phosphonique et son ester diéthylique, 3-méthyl(éthyl, isopropyl, tert-butyl, phényl et éthoxycarbonyl)-isoxazol-5-yl-méthyl-phosphonate de diéthyle, acide 3-(4-fluoro- et 4-chlorophényl)-isoxazol-5-yl-(P-méthyl)-phosphinique et 3-phényl-isoxazol-5-yl-(P-méthyl)-phosphinate de méthyle, dans la mesure où il s'agit de leurs formes éventuellement racémiques.

**13.** Procédé selon la revendication 12, caractérisé en ce que l'on prépare des composés de formule I dans lesquels, $R^1$, X et Y représentent les radicaux définis dans la revendication 4, et A et n ont les significations données dans la revendication 2, à l'exception des composés acide 3-phényl-2-isoxazoline-5-yl-phosphonique et son ester diméthylique, acide 3-phényl-2-isoxazoline-5-yl-méthyl-phosphonique et son ester diéthylique, 3-tert-butyl(et phényl)-isoxazol-5-yl-méthyl-phosphonate de diéthyle, acide 3-(4-fluoro- et 4-chlorophényl)-isoxazol-5-yl-(P-méthyl)-phosphinique et 3-phényl-isoxazol-5-yl-(P-méthyl)-phosphinate de méthyle, dans la mesure où il s'agit de leurs formes éventuellement racémiques.

**14.** Procédé selon la revendication 13, caractérisé en ce que l'on prépare des composés de formule I dans lesquels $R^1$, X et Y représentent les radicaux définis dans la revendication 4 et A et n ont les significations données dans la revendication 5, à l'exception de l'acide 3-phényl-2-isoxazoline-5-yl-phosphonique et de son ester diméthylique, dans la mesure où il s'agit de leurs formes racémiques.

**15.** Procédé selon la revendication 14, caractérisé en ce que l'on prépare des composés de formule I dans lesquels $R^1$, X et Y représentent des radicaux définis dans la revendication 6 et A et n ont les significations données dans la revendication 5, à l'exception de l'acide 3-phényl-2-isoxazoline-5-yl-phosphonique racémique.

**16.** Procédé selon la revendication 15, caractérisé en ce que l'on prépare l'acide 3-phényl- ou 3-tert-butyl-2-isoxazoline-5-yl-(P-méthyl)-phosphinique.

**17.** Procédé selon la revendication 15, caractérisé en ce que l'on prépare les formes énantiomères de l'acide 3-phényl-2-isoxazoline-5-yl-phosphonique.

**18.** Procédé pour la préparation de composés de formule I selon la définition dans la revendication 1, de leurs formes éventuellement stéréoisomères et/ou éventuellement de leurs sels physiologiquement acceptables, pour utilisation dans un procédé pour le traitement prophylactique et/ou thérapeutique de l'organisme humain ou animal.

**19.** Procédé selon la revendication 18, caractérisé en ce que l'on prépare les composés pour utilisation dans un procéde pour le traitement prophylactique et/ou thérapeutique de maladies du système immunitaire de l'organisme humain ou animal.

**20.** Procédé selon la revendication 18, caractérisé en ce que l'on prépare les composés pour utilisation dans un procédé pour le traitement prophylactique et/ou thérapeutique de tumeurs, d'infections et/ou de maladies auto-immunes de l'organisme humain ou animal, ainsi que pour l'addition d'adjuvant à des vaccins.

**21.** Utilisation de composés de formule I, selon la définition dans la revendication 1, de leurs formes éventuellement stéréoisomères et/ou éventuellement de leurs sels physiologiquement acceptables, pour la fabrication de médicaments.

**22.** Utilisation de composés de formule I, selon la définition dans la revendication 1, de leurs formes éventuellement stéréoisomères et/ou éventuellement de leurs sels physiologiquement acceptables, pour la fabrication de médicaments selon la revendication 21, qui sont destinés à la prophylaxie et/ou au traitement de maladies du système immunitaire.

**23.** Utilisation des composés de formule I, selon la définition dans la revendication 1, de leurs formes éventuellement stéréoisomères et/ou éventuellement de leurs sels physiologiquement acceptables, pour la fabrication de médicaments selon la revendication 22, qui sont destinés à la prophylaxie et/ou au traitement de tumeurs, d'infections et/ou de maladies auto-immunes et pour l'addition d'adjuvant à des vaccins.